# EUROPEAN PATENT SPECIFICATION

(11) **EP 4 038 064 B1**
(45) Date of publication and mention of the grant of the patent: **21.02.2024**
(21) Application number: 20789276.1
(22) Date of filing: 29.09.2020
(51) Int. Cl.: C07D 403/14, A61P 31/18, A61K 31/513

(54) **N-SUBSTUTUTED-6-OXO-1,6-DIHYDROPYRIMIDINE-2-YL DERIVATIVES AS INHIBITORS OF THE HUMAN IMMUNODEFICIENCY VIRUS REPLICATION**
N-SUBSTITUIERTE-6-OXO-1,6-DIHYDROPYRIMIDIN-2-YL-DERIVATE ALS INHIBITOREN DER REPLIKATION DES MENSCHLICHEN IMMUNSCHWÄCHEVIRUS
DÉRIVÉS DE 6-OXO-1,6-DIHYDROPYRIMIDINE-2-YL N-SUBSTITUÉS UTILISÉS EN TANT QU'INHIBITEURS DE LA RÉPLICATION DU VIRUS DE L'IMMUNODÉFICIENCE HUMAINE

(30) Priority: 01.10.2019 US 201962908677 P; 23.10.2019 US 201962924781 P; 30.01.2020 US 202062967761 P
(43) Date of publication of application: 10.08.2022
(73) Proprietor: VIIV Healthcare UK (No.5) Limited, Stevenage SG1 2NY (GB)
(72) Inventor: NAIDU, B. Narasimhulu, Branford, Connecticut 06405 (US); PATEL, Manoj, Branford, Connecticut 06405 (US)
(74) Representative: Matley, Joshua Ellis
(86) International application number: PCT/IB2020/059101
(87) International publication number: WO 2021/064570

(56) References cited:
- WO-A1-2014/110297
- WO-A1-2016/033243
- WO-A1-2018/035359
- WO-A1-2018/203235

## Description

### FIELD OF THE INVENTION

The invention relates to compounds, compositions, and methods for the treatment of human immunodeficiency virus (HIV) infection. More particularly, the invention relates to novel inhibitors of HIV, pharmaceutical compositions containing such compounds, and methods for using these compounds in the treatment of HIV infection. The invention also relates to methods for making the compounds hereinafter described.

### BACKGROUND OF THE INVENTION

Acquired immunodeficiency syndrome (AIDS) is the result of infection by HIV. HIV continues to be a major global public health issue. In 2015, an estimated 36.7 million people were living with HIV (including 1.8 million children) - a global HIV prevalence of 0.8%. The vast majority of this number live in low- and middle- income countries. In the same year, 1.1 million people died of AIDS-related illnesses.

Current therapy for HIV-infected individuals consists of a combination of approved anti-retroviral agents. Close to four dozen drugs are currently approved for HIV infection, either as single agents, fixed dose combinations or single tablet regimens; the latter two containing 2-4 approved agents. These agents belong to a number of different classes, targeting either a viral enzyme or the function of a viral protein during the virus replication cycle. Thus, agents are classified as either nucleotide reverse transcriptase inhibitors (NRTIs), non-nucleotide reverse transcriptase inhibitors (NNRTIs), protease inhibitors (PIs), integrase strand transfer inhibitors (INSTIs), or entry inhibitors (one, maraviroc, targets the host CCR5 protein, while the other, enfuvirtide, is a peptide that targets the gp41 region of the viral gp160 protein). In addition, a pharmacokinetic enhancer (cobicistat or ritonavir) can be used in combinations with antiretroviral agents (ARVs) that require boosting.

Despite the armamentarium of agents and drug combinations, there remains a medical need for new anti-retroviral agents. High viral heterogeneity, drug-associated toxicity, tolerability problems, and poor adherence can all lead to treatment failure and may result in the selection of viruses with mutations that confer resistance to one or more antiretroviral agents or even multiple drugs from an entire class (Beyrer, C., Pozniak A. HIV drug resistance - an emerging threat to epidemic control. N. Engl. J. Med. 2017, 377, 1605-1607; Gupta, R. K., Gregson J., et al. HIV-1 drug resistance before initiation or re-initiation of first-line antiretroviral therapy in low-income and middle-income countries: a systematic review and meta-regression analysis. Lancet Infect. Dis. 2017, 18, 346-355; Zazzi, M., Hu, H., Prosperi, M. The global burden of HIV-1 drug resistance in the past 20 years. PeerJ. 2018, DOI 10.7717/peerj.4848). As a result, new drugs are needed that are easier to take, have high genetic barriers to the development of resistance and have improved safety over current agents. In this panoply of choices, novel mechanisms of action (MOAs) that can be used as part of the preferred antiretroviral therapy (ART) can still have a major role to play since they should be effective against viruses resistant to current agents.

Certain potentially therapeutic compounds have now been described in the art and set forth in Blair, Wade S. et.al. Antimicrobial Agents and Chemotherapy (2009), 53(12), 5080-5087, Blair, Wade S. et al. PLoS Pathogens (2010), 6(12), e1001220, Thenin-Houssier, Suzie; Valente, Susana T. Current HIV Research, 2016, 14, 270-282, and PCT Patent applications with the following numbers: WO 2012065062, WO 2013006738, WO 2013006792, WO 2014110296, WO 2014110297, WO 2014110298, WO 2014134566, WO 2015130964, WO2015130966, WO 2016033243, WO2018035359, WO2018203235, WO 2019161017, and WO 2019161280.

What is now needed in the art are additional compounds which are novel and useful in the treatment of HIV. Additionally, these compounds should provide advantages for pharmaceutical uses, for example, with regard to one or more of their mechanisms of action, binding, inhibition efficacy, target selectivity, solubility, safety profiles, bioavailability or reduced frequency of dosing. Also needed are new formulations and methods of treatment which utilize these compounds.

### SUMMARY OF THE INVENTION

The description may encompass subject-matter extending beyond the scope of the claims. However the scope of the invention and of protection is solely defined by the appended claims and shall not extend beyond their scope. In particular, the scope of protection shall not include any methods of therapeutic treatment of the human or animal body, even if disclosed or implied herein.

Briefly, in one aspect, the present invention discloses a compound of Formula I, or a pharmaceutically acceptable salt thereof: 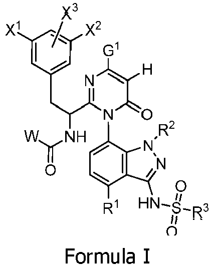 wherein:
X¹ and X² are independently selected from H, F, Cl, and -CH₃, and X³ is H, F, Cl, -CH₃, -OCH₃, - OCHF₂, or -OCF₃ with the proviso that within the group X¹, X², and X³ the substituent Cl is not used more than twice and the substituent -CH₃ is not used more than twice;
R¹ is hydrogen, Cl, or CH₃;
R² is hydrogen, C₁-C₃alkyl optionally substituted with 1-3 fluorines, or C₃-C₆cycloalkyl optionally substituted with 1-2 fluorines;
R³ is C₁-C₃alkyl or C₃-C₄ cycloalkyl;
G¹ is phenyl optionally substituted 1-3 times with a substituent independently selected from fluorine, chlorine, -CH₃, and -OCH₃, or G¹ is selected from: 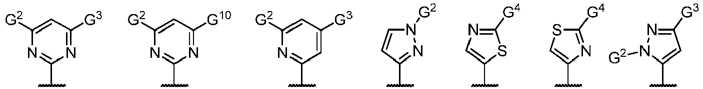 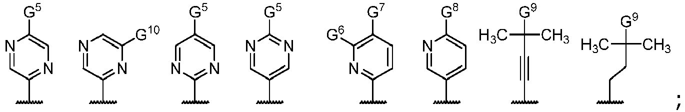
wherein G² and G³ are independently selected from hydrogen and C₁-C₃alkyl optionally substituted with 1-3 fluorines;
G⁴ is -C(CH₃)₂OH, -SO₂(C₁-C₄alkyl), or C₁-C₃alkyl optionally substituted with 1-3 fluorines;
G⁵ is H, F, C₁-C₄alkyl optionally substituted with 1-3 fluorines, or O(C₁-C₄alkyl) optionally substituted with 1-3 fluorines;
G⁶ is H, F, or Cl;
G⁷ is H, F, Cl, C₁-C₄alkyl optionally substituted with 1-3 fluorines, or O(C₁-C₄alkyl) optionally substituted with 1-3 fluorines;
G⁸ is F, Cl, -CN, C₁-C₄alkyl, or O(C₁-C₄alkyl) optionally substituted with 1-3 fluorines;
G⁹ is -O(C₁-C₃alkyl), -O(C₃-C₄cycloalkyl), -SO₂(C₁-C₃alkyl), or -SO₂(C₃-C₄cycloalkyl);
G¹⁰ is -OCH₃, -OCHF₂, or -OCF₃;
W is selected from: 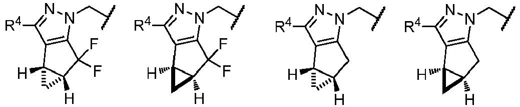 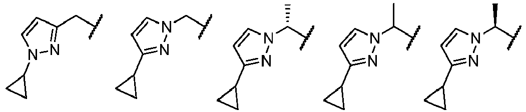
wherein R⁴ is methyl optionally substituted with 1-3 fluorines.

In another aspect, the present invention discloses a pharmaceutical composition comprising a compound of Formula I or a pharmaceutically acceptable salt thereof.

In another aspect, the present invention discloses a method of treating HIV infection in a human comprising administering a compound of Formula I or a pharmaceutically acceptable salt thereof to a patient.

In another aspect, the present invention discloses a compound of Formula I or pharmaceutically acceptable salt thereof for use in therapy.

In another aspect, the present invention discloses a compound of Formula I or pharmaceutically acceptable salt thereof for use in treating HIV infection in a human.

In another aspect, the present invention discloses the use of a compound of Formula I or pharmaceutically acceptable salt thereof in the manufacture of a medicament for the treatment of HIV infection in a human.

### DETAILED DESCRIPTION OF THE INVENTION

In one embodiment, the present invention discloses compounds of Formula I, or pharmaceutically acceptable salts thereof, wherein W is the following:

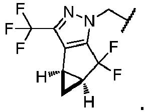

In another embodiment, the present invention discloses compounds of Formula I, or pharmaceutically acceptable salts thereof, wherein W is the following:

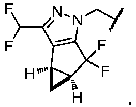

In another embodiment, the present invention discloses compounds of Formula I, or pharmaceutically acceptable salts thereof, wherein W is one of the following: 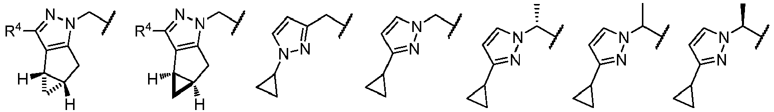 wherein R⁴ is methyl optionally substituted with 1-3 fluorines.

In one embodiment, the present invention discloses compounds of Formula I, or pharmaceutically acceptable salts thereof, wherein R¹ is Cl; R² is methyl, 2,2-difluoroethyl, or 2,2,2-trifluoroethyl; and R³ is methyl or cyclopropyl.

In one embodiment, the present invention discloses compounds of Formula I, or pharmaceutically acceptable salts thereof, wherein X³ is H.

In one embodiment, the present invention discloses compounds of Formula I, or pharmaceutically acceptable salts thereof, wherein X¹ is F, X² is F, and X³ is H.

In one embodiment, the present invention discloses compounds of Formula I, or pharmaceutically acceptable salts thereof, wherein if X³ is H then at least one of X¹ and X² is other than F.

In one embodiment, the present invention discloses compounds of Formula I, or pharmaceutically acceptable salts thereof, wherein G¹ is:

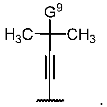

In one embodiment, the present invention discloses compounds of Formula I, or pharmaceutically acceptable salts thereof, wherein G¹ is:

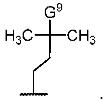

In one embodiment, the present invention discloses compounds of Formula I, or pharmaceutically acceptable salts thereof, wherein G¹ is one of the following:

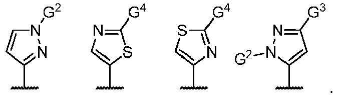

In one embodiment, the present invention discloses compounds of Formula I, or pharmaceutically acceptable salts thereof, wherein G¹ is one of the following:

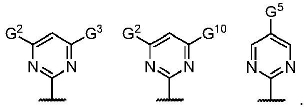

In one embodiment, the present invention discloses compounds of Formula I, or pharmaceutically acceptable salts thereof, wherein G¹ is:

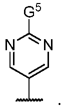

In one embodiment, the present invention discloses compounds of Formula I, or pharmaceutically acceptable salts thereof, wherein G¹ is one of the following:

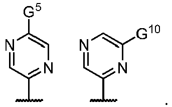

In one embodiment, the present invention discloses compounds of Formula I, or pharmaceutically acceptable salts thereof, wherein G¹ is one of the following:

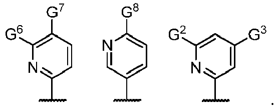

In one embodiment, the present invention discloses compounds of Formula I, or pharmaceutically acceptable salts thereof, wherein G¹ is one of the following:

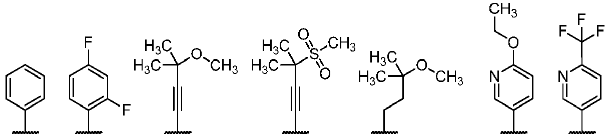

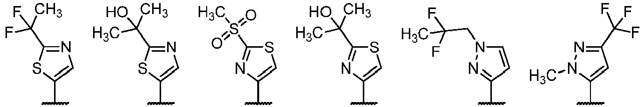

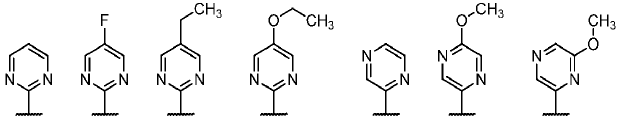

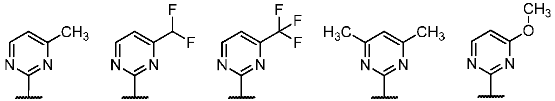

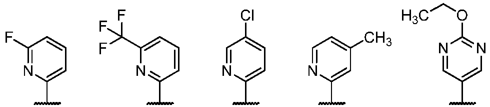

In one embodiment, the present invention discloses compounds of Formula I, or pharmaceutically acceptable salts thereof, wherein G¹ is:

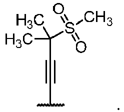

In one embodiment, the present invention discloses compounds of Formula I, or pharmaceutically acceptable salts thereof, wherein G¹ is:

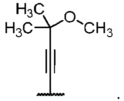

In one embodiment, the present invention discloses compounds of Formula I, or pharmaceutically acceptable salts thereof, wherein G¹ is:

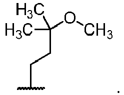

In one embodiment, the present invention discloses compounds of Formula I, or pharmaceutically acceptable salts thereof, wherein G¹ is one of the following:

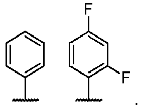

In one embodiment, the present invention discloses compounds of Formula I, or pharmaceutically acceptable salts thereof, wherein G¹ is one of the following:

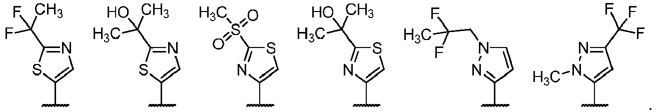

In one embodiment, the present invention discloses compounds of Formula I, or pharmaceutically acceptable salts thereof, wherein G¹ is one of the following:

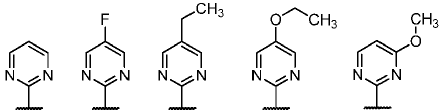

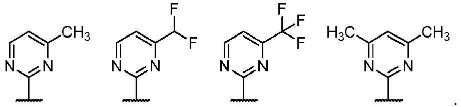

In one embodiment, the present invention discloses compounds of Formula I, or pharmaceutically acceptable salts thereof, wherein G¹ is one of the following:

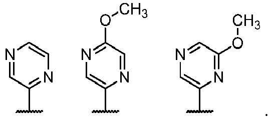

In one embodiment, the present invention discloses compounds of Formula I, or pharmaceutically acceptable salts thereof, wherein G¹ is one of the following:

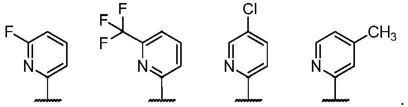

In one embodiment, the present invention discloses compounds of Formula I, or pharmaceutically acceptable salts thereof, wherein G¹ is one of the following:

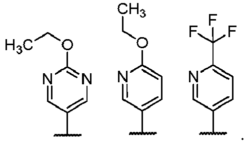

In one embodiment, the present invention discloses compounds of Formula I, or pharmaceutically acceptable salts thereof, wherein the stereochemistry is as depicted below:

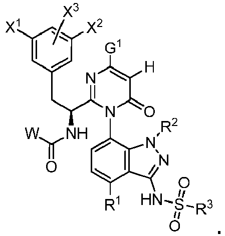

In one embodiment, the present invention discloses compounds of Formula I, or pharmaceutically acceptable salts thereof, wherein the stereochemistry is as depicted below:

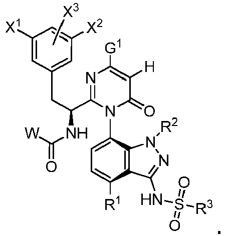

In one embodiment, the present invention discloses compounds and salts, selected from the group consisting of: 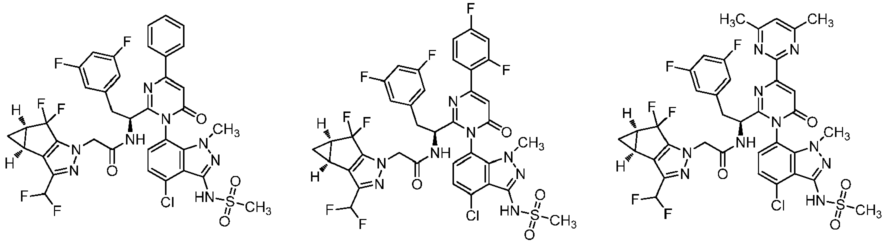 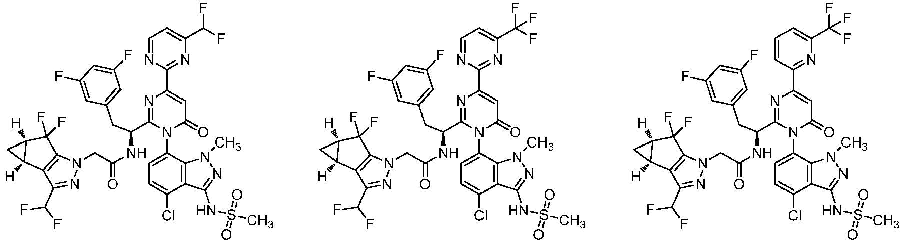 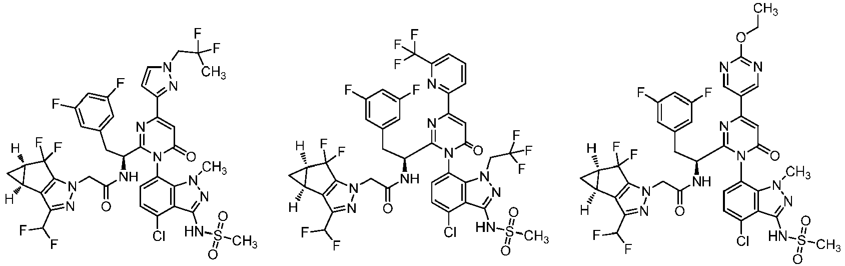 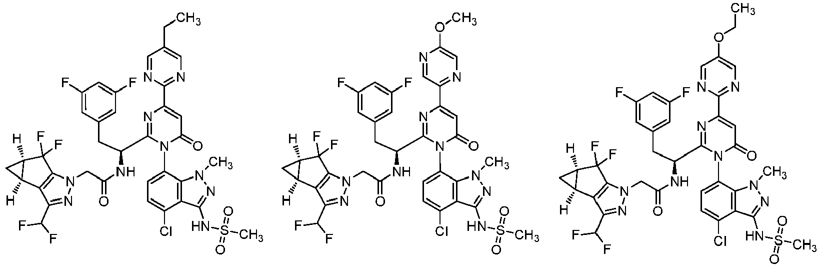 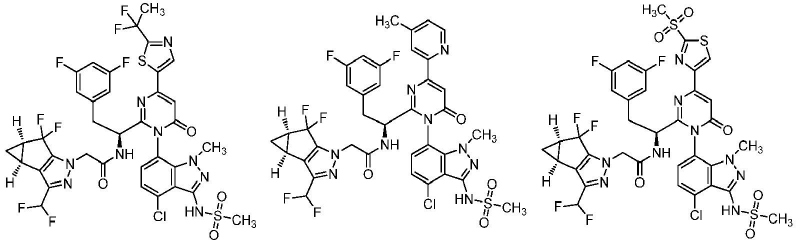 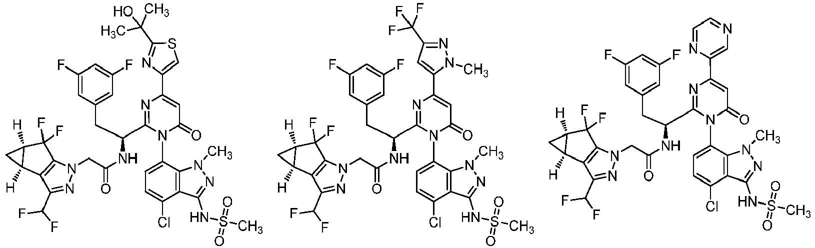 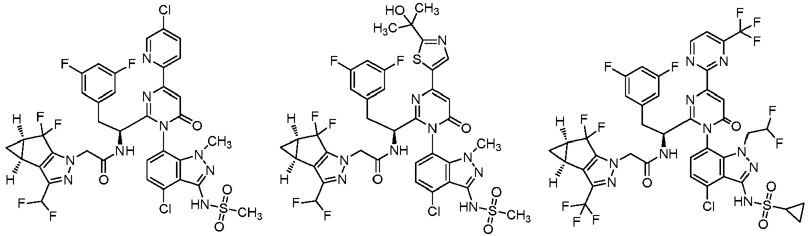 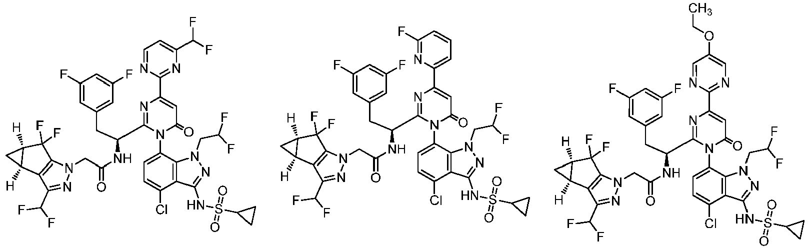 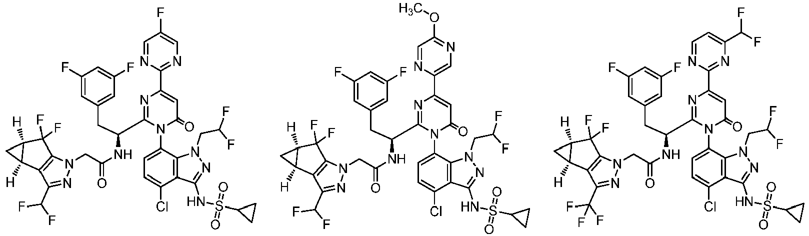 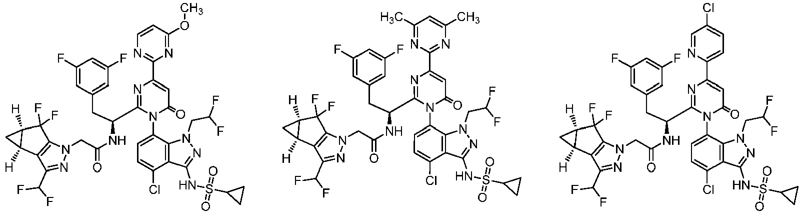 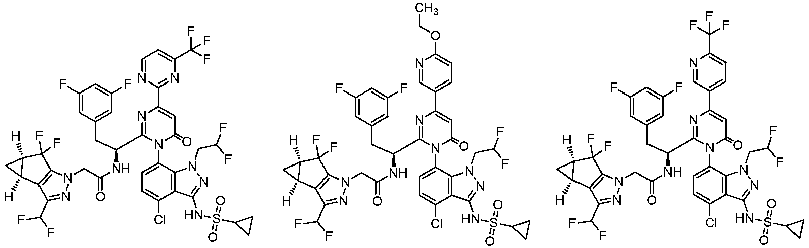 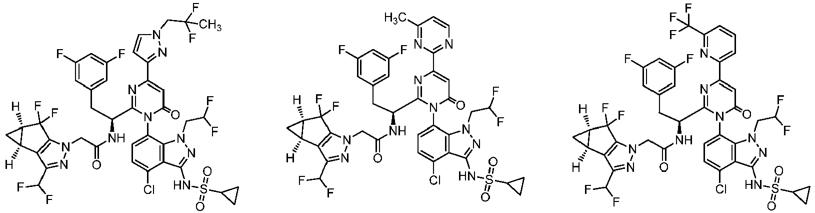 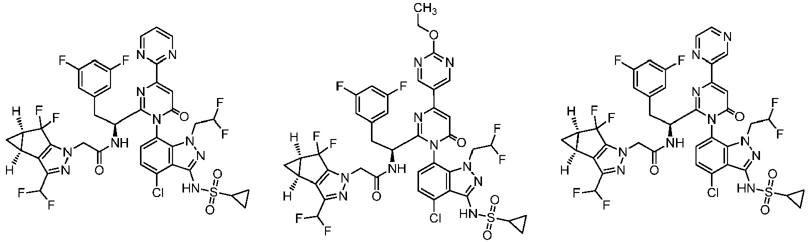 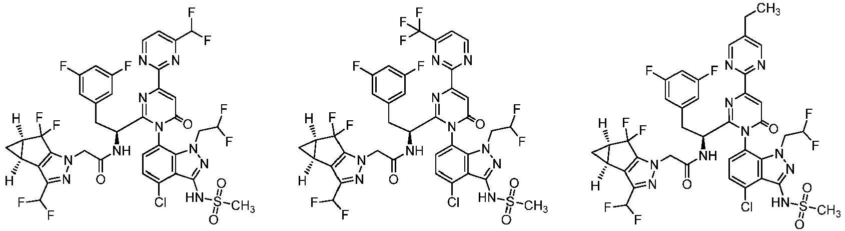 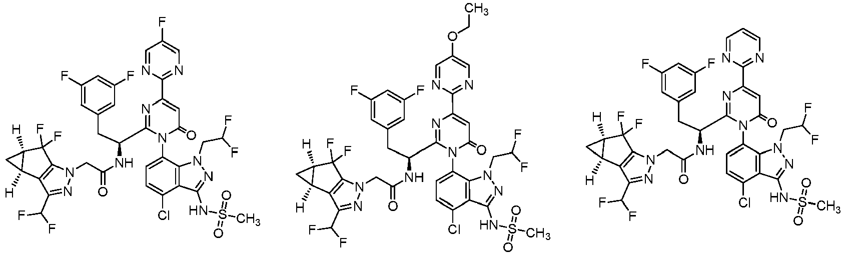 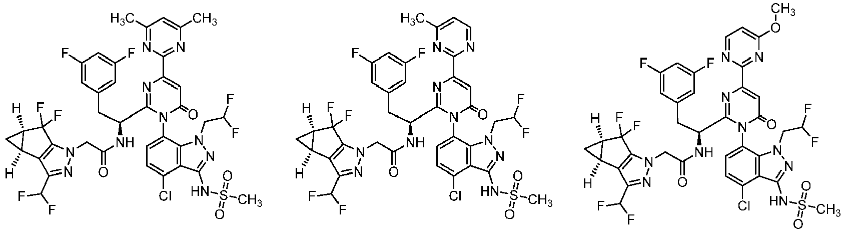 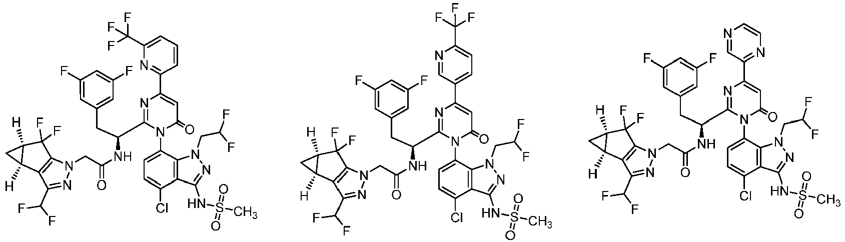 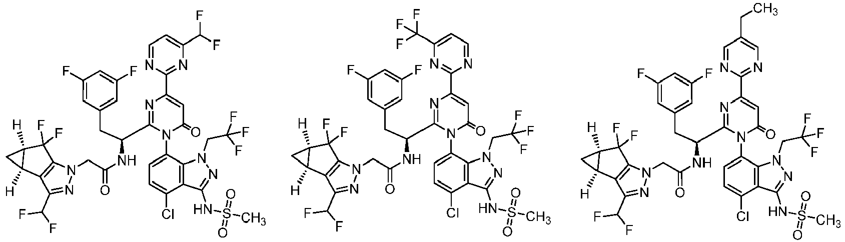 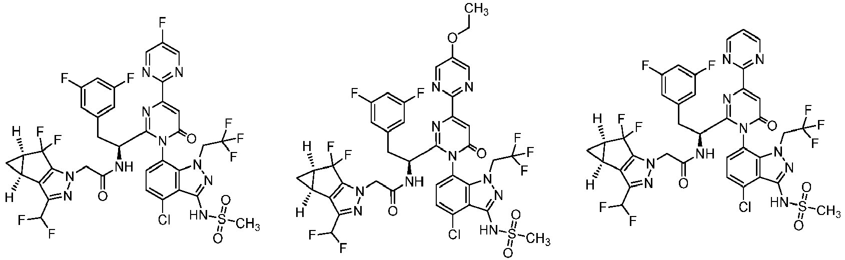 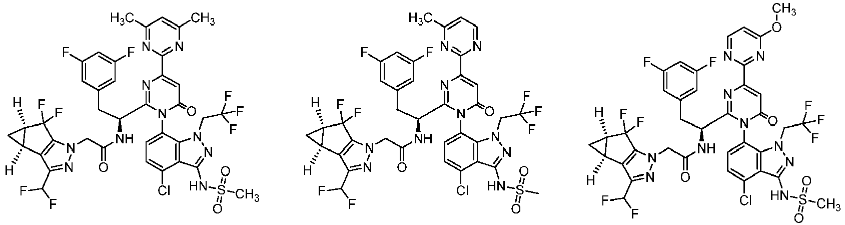 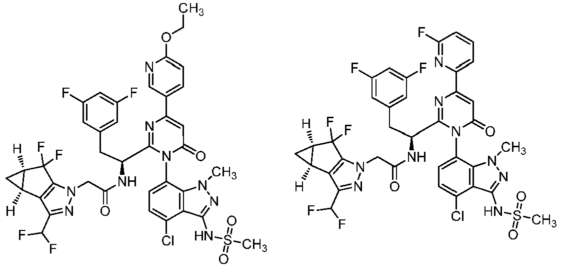 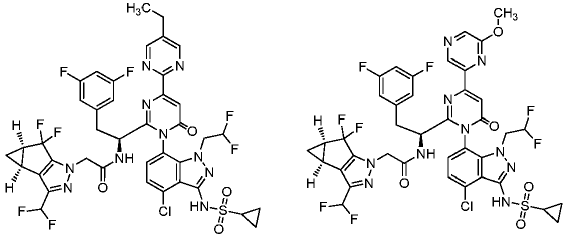 and pharmaceutically acceptable salts thereof.

In one embodiment, the present invention discloses compounds and salts, selected from the group consisting of: 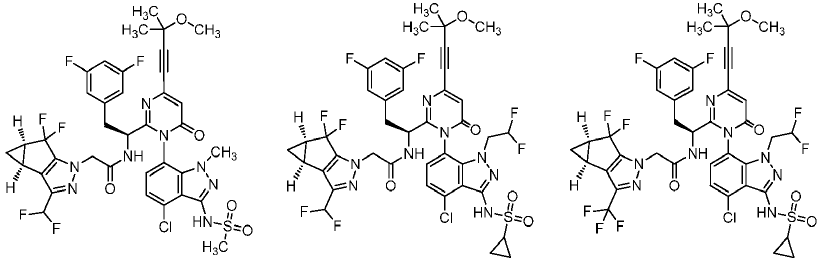 and pharmaceutically acceptable salts thereof.

In one embodiment, the present invention discloses compounds and salts, selected from the group consisting of: 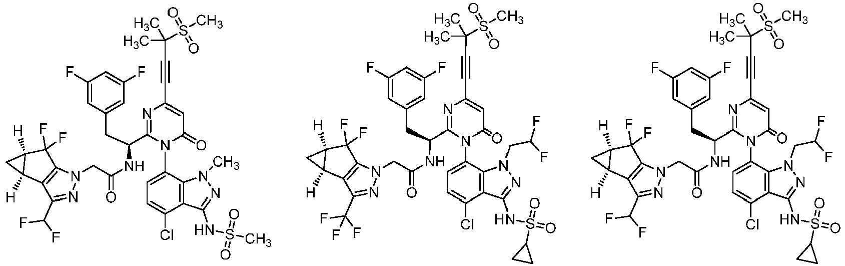 and pharmaceutically acceptable salts thereof.

In one embodiment, the present invention discloses the compound: 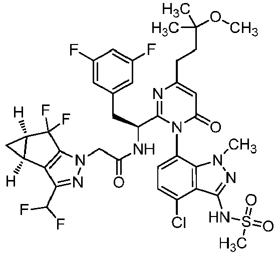 and pharmaceutically acceptable salts thereof.

The salts of the invention are pharmaceutically acceptable. Such salts may be acid addition salts or base addition salts. For a review of suitable pharmaceutically acceptable salts see, for example, Berge et al, J. Pharm, Sci., 66, 1-19, 1977.

Representative pharmaceutically acceptable acid addition salts include, but are not limited to, 4-acetamidobenzoate, acetate, adipate, alginate, ascorbate, aspartate, benzenesulfonate (besylate), benzoate, bisulfate, bitartrate, butyrate, calcium edetate, camphorate, camphorsulfonate (camsylate), caprate (decanoate), caproate (hexanoate), caprylate (octanoate), cinnamate, citrate, cyclamate, digluconate, 2,5-dihydroxybenzoate, disuccinate, dodecylsulfate (estolate), edetate (ethylenediaminetetraacetate), estolate (lauryl sulfate), ethane-1,2-disulfonate (edisylate), ethanesulfonate (esylate), formate, fumarate, galactarate (mucate), gentisate (2,5-dihydroxybenzoate), glucoheptonate (gluceptate), gluconate, glucuronate, glutamate, glutarate, glycerophosphorate, glycolate, hexylresorcinate, hippurate, hydrabamine (*N*,*N*'-di(dehydroabietyl)-ethylenediamine), hydrobromide, hydrochloride, hydroiodide, hydroxynaphthoate, isobutyrate, lactate, lactobionate, laurate, malate, maleate, malonate, mandelate, methanesulfonate (mesylate), methylsulfate, mucate, naphthalene-1,5-disulfonate (napadisylate), naphthalene-2-sulfonate (napsylate), nicotinate, nitrate, oleate, palmitate, *p*-aminobenzenesulfonate, *p*-aminosalicyclate, pamoate (embonate), pantothenate, pectinate, persulfate, phenylacetate, phenylethylbarbiturate, phosphate, polygalacturonate, propionate, *p*-toluenesulfonate (tosylate), pyroglutamate, pyruvate, salicylate, sebacate, stearate, subacetate, succinate, sulfamate, sulfate, tannate, tartrate, teoclate (8-chlorotheophyllinate), thiocyanate, triethiodide, undecanoate, undecylenate, and valerate.

Representative pharmaceutically acceptable base addition salts include, but are not limited to, aluminium, 2-amino-2-(hydroxymethyl)-1,3-propanediol (TRIS, tromethamine), arginine, benethamine (/V-benzylphenethylamine), benzathine (*N*,*N'-*dibenzylethylenediamine), *bis*-(2-hydroxyethyl)amine, bismuth, calcium, chloroprocaine, choline, clemizole (1-*p* chlorobenzyl-2-pyrrolildine-1'-ylmethylbenzimidazole), cyclohexylamine, dibenzylethylenediamine, diethylamine, diethyltriamine, dimethylamine, dimethylethanolamine, dopamine, ethanolamine, ethylenediamine, L-histidine, iron, isoquinoline, lepidine, lithium, lysine, magnesium, meglumine (*N*-methylglucamine), piperazine, piperidine, potassium, procaine, quinine, quinoline, sodium, strontium, *t*-butylamine, and zinc.

In one embodiment, the compositions of this invention further comprise a pharmaceutically acceptable excipient. In the method of this invention, preferred routes of administration are oral and by injection to deliver subcutaneously or intramuscularly. Therefore, preferred pharmaceutical compositions include compositions suitable for oral administration (for example tablets) and compositions suitable for subcutaneous or intramuscular injection.

Also disclosed are methods of preventing HIV infection in a human or reducing the risk of infection, comprising administering a compound or salt of this invention. Pre-exposure prophylaxis (or PrEP) is when people at risk for HIV infection take daily medicine to lower their chances of getting HIV infection. PrEP has been shown to be effective in reducing the risk of infection.

The compounds and salts of this invention are believed to have as their biological target the HIV capsid and thus their mechanism of action is to modify in one or more ways the function of the HIV capsid. As used herein, "HIV" or "Human Immunodeficiency Virus" refers to HIV-1 and/or to HIV-2.

The compounds and salts of the present invention may be employed alone or in combination with other therapeutic agents. Combination therapies according to the present invention thus comprise the administration of at least one compound or salt of the invention, and the administration of at least one other agent which may be useful in the treatment of HIV infection. A compound or salt of the present invention, and the other agent may be formulated and administered together in a single pharmaceutical composition or may be formulated and administered separately. When formulated and administered separately, administration may occur simultaneously or sequentially in any order. Suitable other agents include, for example, abacavir, atazanavir, bictegravir, cabotegravir, darunavir, delavirdine, didanosine, dideoxyinosine, dolutegravir, doravirine, efavirenz, elvitegravir, emtricitabine, etavirine, fosamprenavir, fostemsavir, GSK3640254, the antibody N6LS, GSK3739937/VH3739937 and GSK4000422/VH4000422, indinavir, lamivudine, lopinavir, maraviroc, nelfinavir, nevirapine, raltegravir, rilpiverine, ritonavir, saquinavir, slatravir, stavudine, tipranavir, tenofovir, tenofovir alafenamide, tenofovir disoproxil fumarate, zalcitabine, zidovudine, and S-648414. Preferred agents include, for example, bictegravir, cabotegravir, dolutegravir, fostemsavir, islatravir, and lamivudine. Particularly preferred agents include, for example, bictegravir, cabotegravir, dolutegravir, fostemsavir, and lamivudine.

### EXAMPLES

### LCMS Method A:

Column = Acquity BEH C18, 2.1 × 30 mm, 1.7 µm particles; Solvent A = 0.1% Formic acid in 100% Water; Solvent B = 0.1% Formic Acid in 100% Acetonitrile; Flow Rate = 0.8 mL/min.; Start % B = 5; Final % B = 95; Gradient Time = 1.7 min, then a 0.2 min hold at 95% B; Wavelength = 215 and 254 nm; ESI+ Range: 150 to 1500 Dalton; System: Agilent 1290 Infinity II.

### LCMS Method B:

Column = Acquity UPLC BEH C18, 2.1 × 100 mm, 1.7 µm particles; Solvent A = 0.1% Formic acid in 95:5 Water:MeCN; Solvent B = 0.1% Formic Acid in 5:95 Water:MeCN; Flow Rate = 0.8 mL/min; Start % B = 0; Final % B = 100; Gradient Time = 3.5 min, then a 2 min hold at 100% B; Wavelength = 220 and 254 nm.

### LCMS Method C:

Column = Acquity UPLC BEH C18, 2.1 × 100 mm, 1.7 µm particles; Solvent A = 0.1% Formic acid in 95:5 Water:MeCN; Solvent B = 0.1% Formic Acid in 5:95 Water:MeCN; Flow Rate = 0.8 mL/min; Start % B = 50; Final % B = 100; Gradient Time = 3.5 min, then a 2 min hold at 100% B; Wavelength = 220 and 254 nm.

### HPLC Purification:

HPLC purification was performed following the general method described below: Column = Waters XSelect CSH C18, 19 x 100 mm, 5 µm particles; Solvent A = 0.1% Formic Acid in 100% Water; Solvent B = Acetonitrile; Flow Rate = 40 mL/min.; Wavelength = 215 and 254 nm; ESI+ Range: 150 to 2000 Dalton. The column was eluted with a gradient. The start %B and ending %B were optimized for each compound. Generally, the gradient ran for 6 minutes followed by a 2 minute hold at 98% B.

### Preparation of bicyclo[3.1.0]hexan-3-ol

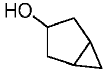

To a stirred solution of cyclopent-3-enol (130 g, 1545 mmol) in DCM (1200 mL) under N₂ atmosphere at 0-5 °C was added dropwise a solution of diethyl zinc in hexane (1.0 M, 3091 mL, 3091 mmol) over a period of 3 h. To the solution at 0 °C was added dropwise a solution of diiodomethane (249 mL, 3091 mmol) in DCM (300 mL) over a period of 1h. The reaction mixture was allowed to warm to 27 °C upon which formation of a white precipitation was observed. The mixture stirred for 16 h. Progress of the reaction was monitored by TLC (SiO₂, 20% EtOAc/pet, Rf = 0.3, UV-inactive, PMA-active). The reaction mixture was quenched via the careful addition of aq. saturated NH₄Cl solution (1.5 L). The mixture was filtered through pad of Celite. The aqueous layer was extracted with DCM (2 × 1L). The combined organic layers were dried over anhydrous Na₂SO₄, filtered and then concentrated under reduced pressure to afford crude bicyclo[3.1.0]hexan-3-ol as red liquid, 180 g. ¹H NMR (400 MHz, CDCl₃) δ = 4.41 - 4.35 (m, 1H), 2.18 - 2.05 (m, 2H), 1.73 (d, *J* = 13.9 Hz, 2H), 1.35 - 1.25 (m, 2H), 1.21 - 1.14 (m, 1H), 0.57 - 0.43 (m, 2H). GCMS: m/z = 98.1).

### Preparation of bicyclo[3.1.0]hexan-3-one

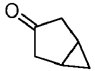

To a stirred solution of bicyclo[3.1.0]hexan-3-ol (210 g, 2054 mmol) in DCM (5000 mL) under N₂ atmosphere at 0 °C was added portion-wise Dess-Martin periodinane (954 g, 225 mmol). The mixture was allowed to warm to 27 °C and was then stirred for 16 h. Progress of the reaction was monitored by TLC (SiO₂, 20% Acetone/Hex, Rf = 0.3, UV inactive, PMA-active). The reaction mixture was filtered through pad of Celite and the filtrate was washed with aq. NaOH (1N, 8× 1 L). The combined aqueous phases were extracted with DCM (5 × 1 L). The combined organic layers were dried over anhydrous Na₂SO₄, filtered, and then concentrated under reduced pressure (bath temperature: 20 °C) to afford crude bicyclo[3.1.0]hexan-3-one as brown liquid. The liquid was further purified by downward distillation at 70 °Cto afford bicyclo[3.1.0]hexan-3-one as a pale yellow viscous liquid, 125 g (62%). ¹H NMR (400 MHz, CDCl₃) δ = 2.61 - 2.54 (m, 2H), 2.17 - 2.12 (m, 2H), 1.54 - 1.46 (m, 2H), 0.92 - 0.86 (m, 1H), -0.01 - -0.08 (m, 1H); GCMS: M/Z = 96.1.

### Preparation of 2-(2,2-difluoroacetyl)bicyclo[3.1.0]hexan-3-one

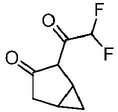

To a stirred solution of bicyclo[3.1.0]hexan-3-one (125 g, 1274 mmol) in THF (1500 mL) under N₂ atmosphere at -78 °C was added LDA (2.0 M in THF, 0.701 L, 1402 mmol). The solution was stirred for 1 h at -78 °C. To the solution was added slowly over 30 minutes a solution of ethyldifluoroacetate (174 g, 1402 mmol) in THF (300 mL) maintaining a temperature of -78 °C. The reaction mixture was allowed to warm to 27 °C and was then stirred for 1 h. Progress of the reaction was monitored by TLC (SiO₂, 20% Acetone/Hexane, Rf = 0.3, UV -active). The reaction mixture was quenched via the addition of aq. HCl (1N, 2000 mL). The mixture was stirred for 30 min. and then was extracted with EtOAc (3 × 1000 mL). The combined organic layers were washed with brine (1000 mL), dried over anhydrous Na₂SO₄ and filtered. The filtrate was concentrated under reduced pressure to afford 2-(2,2-difluoroacetyl)bicyclo[3.1.0]hexan-3-one as a pale yellow viscous liquid, 180 g (71%). ¹H NMR (400 MHz, CDCl₃) δ = 6.18 (t, *J* = 54.8 Hz, 1H), 2.70 - 2.62 (m, 1H), 2.35 (d, *J* = 19.4 Hz, 1H), 2.14 (br s, 1H), 1.26 - 1.21 (m, 1H), 1.04-1.03 (m, 1H), 0.22-0.21 (m, 1H), LCMS: M/Z = 173.17).

### Preparation of ethyl 2-(3-(difluoromethyl)-3b,4,4a,5-tetrahydro-1H-cyclopropa[3,4]cyclopenta[1,2-c]pyrazol-1-yl)acetate

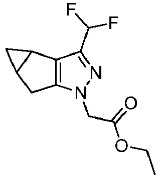

To a stirred solution of 2-(2,2-difluoroacetyl)bicyclo[3.1.0]hexan-3-one (180 g, 910 mmol) in ethanol (2 L) under N₂ atmosphere at 27 °C was added ethyl 2-hydrazinylacetate hydrochloride (422 g, 2729 mmol) followed by sulfuric acid (20 mL, 375 mmol). The mixture was stirred for 30 min. and then was heated to 100 °C and stirred for 16 h. Progress of the reaction was monitored by TLC (SiO₂, 20% Acetone/Hexane, Rf = 0.3, UV-active). The reaction mixture was concentrated under reduced pressure. The residue was dissolved in EtOAc (2000 mL) and was washed with water (2 × 1 L), brine (1.0 L), dried over anhydrous Na₂SO₄, filtered, and then was concentrated under reduced pressure. The resulting residue was subjected to silica gel column chromatography (pet.:acetone 100:0→98:2) to afford ethyl 2-(3-(difluoromethyl)-3b,4,4a,5-tetrahydro-1H-cyclopropa[3,4]cyclopenta[1,2-c]pyrazol-1-yl)acetate as an off-white solid, 110 g (46%). ¹H NMR (400 MHz, DMSO-d₆) δ = 6.86 (t, *J* = 54.8 Hz, 1H), 4.93 (s, 2H), 4.14 (q, *J* = 7.2 Hz, 2H), 2.88 - 2.79 (m, 1H), 2.76 - 2.68 (m, 1H), 2.14 - 2.04 (m, 2H), 1.19 (t, *J* = 7.2 Hz, 3H), 1.10 - 1.03 (m, 1H), 0.14 (q, *J* = 4.3 Hz, 1H).

### Preparation of ethyl 2-(3-(difluoromethyl)-5oxo-3b,4,4a,5-tetrahydro-1H-cyclopropa[3,4]cyclopenta[1,2-c]pyrazol-1-y/)acetate.

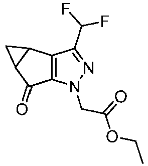

To a stirred solution of ethyl 2-(3-(difluoromethyl)-3b,4,4a,5-tetrahydro-1H-cyclopropa[3,4]cyclopenta[1,2-c]pyrazol-1-yl)acetate (110 g, 422 mmol) and Celite (395 g) in cyclohexane (3.5 L) at 0 °C was added portion wise pyridinium dichromate (794 g, 2110 mmol). To the mixture under nitrogen atmosphere was added dropwise tert-butyl hydroperoxide (355 mL, 2130 mmol) over a period of 10 min. The reaction mixture was warmed to 27 °C and was then stirred at that temperature for 48 h. Progress of the reaction was monitored by TLC (SiO₂, 30% Acetone/pet, Rf = 0.4, UV -active). The reaction mixture was filtered, and the filter cake was extracted with EtOAc (1000 mL). The filtrate was washed with saturated aq. Na₂S₂O₃ (2×500 mL); saturated aq. FeSO₄ (300 mL); and then brine (500 mL). The organic layer was dried over anhydrous Na₂SO₄,; filtered and concentrated under reduced pressure to obtain the crude title compound (150 g).

### Preparation of ethyl 2-(3-(ditluoromethyl)-4,4a-dihydrospiro[cyclopropa[3,4]cyclopenta[1,2-c]pyrazole-5,2'-[1,3]dithiolane]-1(3bH)-y/)acetate.

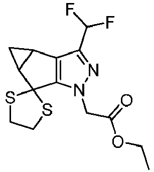

To a stirred solution of ethyl 2-(3-(difluoromethyl)-5-oxo-3b,4,4a,5-tetrahydro-1H-cyclopropa[3,4]cyclopenta[1,2-c]pyrazol-1-yl)acetate (75 g, 269 mmol) in DCM (1500 mL) at 27 °C under nitrogen atmosphere was added ethane-1,2-dithiol (43.0 mL, 511 mmol) followed by the addition of boron trifluoride acetic acid (72.6 mL, 511 mmol). The solution was stirred for 16 h. Progress of the reaction was monitored by TLC (SiO₂, 20% Acetone/Pet, Rf = 0.35, UV -Active). After completion, the reaction mixture was cooled to 0 °C and quenched via the addition of aq. saturated NaHCO₃ (500 mL). The mixture was extracted with DCM (2 × 1000 mL). The combined organics were washed with brine (1000 mL), dried over anhydrous Na₂SO₄, filtered and concentrated under reduced pressure to obtain a brown liquid. This material was subjected to silica gel column chromatography (Pet.:EtOAc 95:5→90:10) to afford ethyl 2-(3-(difluoromethyl)-4,4a-dihydrospiro[cyclopropa[3,4]cyclopenta[1,2-c]pyrazole-5,2'-[1,3]dithiolane]-1(3bH)-yl)acetate as an off-white solid, 80 g (74%). ¹H-NMR (400 MHz, CDCl₃) δ = 6.61 (t, *J* = 55.2 Hz, 1H), 5.00 - 4.85 (m, 2H), 4.29 - 4.19 (m, 2H), 3.55 - 3.46 (m, 4H), 2.63 - 2.53 (m, 1H), 2.49 - 2.38 (m, 1H), 1.30 - 1.24 (m, 4H), 0.65 - 0.60 (m, 1H). LCMS M+H = 346.9.

### Preparation of ethyl 2-(3-(difluoromethyl)-5,5-difluoro-3b,4,4a,5-tetrahydro-1H-cyclopropa[3,4]cyclopenta[1,2-c]pyrazol-1-y/)acetate

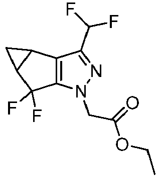

To a stirred solution of 1,3-dibromo-5,5-dimethylimidazolidine-2,4-dione (26.3 g, 92 mmol) in DCM (20 mL) at -70 °C under N₂ atmosphere was added HF-pyridine (2.460 g, 24.83 mmol). The solution was for 30 min. To the solution was added a solution of ethyl 2-(3-(difluoromethyl)-4,4a-dihydrospiro[cyclopropa[3,4]cyclopenta[1,2-c]pyrazole-5,2'-1,3]dithiolane]-1(3bH)-yl)acetate (10 g, 25 mmol) in DCM (20 mL). The reaction mixture was allowed to warm to -40 °C and then was stirred at that temperature for 1 h. Progress of the reaction was monitored by TLC (SiO₂, 30% EtOAc/Pet, Rf = 0.3, UV in-active). The reaction mixture was quenched via the addition of aq. sat. NaHCO₃ (200 mL). The mixture was warmed to room temperature and was then extracted with EtOAc (2 × 100 mL). The combined organics were washed with brine (50 mL); dried over anhydrous Na₂SO₄; filtered; and were concentrated under reduced pressure to afford a brown solid. This material was subjected to silica gel column chromatography (Pet.:EtOAc 100:0→75-25) to afford ethyl 2-(3-(difluoromethyl)-5,5-difluoro-3b,4,4a,5-tetrahydro-1H-cyclopropa[3,4]cyclopenta[1,2-c]pyrazol-1-yl)acetate as a pale yellow solid, 8.5 g (91%). ¹H NMR (400 MHz, CDCl₃) δ = 6.62 (t, J = 55.2 Hz, 1H), 4.82 (s, 2H), 4.30 - 4.18 (m, 2H), 2.51 - 2.37 (m, 2H), 1.42 - 1.35 (m, 1H), 1.31 - 1.23 (m, 3H), 1.14 - 1.08 (m, 1H). LCMS M+H = 293.07.

### Preparation of 2-(3-(difluoromethyl)-5,5-difluoro-3b,4,4a,5-tetrahydro-1H-cyclopropa[3,4]cyclopenta[1,2-c]pyrazol-1-yl)acetic acid

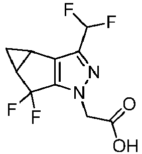

To a stirred solution of ethyl 2-(3-(difluoromethyl)-5,5-difluoro-3b,4,4a,5-tetrahydro-1H-cyclopropa[3,4]cyclopenta[1,2-c]pyrazol-1-yl)acetate (15 g, 50 mmol) in THF (17 mL) and MeOH (66 mL) at 0 °C under N₂ atmosphere was added a solution of LiOH (1.788 g, 74.7 mmol) in water (66 mL). The reaction mixture was allowed to warm to 27 °C and was then stirred for 3 h at that temperature. Progress of the reaction was monitored by TLC (SiO₂, 5% MeOH/DCM, Rf = 0.2, UV Active). After completion, the reaction mixture was concentrated under reduced pressure; diluted with water (50 mL); and washed with EtOAc (2 × 250 mL) to remove impurities. The aqueous layer was adjusted to pH 2-3 using aq. HCl (1M), then was extracted with EtOAc (3 × 1000 mL). The combined organics were dried over anhydrous Na₂SO₄; filtered; and concentrated under reduced pressure to afford 2-(3-(difluoromethyl)-5,5-difluoro-3b,4,4a,5-tetrahydro-1H-cyclopropa[3,4]cyclopenta[1,2-c]pyrazol-1-yl)acetic acid as an off white solid, 14 g (98%). LCMS M+H = 265.15.

### Separation affording 2-((3bS,4aR)-3-(difluoromethyi)-5,5-difluoro-3b,4,4a,5-tetrahydro-1H-cyclopropa[3,4]cyclopenta[1,2-c]pyrazol-1-yl)acetic acid and 2-((36R,4aS)3-(difluoromethyl)-5,5-difluoro-3b,4,4a,5-tetrahydro-1H-cyclopropa[3,4]cyclopenta[1,2-c]pyrazol-1-yl)acetic acid

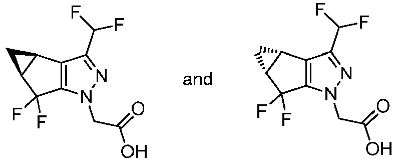

2-(3-(difluoromethyl)-5,5-difluoro-3b,4,4a,5-tetrahydro-1H-cyclopropa[3,4]cyclopenta[1,2-c]pyrazol-1-yl)acetic acid (5.5 g) was dissolved in isopropanol (20 mL). The solution was subjected portion-wise to SFC chiral separation as follows: Instrument = Thar 80; column = Chiralpak IC 30×250mm, 5 micron; solvent A = super critical CO₂; solvent B = isopropanol with 0.5% isopropylamine (v/v); eluent composition = 70%A:30%B; flow-rate = 65 g/min; back-pressure = 100 bar; temperature = 30 °C; injection volume = 2.5 mL; detection = 220 nm. 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid was collected as peak eluting from 7.5 min. to 14 min; 2-((3bR,4aS)-3-(difluoromethyl)-5,5-difluoro-3b,4,4a,5-tetrahydro-1H-cyclopropa[3,4]cyclopenta[1,2-c]pyrazol-1-yl)acetic acid was collected as a peak eluting from 2.7 min. to 5.8 min. For each enantiomer, the resulting solution was concentrated under reduced pressure and the resulting solids were dissolved in EtOAc, then twice washed with aq. citric acid (1M) followed by water followed by brine. The organic solution was dried over Na₂SO₄; filtered; then concentrated in vacuo to afford the separated enantiomer in 80-90% recovery.

### Preparation of N-(7-amino-4-chloro-1-methyl-1H-indazol-3-yl)-N-(4-methoxybenzyl)methanesulfonamide.

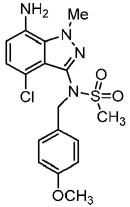

### Synthesis Scheme:

### Step 1: Preparation of 2,6-dichloro-3-nitrobenzaldehyde

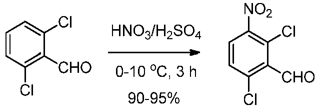

To a solution of sulfuric acid (H₂SO₄) (5.63 L, 4.5 V) in a round-bottom flask at 0-5 °C was added 2,6-dichlorobenzaldehyde (1.25 kg, 7.10 mol, 1.0 equiv.) in portions at below 15 °C. The reaction mass was stirred at 0-5 °C for 30 min. A solution of freshly prepared nitration mixture [Prepared from Conc. H₂SO₄ (0.425 L, 0.34 V) and 70% HNO₃ (0.85 kg, 13.49 mol, 1.30 equiv.) at 0 °C] was added to the above reaction mixture at below 10 °C **[Note:** Reaction is slightly exothermic (3-6 °C); so that addition is preferred at lower temperature]. The reaction mixture was stirred at 5-10 °C for 2-3 h. After completion of the reaction (monitored by TLC), it was quenched with ice cold water (18.75 L, 15 V) at below 25 °C. Then the reaction mass was allowed warm to room temperature and stirred for 2 h. The solids were isolated by filtration and then were washed with water (2.5 L, 2.0 V). Bulk residual water was removed from the solids by maintaining vacuum filtration for 60-90 min. The crude wet solid was initially dried under air atmosphere; then in a hot air oven at 50-55 °C for 10-12 h (until moisture content is not more than 5.0 %) to get the dried title product, 2,6-dichloro-3-nitrobenzaldehyde (1.44 kg, 92% yield) as a yellow solid. ¹H NMR (400 MHz, CDCl₃): *δ*10. 44 (s, 1H), 7.88 (d, *J* = 8.4 Hz, 1H), 7.56 (d, *J* = 8.8 Hz, 1H).

### Step 2: Preparation of 2,6-dichloro-3-nitrobenzonitrile

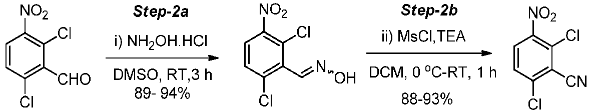

(Step-2a) To a solution of DMSO (5.9 L, 5.0 V)) in a round-bottom flask was added 2,6-dichloro-3-nitrobenzaldehyde (1.17 kg, 5.31 mol, 1.0 equiv.) at room temperature. After being stirred for 30 min at room temperature, hydroxylamine hydrochloride (0.63 kg, 9.04 mol, 1.70 equiv.) was added and the reaction mass was stirred at room temperature for 3 h. After completion of the reaction (monitored by TLC), the reaction mass was quenched by the addition of ice-cold water (18.0 L, 15.0 V) added at a rate sufficient to maintain the temperature below 30 °C (Observation: Solids formed upon water addition). The reaction mass was stirred at room temperature for 60-90 min. The solids were isolated by filtration; washed with water (2.5 L, 2.0 V); followed by washing with a mixture of acetone and hexanes (6.0 L, 1:1 ratio). Bulk residual water was removed from the solids by maintaining vacuum filtration for 60-90 min. The wet solid was initially air dried and then finally dried in a hot air oven at 50-55 °C for 10-12 h (until moisture content was not more than 1.0 %) to get the dried target product, 2,6-dichloro-3-nitrobenzaldehyde oxime (1.22 kg, 92% yield) as an off-white solid. The crude product (which contains 10-20% of 2,6-dichloro-3-nitrobenzonitrile) was used directly in the next step without further purification.

(Step-2b) To a stirred solution of the crude oxime (preparation described above, 1.13 kg, 4.80 mol, 1.0 equiv.) in DCM (9.04 L, 8.0 V) at 0-5 °C was added triethylamine ("TEA", 1.02 kg, 10.09 mol, 2.1 equiv.). After being stirred for 5 min, methanesulfonyl chloride (0.60 kg, 5.29 mol, 1.1 equiv.) was added (Observation: An exotherm is noted during the addition) slowly at 15 °C. Then the reaction mass was stirred at room temperature for 30-45 min. After completion of the reaction (progress of reaction was monitored by TLC; mobile phase: 20% ethyl acetate in hexanes), the reaction mass was diluted with water (6.78 L, 6.0 V); the organic layer was separated; and the aqueous layer was extracted with DCM (3.4 L, 3.0 V). The combined organic layers were washed with brine (5.65 L, 5.0 V); dried over Na₂SO₄; and concentrated under vacuum. The resulting crude solids were triturated with hexanes (4.50 L, 4.0 V) at room temperature. The wet material was dried in a hot air oven at 50-55 °C for 5- 6 h to get the dried product, 2,6-dichloro-3-nitrobenzonitrile (0.95 kg, 91% yield) as a yellow solid. ¹H NMR (400 MHz, CDCl₃): *δ*8.07 (d, *J* = 8.8 Hz, 1H), 7.63 (d, *J* = 8.8 Hz, 1H).

### Step 3: Preparation of 4-chloro-7-nitro-1H-indazol-3-amine

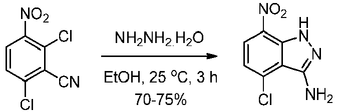

To a stirred solution of 2,6-dichloro-3-nitrobenzonitrile (750.0 g, 3.45 mol, 1.0 equiv.) in ethanol (7.5 L, 10.0 V) at 15-20 °C. was slowly added hydrazine hydrate (519.0 g, 10.36 mol, 3.0 equiv.) while maintaining the reaction mass below 25 °C (Observation: Addition is slightly exothermic and solid formation will begin upon addition). The reaction mixture temperature was slowly raised to room temperature and then the mixture was stirred for 3 h (Observation: the quantity of solids will increase during this time). After completion of the reaction (monitored by TLC), the mixture was diluted with water (7.5 L, 10.0 V) and further stirred for 1 h at room temperature. The solids were isolated via filtration and then were washed with water (2.25 L, 3.0 V). The wet solid was washed with a 1:1 ratio mixture of acetone (1.875 L, 2.5 V) and hexanes (1.875 L, 2.5 V). Bulk residual water was removed from the solids by maintaining vacuum filtration for 60-90 min. The wet solid was finally dried in a hot air oven for 7-8 h at 50 °C (until moisture content reaches below 1.5%) to get the dried product, 4-chloro-7-nitro-1*H*-indazol-3-amine (549.0 g, 75% yield) as a brick red-colored solid. ¹H NMR (400 MHz, CDCl₃): *δ*10.36 (bs, 1H), 8.20 (d, *J* = 8.4 Hz, 1H), 7.07 (d, *J* = 8.40 Hz, 1H), 4.73 (bs, 2H).

### Step 4: Preparation of 4-chloro-1-methyl-7-nitro-1Hindazol-3-amine

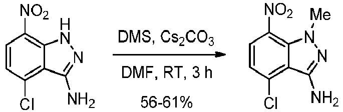

To a stirred solution of 4-chloro-7-nitro-1*H*-indazol-3-amine (500 g, 0.42 mol, 1.0 equiv.) in DMF (5.0 L, 10.0 V) at 5-10 °C was slowly added cesium carbonate (Cs₂CO₃) (1.91 kg, 5.88 mol, 2.5 equiv.) while maintaining the reaction mass below 10 °C. After being stirred for 5-10 min, dimethyl sulphate (326.3 g, 2.59 mol, 1.1 equiv.) was added while maintaining the reaction mass below 10 °C (Note: Slow addition is preferred for obtaining more favorable regio-selectivity). Then, the reaction temperature was slowly raised to room temperature and stirring was continued an additional 2 h at the same temperature. After completion of the reaction (monitored by TLC), the reaction mass was quenched by the addition of ice-cold water (15.0 L, 30.0 V) and the resulting mixture was then stirred for 6-8 h at room temperature. The solids were isolated via filtration and were then washed with water (1.5 L, 3.0 V). The wet solid was washed with IPA (1.5 L, 3.0 V) followed by hexanes (1.0 L, 2.0 V). Bulk residual water was removed from the solids by maintaining vacuum filtration for 60-90 min. The wet solid was dried in a hot air oven for 7-8 h at 50 °C (until moisture content is below 1.0%). The isolated material, 4-chloro-1-methyl-7-nitro-1*H-*indazol-3-amine (319.0 g, 60% yield), was used in the next step without further purification. ¹H NMR (400 MHz, CDCl₃): *δ*7.97 (d, *J* = 8.32 Hz, 1H), 6.97 (d, *J* = 8.24 Hz, 1H), 4.63 (bs, 2H), 3.96 (s, 3H).

### Step 5: Preparation of N-(4-chloro-l-methyl-7-nitro-1H-indazol-3-yl)methanesulfonamide

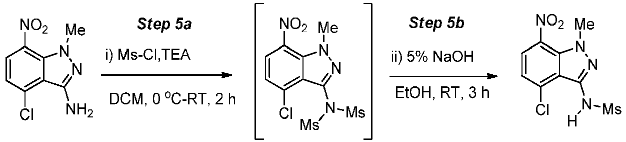

(Step 5a) To a solution of 4-chloro-1-methyl-7-nitro-1*H-*indazol-3-amine (625.0 g, 2.76 mol, 1.0 equiv.) in DCM (6.25 L, 10.0 V) at 0-5 °C. was added triethylamine (TEA) (837.0 g, 8.27 mol, 3.0 equiv.); followed by the addition of 4-dimethylaminopyridine (DMAP) (20.60 g, 0.165 mol, 0.06 equiv.). The reaction mass was stirred for 5-10 min., then methanesulfonyl chloride (MsCI) (790.0 g, 6.89 mol, 2.5 equiv.) added slowly while maintaining the reaction mass below 10 °C. The reaction mixture was allowed to warm to room temperature and was then stirred for 1.5-2.0 h. After completion of the reaction (monitored by TLC), the mixture was diluted with water (6.25 L, 10.0 V) and then stirred at room temperature for 15 min. The organic layer was separated, and the aqueous layer was extracted with DCM (6.25 L, 10.0 V). The combined organic layers were washed with brine (1.25 L, 2.0 V), dried over Na₂SO₄ and concentrated to get the crude solids. The solids were triturated with hexanes (1.25 L, 2.0 V) at room temperature to obtain the intermediate, N-(4-chloro-1-methyl-7-nitro-1H-indazol-3-yl)-N-(methylsulfonyl)methanesulfonamide, which was used directly in the next step. (ii) To a stirred solution of N-(4-chloro-1-methyl-7-nitro-1H-indazol-3-yl)-N-(methylsulfonyl)methanesulfonamide (prepared above) in ethanol (10.5 L, 20.0 V) at room temperature was added slowly an aq. 5% NaOH solution (4.38 L, 7.0 V) [Note: Slow addition is preferred via dropping funnel]. The reaction mass was stirred at the same temperature for 3 h. After completion of the reaction (monitored by TLC) [Sample preparation for TLC analysis: ~1.0 ml of sample acidified with aq. 2.0 N HCl to reach the pH: 2-3, extract it with ethyl acetate and analyze the organic layer by TLC], the reaction mass was cooled to 0-5 °C and the pH was adjusted to 2-3 by the addition of aq. 2.0 N HCl (3.13 L, 5.0 V) while maintain the reaction temperature below 10 °C [Note: Precipitation occurred upon addition of HCl and increased with stirring]. The reaction mixture was warmed to room temperature and then stirred for 1.5-2.0 h. Solids obtained were isolated via filtration and were then washed with water (1.25 L, 2.0 V); followed by washing with hexanes (1.25 L, 2.0 V). Bulk residual water was removed from the solids by maintaining vacuum filtration for 60-90 min. The wet material was dried in a hot air oven at 50 °C for 6-7 h (Until the moisture content is below 1.0%) to get the dried product, *N*-(4-chloro-l-methyl-7-nitro-1*H*-indazol-3-yl)methanesulfonamide (640.0 g, 76%) as a yellow solid. ¹H NMR (400 MHz, CDCl₃): *δ* 8.05 (d, *J* = 8.32 Hz, 1H), 7.32 (bs, 1H), 7.17 (d, *J* = 8.28 Hz, 1H), 4.15 (s, 3H), 3.45 (s, 3H).

### Step 6: Preparation of N-(4-chloro-1-methyl-7-nitro-1H-indazol-3-yl)-N-(4-methoxybenzyl)methanesulfonamide

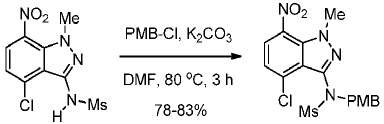

To a mixture of *N*-(4-chloro-l-methyl-7-nitro-1*H*-indazol-3-yl)methanesulfonamide (635.0 g, 2.08 mol, 1.0 equiv.) and 1-(chloromethyl)-4-methoxybenzene (359.0 g, 2.30 mol, 1.1 equiv.) in DMF (6.35 L, 10.0 V) at room temperature was added potassium carbonate (374.7 g, 2.70 mol, 1.3 equiv.). The reaction mixture was heated to 80-90 °C and maintained at that temperature for 3 h. After completion of the reaction (monitored by TLC), the mixture was poured into ice cold water (19.05 L, 30.0 V) [Note: Slow quenching with vigorous stirring is preferred to avoid clumping as the product precipitates]. The resulting solids were isolated via filtration and washed with water (1.90 L, 3.0 V); then the solids were washed with hexanes (1.27 L, 2.0 V). Bulk residual water was removed from the solids by maintaining vacuum filtration for 60-90 min. The isolated solid was dissolved in Ethyl acetate (12.7 L, 20.0 V) and charcoal was added (63.5 g). The mixture was heated to 60-70 °C and then stirred for 30-45 min. at that temperature. The mixture was filtered while still hot (40-50 °C) through a pad of Celite and the Celite pad was then extracted with ethyl acetate (3.17 L, 5.0 V). The combined filtrates were concentrated to dryness under reduced pressure at below 50 °C. Ethyl acetate (0.635 L, 1.0 V) was added to the solids at room temperature. The resultant solid suspension was stirred for 30 min. The solids were isolated via filtration and then were washed with hexanes (1.27 L, 2.0 V). Residual water was removed from the solids by maintaining vacuum filtration for 45-60 min. to afford the product *N-*(4-chloro-1-methyl-7-nitro-1*H*-indazol-3-yl)-*N*-(4-methoxybenzyl) methane sulfonamide (705.0 g, 80% yield) as a yellow solid. ¹H NMR (400 MHz, CDCl₃): *δ* 7.99 (d, *J* = 8.24 Hz, 1H), 7.27 (d, *J* = 8.68 Hz, 2H), 7.19 (d, *J* = 8.24 Hz, 1H), 6.80 (d, *J* = 8.44 Hz, 2H), 4.95-4.76 (m, 2H), 4.17 (s, 3H), 3.76 (s, 3H), 3.01 (s, 3H).

### Step 7: Preparation of N-(7-Amino-4-chloro-l-methyl-1H-indazol-3-yl)-N-(4-methoxybenzyl)methanesulfonamide

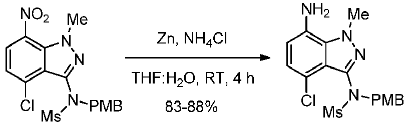

To a stirred suspension of zinc powder (540.0 g, 8.23 mol, 10.0 equiv.) in a mixture of THF (3.50 L, 10.0 V) and water (7.0 L, 20.0 V) at room temperature was added ammonium chloride (NH₄Cl) (449.0 g, 8.23 mol, 10.0 equiv.). To the mixture was added *N-*(4-chloro-1-methyl-7-nitro-1*H*-indazol-3-yl)-*N-*(4-methoxybenzyl)methanesulfonamide (350 g, 0.823 mol, 1.0 equiv.) in THF (7.0 L, 20.0 V). The reaction mixture was stirred at room temperature for 3-4 h. After completion of the reaction (monitored by in-process TLC/HPLC), the mixture was diluted with ethyl acetate (3.5 L, 10.0 V) and water (1.12 L, 2.5 V). The mixture was stirred for 15 min. The reaction mass was filtered through a pad of Celite bed washing with ethyl acetate (1.75 L, 5.0 V). The bi-phasic filtrate was collected, and the phases were separated. The aqueous layer was extracted with ethyl acetate (3.50 L, 10.0 V). The combined organic layers were washed with brine (3.50 L, 10 V), dried over Na₂SO₄, and then concentrated in *vacuo* to afford a crude solid. To the crude product was added MTBE (3.25 L, 10 V) and the suspension was stirred for 30 min at room temperature. The solids were isolated by filtration. Bulk residual water was removed from the solids by maintaining vacuum filtration for 30-45 min. The wet product was dried in a hot air oven (50 °C) for 2 h to afford the title product, *N-*(7-amino-4-chloro-1-methyl-1*H-*indazol-3-yl)-*N-*(4-methoxybenzyl)methanesulfonamide (276.0 g, 85% yield) as off-white solid. ¹H NMR (400 MHz, CDCl₃): *δ* 7.29-7.26 (m, 2H), 6.86-6.79 (m, 2H), 6.42 (d, *J* = 7.80 Hz, 1H), 4.99-4.70 (m, 2H), 4.25 (s, 3H), 3.77 (s, 5H), 2.98 (s, 3H).

### Preparation of N-(7-amino-4-chloro-1-(2,2-difluoroethyl)-1H-indazol-3-yl)-N-(4-methoxybenzyl)methanesulfonamide:

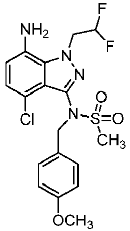

### Synthesis Scheme:

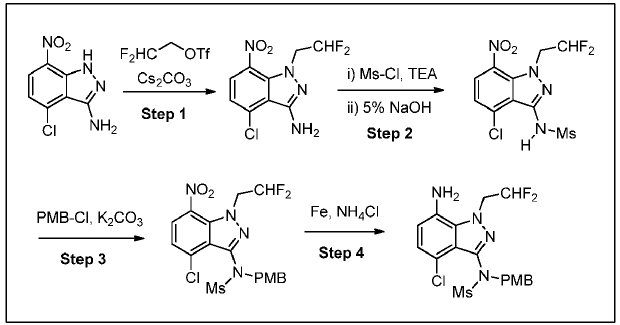

### Step 1: Preparation of 4-chloro-1-(2,2-difluoroethyl)-7-nitro-1H-indazol-3-amine

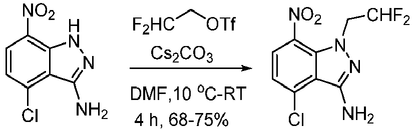

To a stirred solution of 4-chloro-7-nitro-1*H*-indazol-3-amine (180 g, 0.85 mol, 1.0 equiv.) in DMF (1.8 L, 10.0 V) at 10-15 °C was added cesium carbonate (Cs₂CO₃) (551 g, 1.70 mol, 2.0 equiv.) at a rate necessary to maintaining the reaction mass below 20 °C. The mixture was stirred for 5-10 min, then to the stirred mixture at 10-15 °C was added 2,2-difluoroethyl trifluoromethanesulfonate (133 mL, 0.93 mol, 1.1 equiv.) at a rate necessary to maintain the reaction mass below 20 °C (Note: Slow addition is preferred to obtain more favorable regio-selectivity). The reaction mass was allowed to slowly warm to room temperature and was then stirred at the same temperature for 3 h. After completion of the reaction (monitored by TLC), the reaction mass was quenched by the addition of ice-cold water (5.4 L, 30.0 V) and the resulting mixture was allowed to warm to room temperature with stirring for 6-8 h. The solids were isolated via filtration and were then washed with water (540 mL, 3.0 V). The wet solid was washed with hexanes (0.9 L, 5.0 V). Bulk residual water was removed from the solids by maintaining vacuum filtration for 60-90 min. The wet solid was dried in a hot air oven for 7-8 h at 50 °C (until the moisture content was below 1.0%). The isolated material, 4-chloro-1-(2,2-difluoroethyl)-7-nitro-1*H*-indazol-3-amine (160 g, 71% yield), was used in the next step without further purification. ¹H NMR (400MHz, CDCl₃): *δ* 8.05 (d, *J* = 8.4 Hz, 1H), 7.07 (d, *J* = 8.4 Hz, 1H), 6.00 (tt, *J₁* = 3.9 Hz, *J₂* = 7.7 Hz, 1H), 4.76 - 4.84 (m, 4H).

### Step 2: Preparation of N-(4-chloro-1-(2,2-difluoroethyl)-7-nitro-1H-indazol-3-yl)methane sulfonamide

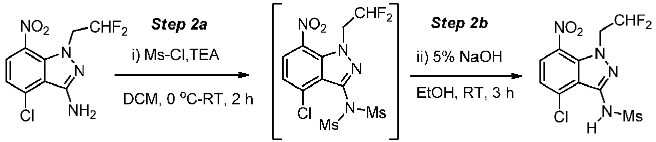

Step 2a: To a solution of 4-chloro-1-(2,2-difluoroethyl)-7-nitro-1*H*-indazol-3-amine (170.0 g, 0.96 mol, 1.0 equiv.) in DCM (1.7 L, 10.0 V) at 0-5 °C was added triethyl amine (264 mL, 2.88 mol, 3.0 equiv.), followed by 4-dimethylaminopyridine (3.4 g, 0.048 mol, 0.05 equiv.). The reaction mass was stirred for 5-10 min., then methanesulfonyl chloride (120 mL, 2.4 mol, 2.5 equiv.) was added slowly while maintaining the reaction mass below 10 °C. The reaction mixture was allowed to warm to room temperature and then was stirred for 1.5-2.0 h. After completion of the reaction (monitored by TLC), the mixture was diluted with water (1.7 L, 10.0 V) and then stirred at room temperature for 15 min. The organic layer was separated, and the aqueous layer was extracted with DCM (1.7 L, 10.0 V). The combined organic layers were washed with 10% brine solution (340 mL, 2.0 V), dried over Na₂SO₄ and concentrated to afford the product as a crude solid. The solids were triturated with hexanes (340 mL, 2.0 V) at room temperature to obtain *N-*(4-chloro-1-(2,2-difluoroethyl)-7-nitro-1*H-*indazol-3-yl)-*N*-(methylsulfonyl) methanesulfonamide which was used directly in the next step. Step 2b: To a stirred solution of *N*-(4-chloro-1-(2,2-difluoroethyl)-7-nitro-1*H*-indazol-3-yl)-*N*-(methylsulfonyl) methanesulfonamide (entirety of material prepared above) in ethanol (1.7 L, 10.0 V) at room temperature was added slowly aq. 5% NaOH solution (1.19 L, 7.0 V) [Note: Slow addition is preferred via dropping funnel]. The reaction mass was stirred at the same temperature for 3 h. After completion of the reaction [Sample preparation for TLC analysis: an aliquot of reaction solution (~1 mL) was acidified with aq. 2.0 N HCl to reach the pH 2-3; then the mixture was extracted with ethyl acetate and organic layer was analyzed by TLC], the reaction mass was cooled to 0-5 °C and the pH was adjusted to 2-3 by the addition of aq. 2.0 N HCl (~850 mL, 5.0 V) at below 10 °C [Note: Precipitation occurred upon addition of HCl and the solids increased gradually with stirring]. The reaction mixture was warmed to room temperature and then stirred for 1.5-2.0 h. Solids obtained were isolated via filtration and were then washed with water (340 mL, 2.0 V); followed by washing with hexanes (340 mL, 2.0 V). Bulk residual water was removed from the solids by maintaining vacuum filtration for 60-90 min. The wet material was dried in a hot air oven at 50 °C for 6-7 h (until the moisture content was below 1.0%) to afford the dried product, *N*-(4-chloro-1-(2,2-difluoroethyl)-7-nitro-1*H*-indazol-3-yl)methanesulfonamide (170.0 g, 75%) as a yellow solid. ¹H NMR (400MHz, CDCl₃): *δ* 8.15 (d, *J* = 8.3 Hz, 1H), 7.52 (bs, 1H), 7.24 (d, *J* = 8.3 Hz, 1H), 6.04 (tt, *J₁* = 3.7 Hz, *J₂* = 7.9 Hz, 1H), 5.02 (td, *J₁* = 3.9 Hz, *J₂* = 14.3 Hz, 2H), 3.42 (s, 4H).

### Step 3: Preparation of N-(4-chloro-1-(2,2-difluoroethyl)-7-nitro-1H-indazol-3-yl)-N-(4-methoxy benzyl)methanesulfonamide

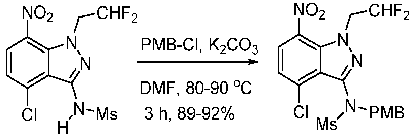

To a mixture of *N*-(4-chloro-1-(2,2-difluoroethyl)-7-nitro-1*H*-indazol-3-yl)methane sulfonamide (160.0 g, 0.45 mol, 1.0 equiv.) and 1-(chloromethyl)-4-methoxybenzene (67.6 mL, 0.5 mol, 1.1 equiv.) in DMF (1.6 L, 10.0 V) at room temperature was added potassium carbonate (93.8 g, 0.59 mol, 1.3 equiv.). The reaction mixture was heated to 80-90 °C and maintained at the same temperature for 3 h. After completion of the reaction (monitored by TLC), the mixture was poured into ice cold water (4.8 L, 60.0 V) [Note: Slow quenching with vigorous stirring is preferred to avoid clumping as the product precipitates]. The resulting solids were isolated via filtration and washed with water (480 mL, 3.0 V); then the solids were washed with hexanes (320 mL, 2.0 V). Bulk residual water was removed from the solids by maintaining vacuum filtration for 1-2 h. The isolated solid was dissolved in ethyl acetate (1.6 L, 10.0 V) and charcoal was added (16.0 g). The mixture was heated to 60-70 °C and then stirred for 30-45 min. at that temperature. The mixture was filtered while hot (40-50 °C) through a pad of Celite and the Celite pad was then extracted with ethyl acetate (800 mL, 5.0 V). The combined filtrates were concentrated to dryness under reduced pressure at below 50 °C. To the resulting solids at room temperature was added ethyl acetate (160 mL, 1.0 V). The suspension was stirred for 30 min. The solids were isolated via filtration and then were washed with hexanes (320 mL, 2.0 V). Residual water was removed from the solids by maintaining vacuum filtration for 45-60 min. to afford the product *N*-(4-chloro-1-(2,2-difluoroethyl)-7-nitro-1*H*-indazol-3-yl)-*N*-(4-methoxybenzyl)methanesulfonamide (180.0 g, 92% yield) as a yellow solid. ¹H NMR (400MHz, CDCl₃): *δ* 8.06 (d, *J* = 8.4 Hz, 1H), 7.52 (bs, 1H), 7.27 - 7.21 (m, 4H), 6.77 (d, *J* = 8.3 Hz, 2H), 6.01 (tt, *J₁* = 3.8 Hz, *J₂* = 7.9 Hz, 1H), 5.12 - 4.78 (m, 4H), 3.74 (s, 3H), 3.02 (s, 3H).

### Step 4: Preparation of N-(7-amino-4-chloro-1-(2,2-difluoroethyl)-1N-indazol-3-yl)-N-(4-methoxybenzyl)methanesulfonamide

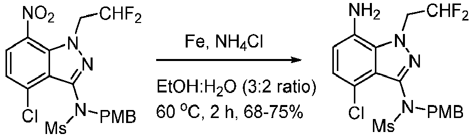

To a stirred suspension of iron powder (76.5 g, 1.37 mol, 5.0 equiv.) in a mixture of EtOH (650 mL, 5.0 V) and water (780 mL, 6.0 V) at room temperature was added ammonium chloride (118.0 g, 2.18 mol, 8.0 equiv.). To the mixture was added *N*-(4-chloro-1-(2,2-difluoroethyl)-7-nitro-1*H*-indazol-3-yl)-*N*-(4-methoxybenzyl)methanesulfonamide (130 g, 0.27 mol, 1.0 equiv.) in EtOH (520 mL, 4.0 V). The reaction mixture was heated to 60 °C and then stirred for 2 h. After completion of the reaction (monitored by in-process TLC/HPLC), the mixture was cooled to room temperature and diluted with ethyl acetate (1.3 L, 10.0 V) and water (390 mL, 3.0 V). The mixture was stirred for 15 min. The mixture was filtered through a pad of Celite and the Celite pad was then extracted with ethyl acetate (650 mL, 5.0 V). The bi-phasic filtrate was partitioned, and the organic phase was reserved while the aqueous layer was extracted with ethyl acetate (650 mL, 5.0 V). The combined organic layers were washed with brine (1.3 L, 10 V), dried over Na₂SO₄, and then concentrated *in vacuo* to afford a crude solid. To the crude product was added MTBE (650 mL, 5.0 V) and the suspension was stirred for 30 min. at room temperature. The solids were isolated via filtration. Bulk residual water was removed from the solids by maintaining vacuum filtration for 30-45 min. The wet product was dried in a hot air oven (50 °C) for 2 h to afford the title compound *N-*(7-amino-4-chloro-1-(2,2-difluoroethyl)-1*H*-indazol-3-yl)-*N*-(4-methoxy benzyl)methanesulfonamide (100.0 g, 70% yield) as off-white solid. ¹H NMR (400MHz, CDCl₃): *δ* 7.21 (d, *J* = 8.5 Hz, 2H), 6.87 (d, J= 8.4 Hz, 1H), 6.78 (d, *J* = 8.5 Hz, 2H), 6.52 (d, *J* = 8.3 Hz, 1H), 6.01 (tt, *J₁* = 3.8 Hz, *J₂* = 7.7 Hz, 1H), 4.98-4.69 (m, 4H), 3.75 (s, 3H), 2.98 (s, 3H).

### Preparation of N-(7-amino-4-chloro-1-(2,2-difluoroethyl)-1H-indazol-3-yl)-N-(4-methoxybenzyl)cydopropanesulfonamide

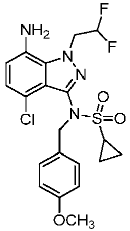

### Synthesis Scheme:

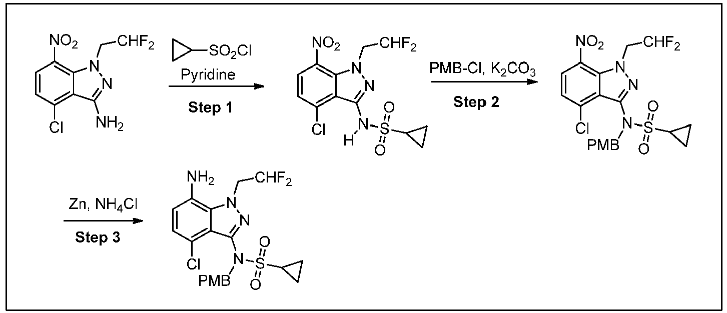

### Step 1: Preparation of N-(4-chloro-1-(2,2-difluoroethyl)-7-nitro-1H-indazol-3-yl)cyclopropanesulfonamide

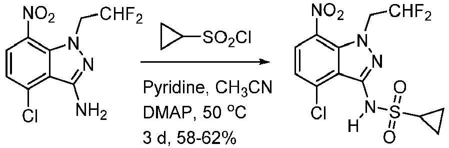

To a stirred solution of 4-chloro-1-(2,2-difluoroethyl)-7-nitro-1*H*-indazol-3-amine (150.0 g, 0.54 mol, 1.0 equiv.) in acetonitrile (600 mL, 4.0 V) at room temperature was added pyridine (600 mL, 4.0 V), followed by the addition of 4-dimethylaminopyridine (30.0 g, 0.27 mol, 0.5 equiv.). The reaction mass was stirred for 5-10 min., then cyclopropylsulfonyl chloride (114 mL, 1.08 mol, 2.0 equiv.) was added at room temperature. The reaction mixture was heated to 50 °C and then stirred at that temperature for 3 days. After completion of the reaction (monitored by TLC), the mixture was cooled to room temperature and diluted with water (1.5 L, 10.0 V) and ethyl acetate (1.5 L, 10.0 V), then stirred at room temperature for 15 min. The organic layer was separated, and the aqueous layer was extracted with EtOAc (300 mL, 2.0 V). The combined organic layers were washed with aq. 1.0 N HCl (600 mL, 4.0 V), followed by 10% brine solution (1.5 L, 10.0 V). The organic layer was dried over Na₂SO₄, filtered, and then concentrated under reduced pressure to afford *N*-(4-chloro-1-(2,2-difluoroethyl)-7-nitro-1*H*-indazol-3-yl)cyclopropanesulfonamide (124.0 g, 61%) as a viscous liquid. ¹H NMR (400MHz, CDCl₃): *δ* 8.11 (d, *J* = 8.5 Hz, 1H), 7.25 (d, *J* = 8.5 Hz, 1H), 6.04 (tt, *J₁* = 3.8 Hz, *J₂* = 7.7 Hz, 1H), 5.05 (td, *J₁* = 3.8 Hz, *J₂* = 14.4 Hz, 2H), 3.06 - 3.00 (m, 1H), 1.65 - 1.42 (m, 2H), 1.19 - 1.13 (m, 2H).

### Step 2: Preparation of N-(4-chloro-1-(2,2-difluoroethyl)-7-nitro-1H-indazol-3-yl)-N-(4-methoxybenzyl)cyclopropanesulfonamide

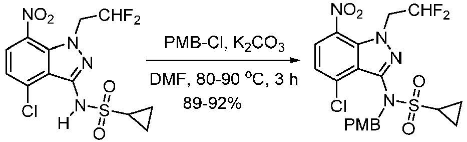

To a mixture of *N*-(4-chloro-1-(2,2-difluoroethyl)-7-nitro-1*H*-indazol-3-yl)cyclopropanesulfonamide (100.0 g, 0.20 mol, 1.0 equiv.) and 1-(chloromethyl)-4-methoxybenzene (39.2 mL, 0.22 mol, 1.1 equiv.) in DMF (1.0 L, 10.0 V) at room temperature was added potassium carbonate (128 g, 0.33 mol, 1.3 equiv.). The reaction mixture was heated to 80-90 °C and maintained at that temperature for 3 h. After completion of the reaction (monitored by TLC), the mixture was poured into ice cold water (3.0 L, 30.0 V) [Note: Slow quenching with vigorous stirring is preferred to avoid clumping as the product precipitates]. The resulting solids were isolated via filtration and washed with water (300 mL, 3.0 V); then the solids were washed with hexanes (300 mL, 3.0 V). Bulk residual water was removed from the solids by maintaining vacuum filtration for 1-2 h. The wet solid was dissolved in ethyl acetate (500 mL, 5.0 V) and charcoal was added (10.0 g). The mixture was heated to 60-70 °C and then stirred for 30-45 minutes at that temperature. The mixture was filtered while hot (40-50 °C) through a pad of Celite and the Celite pad was extracted with ethyl acetate (500 mL, 5.0 V). The combined filtrates were concentrated to dryness under reduced pressure at below 50 °C to afford *N*-(4-chloro-1-(2,2-difluoroethyl)-7-nitro-1*H*-indazol-3-yl)-/V-(4-methoxy- benzyl)cyclopropanesulfonamide (122.0 g, 92% yield) as a yellow solid. ¹H NMR (400MHz, CDCl₃): *δ* 8.05 (d, *J* = 8.6 Hz, 1H), 7.26-7.22 (m, 3H), 6.73 (d, *J* = 8.5 Hz, 2H), 5.98 (tt, *J₁* = 3.7 Hz, *J₂* = 7.8 Hz, 1H), 5.09-4.88 (m, 4H), 3.72 (s, 3H), 2.65-2.60 (m, 1H), 1.15-1.06 (m, 2H), 0.89 - 0.86 (m, 2H).

### Step 3: Preparation of N-(7-amino-4-chloro-1-(2,2-difluoroethyl)-1H-indazol-3-yl)-N-4-methoxybenzyl)cydopropanesulfonamide

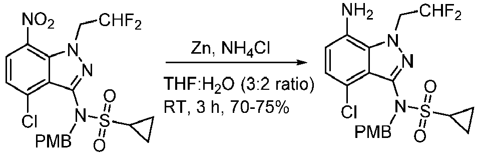

To a stirred suspension of zinc powder (156.0 g, 2.4 mol, 10.0 equiv.) in a mixture of THF (1.2 L, 10.0 V) and water (2.4 L, 20.0 V) at room temperature was added ammonium chloride (129.0 g, 2.40 mol, 10.0 equiv.). To the mixture was added *N*-(4-chloro-1-(2,2-difluoroethyl)-7-nitro-1*H*-indazol-3-yl)-*N*-(4-methoxybenzyl)cyclopropanesulfonamide (120 g, 0.2 mol, 1.0 equiv.) in THF (2.4 L, 20.0 V). The reaction mixture was stirred at room temperature for 2 h. After completion of the reaction (monitored by in-process TLC/HPLC), the mixture was diluted with ethyl acetate (1.2 L, 10.0 V) and water (360 mL, 3.0 V). The mixture was stirred for 15 min. The mixture was filtered through Celite and the Celite pad was extracted with ethyl acetate (600 mL, 5.0 V). The bi-phasic filtrate was partitioned, and the organic phase was reserved while the aqueous layer was extracted with ethyl acetate (600 mL, 5.0 V). The combined organic layers were washed with 10% brine solution (1.2 L, 10 V), dried over Na₂SO₄. filtered, and then concentrated *in vacuo* to afford a crude solid. To the crude product was added MTBE (600 mL, 5.0 V) and the suspension was stirred for 30-45 min. at room temperature. The solids were isolated by filtration and then bulk residual water was removed from the solids by maintaining vacuum filtration for 30-45 min. The wet product was dried in a hot air oven (50 °C) for 2 h to afford the product, *N*-(7-amino-4-chloro-1-(2,2-difluoroethyl)-1*H*-indazol-3-yl)-*N*-(4-methoxybenzyl)cyclopropanesulfonamide (81.0 g, 73% yield) as off-white solid. ¹H NMR (400MHz, CDCl₃): *δ* 7.25 (d, *J* = 8.5 Hz, 2H), 6.93 (d, *J* = 8.4 Hz, 1H), 6.75 (d, *J* = 8.3 Hz, 2H), 6.57 (d, *J* = 8.4 Hz, 1H), 6.03 (tt, *J₁* = 3.7 Hz, *J₂* = 7.9 Hz, 1H), 4.80-4.95 (m, 4H), 3.74 (s, 3H), 2.67-2.61 (m, 1H), 1.14 (d, *J* = 2.4 Hz, 2H), 0.96 (d, *J* = 2.3 Hz, 2H).

### Preparation of N-(7-amino-4-chloro-1-(2,2,2-trifluoroethyl)-1H-indazol-3-yl)-N-(4-methoxybenzyl)methanesulfonamide

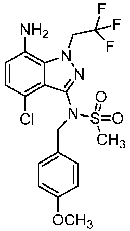

### Synthesis Scheme:

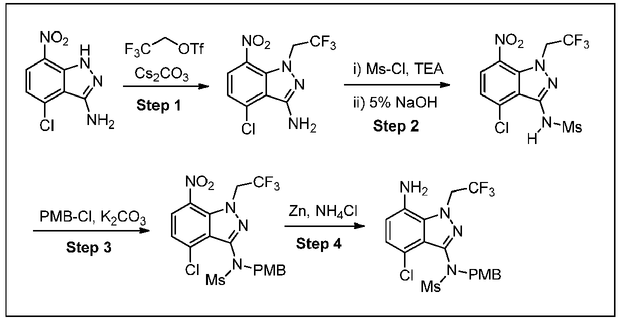

### Step 1: Preparation of 4-chloro-7-nitro-1-(2,2,2-trifluoroethyl)-1H-indazol-3-amine

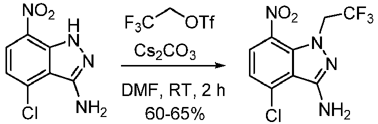

To a stirred solution of 4-chloro-7-nitro-1*H*-indazol-3-amine (50 g, 0.23 mol, 1.0 equiv.) in DMF (500 mL, 10.0 V) at 10-15 °C was added cesium carbonate (Cs₂CO₃) (153.3 g, 0.47 mol, 2.0 equiv.) at a rate sufficient to maintain the reaction mass below 20 °C. The mixture was stirred for 5-10 min, then to the stirred mixture at 10-15 °C was added 2,2,2-trifluoroethyl trifluoromethanesulfonate (60.18 g, 0.26 mol, 1.1 equiv.) at a rate sufficient to maintain the reaction mass below 20 °C (Note: slow addition is preferred for obtaining more favorable regio-selectivity). The reaction mass was allowed to slowly warm to room temperature and was then stirred at the same temperature for 2 h. After completion of the reaction (monitored by TLC), the reaction mass was quenched via the addition of ice-cold water (1.5 L, 30.0 V) and the resulting mixture was allowed to warm to room temperature with stirring for 6-8 h. The solids were isolated via filtration and were then washed with water (150 mL, 3.0 V). The wet solid was washed with hexanes (250 mL, 5.0 V) and then bulk residual water was removed from the solids by maintaining vacuum filtration for 60-90 min. The wet solid was dried in a hot air oven for 7-8 h at 50 °C (until the moisture content was below 1.0%). The isolated material, 4-chloro-7-nitro-1-(2,2,2-trifluoroethyl)-1*H*-indazol-3-amine (45.0 g, 60% yield), was used directly in the next step without further purification. ¹H-NMR (400 MHz, CDCl₃): *δ* 8.09 (d, *J* = 8.40 Hz, 1H), 7.12 (d, *J* = 8.40 Hz, 1H), 5.14 (q, *J* = 8.52 Hz, 2H), 4.77 (bs, H).

### Step 2: Preparation of N-(4-chloro-7-nitro-1-(2,2,2-trifluoroethyl)-1H-indazol-3-yl)methanesulfonamide

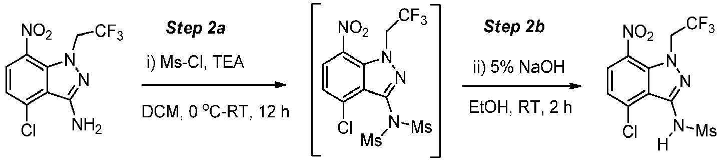

(Step 2a): To a solution of 4-chloro-7-nitro-1-(2,2,2-trifluoroethyl)-1*H*-indazol-3-amine (20.0 g, 0.068 mol, 1.0 equiv.) in DCM (200 mL, 10.0 V) at 0-5 °C. was added triethylamine (29.0 mL, 0.204 mol, 3.0 equiv.), followed by the addition of 4-dimethylaminopyridine (415 mg, 0.03 mol, 0.05 equiv.). The reaction mass was stirred for 5-10 min., then to the mixture was added methanesulfonyl chloride (13.25 mL, 0.17 mol, 2.5 equiv) at a rate sufficient to maintain the reaction mass below 10 °C. The reaction mixture was allowed to warm to room temperature with stirring for 12 h. After completion of the reaction (monitored by TLC), the mixture was diluted with water (200 mL, 10.0 V) and then stirred at room temperature for 15 min. The organic layer was separated, and the aqueous layer was extracted with DCM (200 mL, 10.0 V). The combined organic layers were washed with 10% brine solution (60 mL, 3.0 V), dried over Na₂SO₄, filtered, and concentrated to afford the crude solids. The solids were triturated with hexanes (60 mL, 3.0 V) at room temperature to obtain the intermediate, *N*-(4-chloro-7-nitro-1-(2,2,2-trifluoroethyl)-1*H-*indazol-3-yl)-*N-*(methylsulfonyl)methanesulfonamide, which was used directly in the next step.

(Step 2b): To a stirred solution of *N-*(4-chloro-7-nitro-1-(2,2,2-trifluoroethyl)-1*H-*indazol-3-yl)-*N*-(methylsulfonyl)methanesulfonamide (entirety of the material prepared above) in ethanol (200 mL, 10.0 V) at room temperature was added slowly aq. 5% NaOH solution (140 mL, 7.0 V) [Note: Slow addition is preferred via dropping funnel]. The reaction mass was stirred at the same temperature for 2 h. After completion of the reaction [Sample preparation for TLC analysis: An aliquot of the reaction solution (~1.0 ml) was acidified by the addition of aq. 2.0 N HCl to reach pH 2-3; then the mixture was extracted with ethyl acetate and the organic phase was analyzed by TLC], the reaction mass was cooled to 0-5 °C and the pH was adjusted to 2-3 by the addition of aq. 2.0 N HCl (100 mL, 5.0 V) while maintain the temperature below 10 °C [Note: Precipitation occurred upon addition of HCl and increased with stirring]. The reaction mixture was warmed to room temperature and then stirred for 1.5-2.0 h. The solids were isolated via filtration and were then washed with water (60 mL, 3.0 V), followed by washing with hexanes (60 mL, 3.0 V). Bulk residual water was removed from the solids by maintaining vacuum filtration for 60-90 min. The wet material was dried in a hot air oven at 50 °C for 6-7 h (until the moisture content was below 1.0%) to afford *N*-(4-chloro-7-nitro-1-(2,2,2-trifluoroethyl)-1*H*-indazol-3-yl)methanesulfonamide (22.1 g, 87%) as a yellow solid. ¹H NMR (400 MHz, CDCl₃): *δ* 8.19 (d, *J* = 8.40 Hz, 1H), 7.56 (bs, 1H), 7.30 (d, *J* = 8.40 Hz, 1H), 5.34 (q, *J* = 8.30 Hz, 2H), 3.46 (s, 3H).

### Step 3: Preparation of N-(4-chloro-7-nitro-1-(2,2,2-trifluoroethyl)-1H-indazol-3-yl)-N-(4-methoxybenzyl)methanesulfonamide

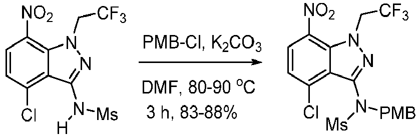

To a mixture of *N-*(4-chloro-7-nitro-1-(2,2,2-trifluoroethyl)-1*H-*indazol-3-yl)methanesulfonamide (50.0 g, 0.134 mol, 1.0 equiv.) and 1-(chloromethyl)-4-methoxybenzene (23.0 g, 0.147 mol, 1.1 equiv.) in DMF (500 mL, 10.0 V) at room temperature was added potassium carbonate (27.8 g, 0.201 mol, 1.5 equiv.). The reaction mixture was heated to 80-90 °C and maintained at that temperature for 3 h. After completion of the reaction (monitored by TLC), the mixture was poured into ice cold water (2.0 L, 40.0 V) [Note: Slow quenching with vigorous stirring is preferred to avoid clumping as the product precipitates]. The resulting solids were isolated via filtration and washed with water (150 mL, 3.0 V); then the solids were washed with hexanes (150 mL, 3.0 V). Bulk residual water was removed from the solids by maintaining vacuum filtration for 1-2 h. The solids were dissolved in ethyl acetate (500 mL, 10.0 V) and to the solution was added charcoal (5.0 g). The mixture was heated to 60-70 °C and then stirred at that temperature for 30-45 min. The mixture was filtered while hot (40-50 °C) through a pad of Celite and the Celite pad was extracted with ethyl acetate (250 mL, 5.0 V). The combined filtrate was concentrated to dryness under reduced pressure at below 50 °C. The solids were combined with ethyl acetate (50 mL, 1.0 V) at room temperature. The resulting suspension was stirred for 30 min. The solids were isolated via filtration and then were washed with hexanes (100 mL, 2.0 V). Residual water was removed from the solids by maintaining vacuum filtration for 45-60 min. to afford *N*-(4-chloro-7-nitro-1-(2,2,2-trifluoroethyl)-1*H-*indazol- 3-yl)-*N-*(4-methoxybenzyl)methanesulfonamide (56.0 g, 85% yield) as a yellow solid. ¹H NMR (400 MHz, CDCl₃): *δ* 8.12 (d, *J* = 8.36 Hz, 1H), 7.31 (d, *J* = 8.36 Hz, 1H), 7.22 (d, *J* = 8.44 Hz, 2H), 6.77 (d, *J* = 8.44 Hz, 2H), 5.50-5.25 (m, 2H), 4.94-4.79 (m, 2H), 3.75 (s, 3H), 3.02 (s, 3H).

### Step 4: Preparation of N-(7-amino-4-chloro-l-(2,2,2-trifluoroethyl)-1H-indazol-3-yl)-N-(4-methoxybenzyl)methanesulfonamide

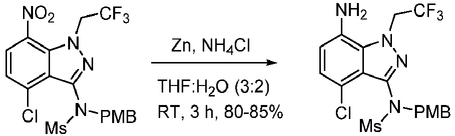

To a stirred suspension of zinc powder (66.31 g, 1.01 mol, 10.0 equiv.) in THF (500 mL, 10.0 V) and water (1.0 L, 20.0 V) at room temperature was added ammonium chloride (54.78 g, 1.01 mol, 10.0 equiv.). To the mixture was added a solution of *N*-(4-chloro-7-nitro-1-(2,2,2-trifluoroethyl)-1*H*-kindazol-3-yl)-*N*-(4-methoxybenzyl)methanesulfonamide (50.0 g, 0.101 mol, 1.0 equiv.) in THF (1.0 L, 20.0 V). The reaction mixture was stirred at room temperature for 3 h. After completion of the reaction (monitored by in-process TLC/HPLC), the mixture was diluted with ethyl acetate (1.0 L, 20.0 V) and water (250 mL, 5.0 V). The mixture was stirred for 15 min. The mixture was filtered through a pad of Celite and the Celite pad was extracted with ethyl acetate (250 mL, 5.0 V). The bi-phasic filtrate was partition and the organic layer was reserved while the aqueous layer was extracted with ethyl acetate (500 mL, 10.0 V). The combined organic layers were washed with 10% brine solution (500 mL, 10.0 V), dried over Na₂SO₄. filtered, and then concentrated *in vacuo* to afford a crude solid. To the crude product was added MTBE (250 mL, 5.0 V) and the resulting suspension was stirred for 30 min. at room temperature. The solids were isolated by filtration and then bulk residual water was removed from the solids by maintaining vacuum filtration for 30-45 min. The wet product was dried in a hot air oven (50 °C) for 2 h to afford the title product *N*-(7-amino-4-chloro-1-(2,2,2-trifluoroethyl)-1*H*-indazol-3-yl)-*H*-(4-methoxybenzyl)methanesulfonamide (39.0 g, 83% yield) as off-white solid. ¹H NMR (400 MHz, CDCl₃): *δ* 7.25 (d, *J* = 8.48 Hz, 2H), 6.98 (d, *J* = 7.80 Hz, 1H), 6.79 (d, *J* = 8.48 Hz, 2H), 6.66 (d, *J* = 7.84 Hz, 1H), 5.35-4.75 (m, 4H), 3.77 (s, 3H), 3.56 (bs, 2H), 2.98 (s, 3H).

### Preparation of tert-Butyl (S)-(1-amino-3-(3,5-difluorophenyl)-1-oxopropan-2-yl)carbamate

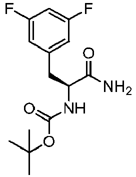

To a solution of (S)-2-((tert-butoxycarbonyl)amino)-3-(3,5-difluorophenyl)propanoic acid (15 g, 49.8 mmol) in CH₂Cl₂ (200 mL) were added HOBT (8.39 g, 54.8 mmol) and 1-(3-dimethylaminopropyl)-3-ethylcarbodiimide hydrochloride (10.50 g, 54.8 mmol). The mixture was then cooled to 0 °C and to the mixture was added dropwise aq. 30 % ammonium hydroxide (4.31 mL, 33.2 mmol). The mixture was allowed to warm to r.t. with stirring for 2 h. To the mixture was added water upon which a precipitate formed. The solids were collected by filtration and then washed with water to afford the title compound tert-butyl (S)-(1-amino-3-(3,5-difluorophenyl)-1-oxopropan-2-yl)carbamate (13.9 g, 46.3 mmol, 93 % yield) as a white solid. ¹H NMR (400 MHz, DMSO-d₆) δ 7.39 (br s, 2H), 7.14 - 6.95 (m, 3H), 6.91 (br d, *J*=9.0 Hz, 1H), 4.14 - 4.05 (m, 1H), 2.98 (br dd, *J*=13.6, 3.8 Hz, 1H), 2.77 - 2.66 (m, 1H), 1.29 (s, 9H). LC/MS: m/z = 323.0 [M+Na].

### Preparation of ethyl 2-((tert-butoxycarbonyl)amino)-3-(3,5-difluorophenyl)propanimidate

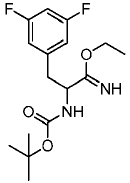

To a suspension of tert-butyl (S)-(1-amino-3-(3,5-difluorophenyl)-1-oxopropan-2-yl)carbamate (13.9 g, 46.3 mmol) in CH₂Cl₂ (300 mL) was added a solution of triethyloxonium tetrafluoroborate (8.79 g, 46.3 mmol) in CH₂Cl₂ (30 mL). The resulting mixture was stirred at room temp for 6 h (the suspension became a clear, homogeneous solution after 3h). To the solution was added aq. saturated NaHCO₃, and the resulting mixture was stirred for 15 min and then extracted with dichloromethane, washed with brine, dried (Na₂SO₄), filtered and concentrated to afford the title compound ethyl 2-((tert-butoxycarbonyl)amino)-3-(3,5-difluorophenyl)propanimidate (13.6 g, 41.4 mmol, 89 % yield) as off-white solid (used as is). ¹H NMR (400 MHz, DMSO-d₆) δ 7.74 (s, 1H), 7.33 (br d, *J*=9.0 Hz, 1H), 7.12 - 7.03 (m, 1H), 6.95 (br d, *J*=7.3 Hz, 2H), 4.24 - 4.15 (m, 1H), 4.04 (q, *J*=6.9 Hz, 2H), 2.98 (br dd, *J*=13.7, 4.6 Hz, 1H), 2.72 (br dd, .7=13.4, 10.7 Hz, 1H), 1.30 (s, 9H), 1.17 (t, *J*=7.0 Hz, 3H).

### Preparation of tert-butyl (1-((4-chloro-3-(N-(4-methoxybenzyl)methylsulfonamido)-1-methyl-1H-indazol-7-yl)amino)-3-(3,5-difluorophenyl)-1-iminopropan-2-yl)carbamate

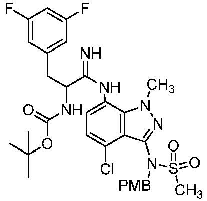

A solution of ethyl 2-((tert-butoxycarbonyl)amino)-3-(3,5-difluorophenyl)propanimidate (13.72 g, 41.8 mmol) and N-(7-amino-4-chloro-1-methyl-1H-indazol-3-yl)-N-(4-methoxybenzyl)methanesulfonamide (11 g, 27.9 mmol) in acetonitrile (300 mL) and acetic acid (2.392 mL, 41.8 mmol) was stirred at room temp for 24 h. The mixture was then diluted with ethyl acetate and washed with aq. 1N Na₂CO₃ and then brine, dried (Na₂SO₄), filtered and concentrated in vacuo. The resulting residue was then purified by silica gel chromatography (5-70% EtOAc in hexanes) to afford the title compound tert-butyl (1-((4-chloro-3-(N-(4-methoxybenzyl)methylsulfonamido)-1-methyl-1H-indazol-7-yl)amino)-3-(3,5-difluorophenyl)-1-iminopropan-2-yl)carbamate (12 g, 17.72 mmol, 63.6 % yield) as a pale-yellow solid. ¹H NMR (500 MHz, DMSO-*d*₆) δ ppm 7.24 (br d, *J*=8.64 Hz, 2 H), 7.05 - 7.16 (m, 4 H), 7.01 (d, *J*=7.75 Hz, 1 H), 6.83 (d, *J*=8.64 Hz, 2 H), 6.51 (br d, *J*=7.45 Hz, 1 H), 4.79 (br d, *J*=13.41 Hz, 2 H), 4.37 (br dd, *J*=8.34, 5.96 Hz, 1 H), 4.08 (s, 2 H), 3.70 (s, 3 H), 3.14 (br d, *J*=11.92 Hz, 1 H), 3.09 (s, 3 H), 2.94 (br dd, *J*=13.11, 10.73 Hz, 1 H), 1.32 (s, 9 H). Methyl sulfone peak is believed to be under DMSO peak. LC/MS: m/z = 677.2 [M+H]⁺.

### Preparation of tert-butyl (1-(1-(4-chloro-3-(N-(4-methoxybenzyl)methylsulfonamido)-1-methyl-1H-indazol-7-yl)-4-hydroxy-6-oxo-1,6-dihydropyrimidin-2-yl)-2-(3,5-difluorophenyl)ethyl)carbamate

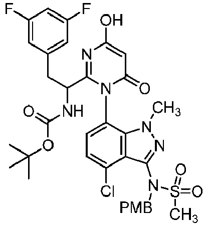

To a solution of tert-butyl (1-((4-chloro-3-(N-(4-methoxybenzyl)methylsulfonamido)-1-methyl-1H-indazol-7-yl)amino)-3-(3,5-difluorophenyl)-1-iminopropan-2-yl)carbamate (11.8 g, 17.43 mmol) in CH₂Cl₂ (200 mL) at 0 °C was added N-methylmorpholine (11.50 mL, 105 mmol) followed by malonyl dichloride (4.24 mL, 43.6 mmol). The mixture was allowed to warm to room temp with stirring and was then stirred for 1 h. To the mixture was added aq. sat. NaHCO₃ and the mixture was then extracted with DCM, dried (Na₂SO₄), filtered and concentrated in vacuo. The resulting residue was purified by silica gel chromatography (0-5% MeOH in DCM) to afford the title tert-butyl (1-(1-(4-chloro-3-(N-(4-methoxybenryl)methylsulfonamido)-1-methyl-1H-indazol-7-yl)-4-hydroxy-6-oxo-1,6-dihydropyrimidin-2-yl)-2-(3,5-difluorophenyl)ethyl)carbamate (5.5 g, 7.38 mmol, 42.4 % yield) as a mixture of diastereomers (atropisomers) and their enantiomers, 4 stereoisomers in total. LCMS (M+Na) = 767.10

### Preparation of 2-(1-((tert-butoxycarbonyl)amino)-2-(3,5-difluorophenyl)ethyl)-1-(4-chloro-3-(N-(4-methoxybenzyl)methylsulfonamido)-1-methyl-1H-indazol-7-yl)-6-oxo-1,6-dihydropyrimidin-4-yl trifluoromethanesulfonate

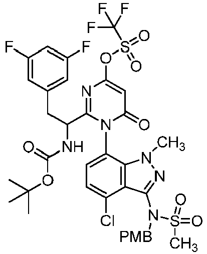

To a solution of tert-butyl (1-(1-(4-chloro-3-(N-(4-methoxybenzyl)methylsulfonamido)-1-methyl-1H-indazol-7-yl)-4-hyd roxy-6-oxo-1,6-dihydropyrimidin-2-yl)-2-(3,5-difluorophenyl)ethyl)carbamate (550 mg, 0.738 mmol) in CH₂Cl₂ (10 mL) at -25 °C was added pyridine (0.298 mL, 3.69 mmol) followed by trifluoromethanesulfonic anhydride (0.156 mL, 0.886 mmol). The mixture was allowed to warm to room temp and with stirring for 1 h. To the solution was added water and the mixture was then extracted with DCM, washed with aq. 1N HCl, dried (Na₂SO₄), filtered and concentrated in vacuo to afford the title compound 2-(1-((tert-butoxycarbonyl)amino)-2-(3,5-difluorophenyl)ethyl)-1-(4-chloro-3-(N-(4-methoxybenryl)methylsulfonamido)-1-methyl-1H-indazol-7-yl)-6-oxo-1,6-dihydropyrimidin-4-yl trifluoromethanesulfonate (550 mg, 0.627 mmol, 85 % yield) as a mixture of diastereomers (atropisomers) and their enantiomers, 4 stereoisomers in total. The material was used in the next step without additional purification. LC/MS: m/z = 877.10 [M + H]⁺.

### Preparation of tert-butyl (1-(1-(4-chloro-3-(N-(4-methoxybenzyl)methylsulfonamido)-1-methyl-1H-indazol-7-yl)-6-oxo-4-phenyl-1,6-dihydropyrimidin-2-yl)-2-(3,5-difluorophenyl)ethyl)carbamate

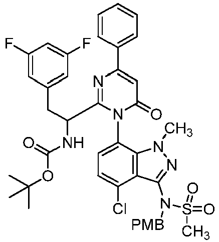

To a solution of 2-(1-((tert-butoxycarbonyl)amino)-2-(3,5-difluorophenyl)ethyl)-1-(4-chloro-3-(N-(4-methoxybenzyl)methylsulfonamido)-1-methyl-1H-indazol-7-yl)-6-oxo-1,6-dihydropyrimidin-4-yl trifluoromethanesulfonate (250 mg, 0.285 mmol), phenylboronic acid (69.5 mg, 0.570 mmol) and K₃PO₄ (181 mg, 0.855 mmol) in THF (1 mL)/Water (0.25 mL) was added dichloro[9,9-dimethyl-4,5-bis(diphenylphosphino)xanthene]palladium(II (21.54 mg, 0.028 mmol) and the resulting mixture was stirred at room temp for 16 h. The mixture was then diluted with water, extracted with ethyl acetate, dried (Na₂SO₄), filtered and concentrated in vacuo. The residue was purified by silica gel chromatography (5-100% EtOAc in hexanes) to afford the title compound tert-butyl (1-(1-(4-chloro-3-(N-(4-methoxybenryl)methylsulfonamido)-1-methyl-1H-indazol-7-yl)-6-oxo-4-phenyl-1,6-dihydropyrimidin-2-yl)-2-(3,5-difluorophenyl)ethyl)carbamate (100 mg, 0.124 mmol, 43.6 % yield) as a mixture of diastereomers (atropisomers) and their enantiomers, 4 stereoisomers in total. LCMS (M - tBu) = 749.15

### Preparation of (S)-N-((6P)-7-(2-(1-amino-2-(3,5-difluorophenyl)ethyl)-6-oxo-4-phenylpyrimidin-1(6H)-yl)-4-chloro-1-methyl-1H-indazol-3-yl)methanesulfonamide

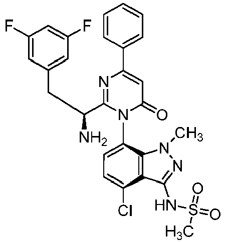

To a stirring solution of tert-butyl (1-(1-(4-chloro-3-(N-(4-methoxybenryl)methylsulfonamido)-1-methyl-1H-indazol-7-yl)-6-oxo-4-phenyl-1,6-dihydropyrimidin-2-yl)-2-(3,5-difluorophenyl)ethyl)carbamate (100 mg, 0.124 mmol) in CH₂Cl₂ (2 mL) was added TFA (1 mL, 12.98 mmol) followed by triflic acid (0.022 mL, 0.248 mmol) and the resulting solution was stirred at room temp for 2h. LCMS analysis at t=2h indicated full conversion (approx 40:60 mixture of atropisomers). The solution was concentrated to a minimum under reduced pressure. The residue was partitioned between EtOAc (50 mL) and aq. NaOH (1M, 10 mL). The aq. phase was tested and determined to be pH >=8.0. The organic phase was isolated and dried over Na₂SO₄, filtered, and then concentrated in vacuo. The residue was then purified via C18 column chromatography (50g RediSep C18 Gold column, 10-60% Mobile Phase A in Mobile Phase B; Mobile Phase A = 5:95 acetonitrile:water with 0.1 % Formic acid; Mobile Phase B = 95:5 acetonitrile:water with 0.1 % Formic; gradient over 30 min). Fractions corresponding to the second eluting atropisomer were pooled and the pH was adjusted to pH > 8 by the addition of 1N NaOH. The mixture was extracted with ethyl acetate, washed with brine, dried (Na₂SO₄), filtered and concentrated in vacuo to afford the title compound (S)-N-((6P)-7-(2-(1-amino-2-(3,5-difluorophenyl)ethyl)-6-oxo-4-phenylpyrimidin-1(6H)-yl)-4-chloro-1-methyl-1H-indazol-3-yl)methanesulfonamide (31 mg, 0.053 mmol, 42.7 % yield) as a mixture of enantiomers. LC/MS: m/z = 585.05 [M+H]⁺.

The first eluting atropisomer was also collected to afford (S)-N-((6M)-7-(2-(1-amino-2-(3,5-difluorophenyl)ethyl)-6-oxo-4-phenylpyrimidin-1(6H)-yl)-4-chloro-1-methyl-1H-indazol-3-yl)methanesulfonamide (25 mg, 0.043 mmol, 34.4 % yield) as a mixture of enantiomers. LC/MS: m/z = 585.05 [M+H]⁺.

### Preparation of Example 1: N-((S)-1-(1-(4-chloro-1-methyl-3-(methylsulfonamido)-1H-indazol-7-yl)-6-oxo-4-phenyl-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide and

### Preparation of Example 2: N-((R)-1-(1-(4-chloro-1-methyl-3-(methylsulfonamido)-1H-indazol-7-yl)-6-oxo-4-phenyl-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

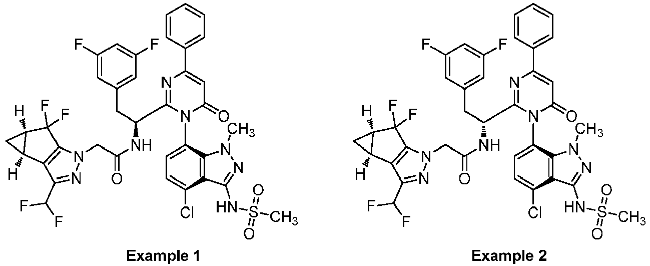

To a solution of 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (11.29 mg, 0.043 mmol) and (S)-N-(7-(2-(1-amino-2-(3,5-difluorophenyl)ethyl)-6-oxo-4-phenylpyrimid in-1(6H)-yl)-4-chloro-1-methyl-1H-indazol-3-yl)methanesulfonamide (25 mg, 0.043 mmol) in DMF (1 mL) was added DIEA (0.022 mL, 0.128 mmol) followed by HATU (19.50 mg, 0.051 mmol) and the resulting mixture was stirred at room temp for 3 h. To the solution was added ammonia in methanol (1M, 0.5 mL) and the resulting solution was stirred for 30 min. To the solution was added water and the mixture was extracted with ethyl acetate, washed with brine, dried (Na₂SO₄), filtered and concentrated in vacuo. The resulting residue was purified by silica gel chromatography (5-50% EtOAc in hexanes) to afford the desired product (25 mg) as mixture of diastereomers (approx 60:40 by analytical SFC). The material was then further purified by chiral SFC chromatography using the following method: Column = Chiralpak IA, 10 x 250 mm, 5 µm; Mobile Phase = 60% IPA in CO₂; Back pressure = 150 bar; Column temperature = 40 °C; Flow rate = 3.5 mL/min.; Loading = 4 mg/injection. Two peaks were collected:

The first peak to elute, Example 1, N-((S)-1-(1-(4-chloro-1-methyl-3-(methylsulfonamido)-1H-indazol-7-yl)-6-oxo-4-phenyl-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. ¹H NMR (500 MHz, METHANOL-*d*₄) δ ppm 8.14 (dd, *J*=7.60, 2.24 Hz, 2 H), 7.39 - 7.54 (m, 3 H), 7.18 (d, *J*=8.05 Hz, 1 H), 7.05 (d, *J*=7.75 Hz, 1 H), 7.00 (s, 1 H), 6.39 - 6.73 (m, 4 H), 4.78 - 4.80 (m, 1 H), 4.36 - 4.51 (m, 2 H), 3.58 (s, 3 H), 3.34 - 3.41 (m, 1 H), 3.13 (s, 3 H), 2.98 - 3.09 (m, 1 H), 2.24 - 2.34 (m, 2 H), 1.21 - 1.28 (m, 1 H), 0.84 - 0.94 (m, 1 H). LCMS Method B: retention time = 3.10 min; m/z = 831.05 [M+H]⁺

The second peak to elute, Example 2, N-((R)-1-(1-(4-chloro-1-methyl-3-(methylsulfonamido)-1H-indazol-7-yl)-6-oxo-4-phenyl-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. ¹H NMR (500 MHz, METHANOL-*d*₄) δ ppm 8.04 - 8.21 (m, 2 H), 7.41 - 7.55 (m, 3 H), 7.19 (d, *J*=7.75 Hz, 1 H), 7.07 (d, *J*=7.75 Hz, 1 H), 7.00 (s, 1 H), 6.37 - 6.73 (m, 4 H), 4.77 - 4.80 (m, 1 H), 4.43 (s, 2 H), 3.58 (s, 3 H), 3.34 - 3.44 (m, 1 H), 3.14 (s, 3 H), 3.02 (dd, *J*=14.16, 9.09 Hz, 1 H), 2.31 (ddd, *J*=11.40, 7.67, 3.87 Hz, 2 H), 1.18 - 1.25 (m, 1 H), 0.77 - 0.91 (m, 1 H). LCMS Method B: retention time = 3.10 min; m/z = 831.05 [M+H]⁺

### Preparation of tert-butyl (1-(4-(benzyloxy)-1-(4-chloro-3-(N-(4-methoxybenzyl)methylsulfonamido)-1-methyl-1H-indazol-7-yl)-6-oxo-1,6-dihydropyrimidin-2-yl)-2-(3,5-difluorophenyl)ethyl)carbamate

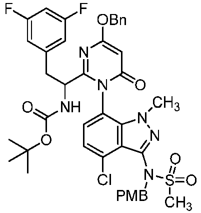

To a solution of tert-butyl (1-(1-(4-chloro-3-(N-(4-methoxybenzyl)methylsulfonamido)-1-methyl-1H-indazol-7-yl)-4-hydroxy-6-oxo-1,6-dihydropyrimidin-2-yl)-2-(3,5-difluorophenyl)ethyl)carbamate (5.0 g, 6.71 mmol) in DMF (100 ml) was added K₂CO₃ (1.391 g, 10.06 mmol) followed by benzyl bromide (0.958 ml, 8.05 mmol) and the resulting mixture was stirred at room temp for 2 h. To the solution was added water and the mixture was then extracted with ethyl acetate, washed with brine, dried (Na₂SO₄), filtered and concentrated in vacuo. The resulting residue was purified by silica gel chromatography (5-70% EtOAc in hexane) to afford the title compound tert-butyl (1-(4-(benzyloxy)-1-(4-chloro-3-(N-(4-methoxybenzyl)methylsulfonamido)-1-methyl-1H-indazol-7-yl)-6-oxo-1,6-dihydropyrimidin-2-yl)-2-(3,5-difluorophenyl)ethyl)carbamate (3.8 g, 4.55 mmol, 67.8 % yield) as a white solid. LC/MS: m/z = 835.20 [M+H]⁺.

### Preparation of (S)-N-((6P)-7-((1P)-2-(1-amino-2-(3,5-difluorophenyl)ethyl)-4-(benzyloxy)-6-oxopyrimidin-1(6H)-yl)-4-chloro-1-methyl-1H-indazol-3-yl)-N-(4-methoxybenzyl)methanesulfonamide

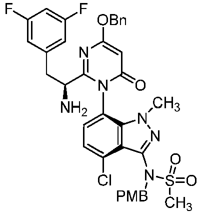

A mixture of tert-butyl (1-(4-(benzyloxy)-1-(4-chloro-3-(N-(4-methoxybenzyl)methylsulfonamido)-1-methyl-1H-indazol-7-yl)-6-oxo-1,6-dihydropyrimidin-2-yl)-2-(3,5-difluorophenyl)ethyl)carbamate (3.7 g, 4.43 mmol) and HCl (4M in dioxane, 33.2 ml, 133 mmol) was stirred at room temp for 1 h. The mixture was concentrated under reduced pressure and the residue was taken up in EtOAc (100 mL) and washed with aq. 1N NaOH (20 mL). The organic layer was collected, dried over Na₂SO₄, filtered and concentrated. The residue was then purified by silica gel chromatography (300 g RediSep Gold column) using an isocratic method of 80% Solvent B in hexanes, where solvent B is ethyl acetate:hexanes:MeOH (9:9:2). This purification separated the two atropisomers: the first atropisomer to elute, (S)-N-((6P)-7-(2-(1-amino-2-(3,5-difluorophenyl)ethyl)-4-(benzyloxy)-6-oxopyrimidin-1(6H)-yl)-4-chloro-1-methyl-1H-indazol-3-yl)-N-(4-methoxybenzyl)methanesulfonamide and its enantiomer was 1.5g; the second atropisomer to elute, (S)-N-((6M)-7-(2-(1-amino-2-(3,5-difluorophenyl)ethyl)-4-(benzyloxy)-6-oxopyrimidin-1(6H)-yl)-4-chloro-1-methyl-1H-indazol-3-yl)-N-(4-methoxybenzyl)methanesulfonamide and its enantiomer was 1.4g. The first eluting atropisomer (desired) was further purified by chiral SFC using the following method: Column = ChiralPak AD-H, 21 × 250 mm; Mobile Phase = 45% Ethanol in CO₂; Flow Rate = 70 mL/min.; Detection = 220 nm; Injection = 2.5 mL of ~125 mg/mL in 3:1 MeOH:DCM. This purification separated two stereoisomers (enantiomers) to provide two isolates of homochiral material:
First stereoisomer to elute, (S)-N-((6P)-7-((1P)-2-(1-amino-2-(3,5-difluorophenyl)ethyl)-4-(benzyloxy)-6-oxopyrimidin-1(6H)-yl)-4-chloro-1-methyl-1H-indazol-3-yl)-N-(4-methoxybenzyl)methanesulfonamide: ¹H NMR (500 MHz, METHANOL-*d*₄) δ ppm 7.54 (d, *J*=7.15 Hz, 2 H), 7.42 - 7.47 (m, 2 H), 7.34 - 7.40 (m, 1 H), 7.23 (br d, *J*=7.15 Hz, 2 H), 7.14 (d, *J*=7.75 Hz, 1 H), 6.70 - 6.88 (m, 3 H), 6.31 - 6.53 (m, 3 H), 5.88 (s, 1 H), 5.58 (d, *J*=12.52 Hz, 1 H), 5.45 (d, *J*=12.52 Hz, 1 H), 3.81 (s, 3 H), 3.73 (br d, *J*=0.60 Hz, 3 H), 3.15 (dd, *J*=12.96, 7.30 Hz, 1 H), 3.02 - 3.11 (m, 3 H), 2.76 - 2.88 (m, 1 H). LC/MS: m/z = 735.10 [M+H]⁺.
Second stereoisomer to elute, (R)-N-((6M)-7-((1M)-2-(1-amino-2-(3,5-difluorophenyl)ethyl)-4-(benzyloxy)-6-oxopyrimidin-1(6H)-yl)-4-chloro-1-methyl-1H-indazol-3-yl)-N-(4-methoxybenzyl)methanesulfonamide: ¹H NMR (500 MHz, METHANOL-*d*₄) δ ppm 7.54 (d, *J*=7.15 Hz, 2 H), 7.42 - 7.47 (m, 2 H), 7.34 - 7.40 (m, 1 H), 7.23 (br d, *J*=7.15 Hz, 2 H), 7.14 (d, *J*=7.75 Hz, 1 H), 6.70 - 6.88 (m, 3 H), 6.31 - 6.53 (m, 3 H), 5.88 (s, 1 H), 5.58 (d, *J*=12.52 Hz, 1 H), 5.45 (d, *J*=12.52 Hz, 1 H), 3.81 (s, 3 H), 3.73 (br d, *J*=0.60 Hz, 3 H), 3.15 (dd, *J*=12.96, 7.30 Hz, 1 H), 3.02 - 3.11 (m, 3 H), 2.76 - 2.88 (m, 1 H). LC/MS: m/z = 735.10 [M+H]⁺.

### Preparation of N-((S)-1-((1P,1P)-4-(benzyloxy)-1-(4-chloro-3-(N-(4-methoxybenzyl)methylsulfonamido)-1-methyl-1H-indazol-7-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

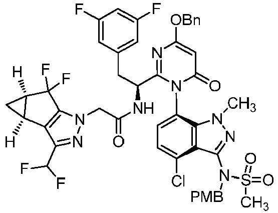

To a solution of (S)-N-((6P)-7-((1P)-2-(1-amino-2-(3,5-difluorophenyl)ethyl)-4-(benzyloxy)-6-oxopyrimidin-1(6H)-yl)-4-chloro-1-methyl-1H-indazol-3-yl)-N-(4-methoxybenzyl)methanesulfonamide (1.28 g, 1.741 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (0.552 g, 2.089 mmol) and HATU (0.794 g, 2.089 mmol) in N,N-Dimethylformamide (DMF) (15 mL) was added DIEA (0.912 mL, 5.22 mmol) at RT. The resulting mixture was stirred at RT for 3.5 hrs. Water was then added and the mixture was extracted with ethyl acetate, washed with brine, dried (Na₂SO₄), filtered and concentrated. The residue was then purified by silica gel chromatography (5-100%, EtOAc in hexane) to afford the title compound N-((S)-1-((1P,1P)-4-(benzyloxy)-1-(4-chloro-3-(N-(4-methoxybenzyl)methylsulfonamido)-1-methyl-1H-indazol-7-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.6 g, 1.630 mmol, 94 % yield) as white solid. ¹H NMR (500 MHz, CDCl₃) δ ppm 7.37 - 7.56 (m, 6 H), 7.30 - 7.36 (m, 2 H), 7.10 - 7.17 (m, 1 H), 6.78 - 6.87 (m, 2 H), 6.74 (tt, *J*=8.75, 2.27 Hz, 1 H), 6.13 - 6.66 (m, 4 H), 5.86 (s, 1 H), 5.23 (q, *J*=12.02 Hz, 2 H), 4.92 - 5.12 (m, 1 H), 4.71 - 4.86 (m, 1 H), 4.48 - 4.68 (m, 3 H), 3.78 (br s, 3 H), 3.62 - 3.74 (m, 2 H), 2.98 (br s, 3 H), 2.69 - 2.84 (m, 1 H), 2.35 - 2.54 (m, 2 H), 1.39 (q, *J*=7.45 Hz, 1 H), 1.10 (br s, 1 H). LC/MS: m/z = 981.05 [M+H]⁺.

### Preparation of N-((S)-1-((1P)-1-(4-chloro-3-(N-(4-methoxybenzyl)methylsulfonamido)-1-methyl-1H-indazol-7-yl)-4-hydroxy-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

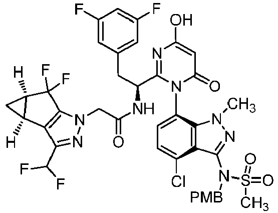

To a mixture N-((S)-1-((1P,1P)-4-(benzyloxy)-1-(4-chloro-3-(N-(4-methoxybenzyl)methylsulfonamido)-1-methyl-1H-indazol-7-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.6 g, 1.630 mmol) in methanol (50 mL) was added 10% Pd on carbon (0.260 g, 0.245 mmol). The mixture was purged with nitrogen gas and then stirred under balloon-pressure hydrogen atmosphere for 1 h. The mixture was purged with nitrogen gas and then was filtered through a pad of Celite. The filtrate was concentrated to afford the title compound N-((S)-1-((1P)-1-(4-chloro-3-(N-(4-methoxybenryl)methylsulfonamido)-1-methyl-1H-indazol-7-yl)-4-hydroxy-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.35 g, 1.515 mmol, 93 % yield) as off-white solid. LC/MS: m/z = 891.10 [M+H]⁺.

### Preparation of (1P)-1-(4-chloro-3-(N-(4-methoxybenryl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate

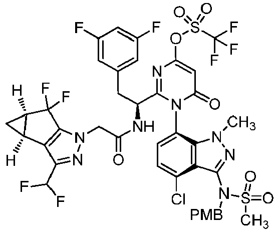

To a solution of N-((S)-1-((1P)-1-(4-chloro-3-(N-(4-methoxybenryl)methylsulfonamido)-1-methyl-1H-indazol-7-yl)-4-hydroxy-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (200 mg, 0.224 mmol) in CH₂Cl₂ (5 mL) at -25 °C was added pyridine (0.091 mL, 1.122 mmol) followed by trifluoromethanesulfonic anhydride (0.047 mL, 0.269 mmol) and the mixture allowed to warm to room temp with stirring for 1 h. Water was then added and the mixture was extracted with DCM, washed with aq. 1N HCl, dried (Na₂SO₄), filtered and concentrated in vacuo to afford the title compound (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (200 mg, 0.195 mmol, 87 % yield) as a brown solid. The material was used in the next step without further purification. LCMS (M+Na) = 1045.05

### Preparation of Example 3: N-((S)-1-((1P)-1-(4-chloro-1-methyl-3-(methylsulfonamido)-1H-indazol-7-yl)-4-(2,4-difluorophenyl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

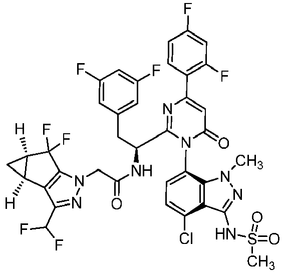

To a solution of (1P)-1-(4-chloro-3-(N-(4-methoxybenryl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (25 mg, 0.024 mmol), (2,4-difluorophenyl)boronic acid (11.57 mg, 0.073 mmol) and K₃PO₄ (15.56 mg, 0.073 mmol) in Tetrahydrofuran (THF) (1 mL)/Water (0.25 mL) was added dichloro[9,9-dimethyl-4,5-bis(diphenylphosphino)xanthene]palladium(II (1.847 mg, 2.443 µmol) and the resulting mixture was stirred at room temp for 16 h. The mixture was then concentrated under reduced pressure and the residue was taken up in TFA (1 mL) and stirred at room temp for 16 h. The reaction mixture was then concentrated in vacuo and the residue was purified by prep-HPLC to afford the title compound N-((S)-1-((1P)-1-(4-chloro-1-methyl-3-(methylsulfonamido)-1H-indazol-7-yl)-4-(2,4-difluorophenyl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (12.9 mg, 0.014 mmol, 57.8 % yield). ¹H NMR (500 MHz, METHANOL-*d*₄) δ ppm 8.42 (td, *J*=8.64, 6.26 Hz, 1 H), 7.29 (d, .7=7.75 Hz, 1 H), 7.16 - 7.24 (m, 3 H), 7.08 (s, 1 H), 6.50 - 6.84 (m, 4 H), 4.45 - 4.57 (m, 2 H), 3.68 (s, 3 H), 3.42 - 3.49 (m, 1 H), 3.23 (s, 3 H), 3.11 (dd, *J*=14.01, 9.54 Hz, 1 H), 2.35 - 2.46 (m, 2 H), 1.31 - 1.40 (m, 1 H), 0.93 - 1.02 (m, 1 H). LCMS Method A: retention time = 1.51 min; *m*/*z* = 867.3 [M+H]⁺.

### Preparation of Example 4: N-((S)-1-((1'P)-1'-(4-chloro-1-methyl-3-(methylsulfonamido)-1H-indazol-7-yl)-4,6-dimethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

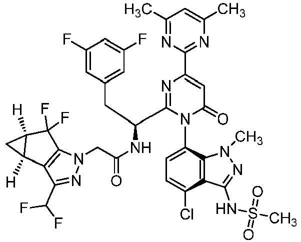

To a mixture of (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (25 mg, 0.024 mmol), 4,6-dimethyl-2-(tributylstannyl)pyrimidine (14.56 mg, 0.037 mmol) and copper(I) iodide (0.465 mg, 2.443 µmol) in DMF (1 mL) was added tetrakis(triphenylphosphine)palladium(0) (2.82 mg, 2.443 µmοl). The mixture was then degassed (vacuum evacuation and refill with argon, repeated three times) and then was heated at 100 °C for 16 h. The mixture was then cooled to RT, filtered and concentrated in vacuo. The residue was taken up in TFA (1 mL) and stirred at room temp for 16 h. The reaction mixture was then concentrated in vacuo and the residue was purified by prep-HPLC to afford the title compound N-((S)-1-((1'P)-1'-(4-chloro-1-methyl-3-(methylsulfonamido)-1H-indazol-7-yl)-4,6-dimethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (9 mg, 9.93 µmοl, 40.6 % yield). ¹H NMR (500 MHz, METHANOL-*d*₄) δ ppm 7.69 (s, 1 H), 7.43 (s, 1 H), 7.30 (d, *J*=7.75 Hz, 1 H), 7.13 (d, *J*=8.05 Hz, 1 H), 6.50 - 6.85 (m, 4 H), 4.64 - 4.72 (m, 2 H), 3.62 (s, 3 H), 3.39 (dd, *J*=14.01, 5.07 Hz, 1 H), 3.22 (s, 3 H), 3.08 (dd, *J*=14.01, 9.24 Hz, 1 H), 2.66 (s, 6 H), 2.35 - 2.45 (m, 2 H), 1.32 - 1.40 (m, 1 H), 1.03 (s, 1 H). LCMS Method A: retention time = 1.40 min; *m*/*z* = 861.4 [M+H]⁺.

### Preparation of 4-(difluoromethyl)-2-(tributylstannyl)pyrimidine

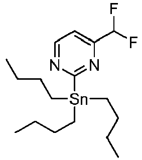

2-chloro-4-(difluoromethyl)pyrimidine (0.80 g, 4.9 mmol), 1,1,1,2,2,2-hexabutyldistannane (2.95 ml, 5.83 mmol), and Pd(Ph₃P)₄ (0.562 g, 0.486 mmol) were combined under Ar with DMF (24 ml). The reaction was degassed with Ar and then stirred at 110°C overnight (approximately 18 hours). The reaction was diluted with EtOAc and washed with water and then brine. The organic phase was concentrated, adsorbed onto Celite, and the resulting powder was subjected to silica gel chromatography (80g column) using a gradient of 0-50% EtOAc in hexanes. This purification afforded 4-(difluoromethyl)-2-(tributylstannyl)pyrimidine contaminated with 1 equiv of triphenylphosphane (1.0 g, 1.47 mmol, 30.2 % yield). LCMS Method B: retention time = 4.546 min, observed mass = 421.05 (M+H). ¹H NMR (500 MHz, CDCl3, 303 K) δ (ppm) = 8.88 - 8.80 (m, 1H), 7.42 - 7.38 (m, 1H), 6.65 - 6.36 (m, 1H), 1.61 - 1.55 (m, 7H), 1.32 - 1.30 (m, 5H), 1.21 - 1.16 (m, 5H), 0.90 - 0.86 (m, 10H).

### Preparation of Example 5: N-((S)-1-((1'P)-1'-(4-chloro-1-methyl-3-(methylsulfonamido)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

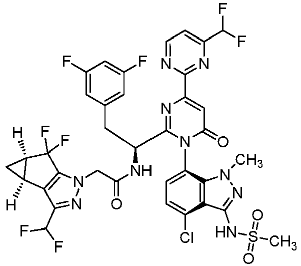

To a mixture of (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (25 mg, 0.024 mmol), 4-(difluoromethyl)-2-(tributylstannyl)pyrimidine (15.36 mg, 0.037 mmol) and copper(I) iodide (0.465 mg, 2.443 µmol) in DMF (1 mL) was added tetrakis(triphenylphosphine)palladium(0) (2.82 mg, 2.443 µmοl). The mixture was then degassed (vacuum evacuation and refill with argon, repeated three times) and then was heated at 100 °C for 16 h. The mixture was then cooled to RT, filtered and concentrated in vacuo. The residue was taken up in TFA (1 mL) and stirred at room temp for 16 h. The reaction mixture was then concentrated in vacuo and the resulting residue was purified by prep-HPLC to afford the title compound N-((S)-1-((1'P)-1'-(4-chloro-1-methyl-3-(methylsulfonamido)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (10 mg, 10.76 µmοl, 44.0 % yield). ¹H NMR (500 MHz, METHANOL-*d*₄) δ ppm 9.31 (d, *J*=5.07 Hz, 1 H), 7.94 (d, *J*=5.07 Hz, 1 H), 7.76 (s, 1 H), 7.32 (d, *J*=7.75 Hz, 1 H), 7.21 (d, *J*=8.05 Hz, 1 H), 6.42 - 7.07 (m, 5 H), 4.45 - 4.57 (m, 2 H), 3.63 (s, 3 H), 3.38 - 3.45 (m, 1 H), 3.22 (s, 3 H), 3.12 (dd, *J*=14.01, 9.54 Hz, 1 H), 2.32 - 2.46 (m, 2 H), 1.32 - 1.38 (m, 1 H), 0.94 - 1.01 (m, 1 H). LCMS Method A: retention time = 1.38 min; *m*/*z=* 883.3 [M+H]⁺.

### Preparation of 2-(tributylstannyl)-4-(trifluoromethyl)pyrimidine

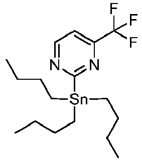

2-chloro-4-(trifluoromethyl)pyrimidine (0.662 ml, 5.48 mmol), 1,1,1,2,2,2-hexabutyldistannane (3.32 ml, 6.57 mmol), and Pd(Ph₃P)₄ (0.633 g, 0.548 mmol) were combined under Ar with DMF (27 ml). The reaction was degassed with Ar and then was stirred at 110°C overnight (approximately 18 hours). The reaction was diluted with EtOAc and washed with water and then brine. The organic phase was concentrated, adsorbed onto Celite, and the resulting powder was subjected to silica gel chromatography (80g column) running a gradient of 0-25% EtOAc in hexanes to afford 2-(tributylstannyl)-4-(trifluoromethyl)pyrimidine contaminated with 1 equiv of triphenylphosphane (1.0 g, 1.430 mmol, 26.1 % yield). LCMS Method B: retention time = 4.839 min, observed mass = 439.0 (M+H). ¹H NMR (500 MHz, CDCl3, 303 K) δ (ppm) = 8.91 (d, J = 5.4 Hz, 1H), 7.42 (d, J = 5.1 Hz, 1H), 1.62 - 1.58 (m, 3H), 1.38 - 1.28 (m, 10H), 1.23 - 1.17 (m, 5H), 0.90 - 0.86 (m, 9H).

### Preparation of Example 6: N-((S)-1-((1'P)-1'-(4-chloro-1-methyl-3-(methylsulfonamido)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

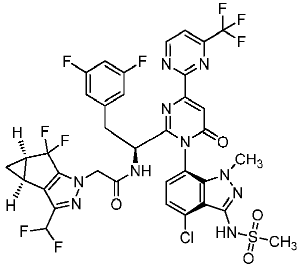

To a mixture of (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (25 mg, 0.024 mmol), 2-(tributylstannyl)-4-(trifluoromethyl)pyrimidine (16.02 mg, 0.037 mmol) and copper(I) iodide (0.465 mg, 2.443 µmol) in DMF (1 mL) was added tetrakis(triphenylphosphine)palladium(0) (2.82 mg, 2.443 µmοl). The mixture was then degassed (vacuum evacuation and refill with argon, repeated three times) and then was heated at 100 °C for 16 h. The mixture was then cooled to RT, filtered and concentrated in vacuo. The residue was taken up in TFA (1 mL) and stirred at room temp for 16 h. The reaction mixture was then concentrated in vacuo and the residue purified by prep-HPLC to afford the title compound N-((S)-1-((1'P)-1'-(4-chloro-1-methyl-3-(methylsulfonamido)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (13 mg, 0.014 mmol, 56.1 % yield). ¹H NMR (500 MHz, METHANOL-*d*₄) δ ppm 9.41 (d, *J*=5.07 Hz, 1 H), 8.08 (d, *J*=5.07 Hz, 1 H), 7.74 (s, 1 H), 7.15 - 7.35 (m, 2 H), 6.52 - 6.85 (m, 4 H), 4.45 - 4.57 (m, 2 H), 3.65 (s, 3 H), 3.40 - 3.48 (m, 1 H), 3.23 (s, 3 H), 3.12 (dd, *J*=14.01, 9.54 Hz, 1 H), 2.32 - 2.46 (m, 2 H), 1.32 - 1.38 (m, 1 H), 0.93 - 1.00 (m, 1 H). LCMS Method A: retention time = 1.43 min; *m*/*z=* 901.4 [M+H]⁺.

### Preparation of Example 7: N-((S)-1-((1P)-1-(4-chloro-1-methyl-3-(methylsulfonamido)-1H-indazol-7-yl)-6-oxo-4-(6-(trifluoromethyl)pyridin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

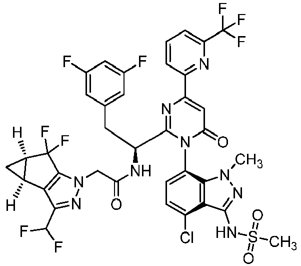

To a mixture of (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (25 mg, 0.024 mmol), 2-(tributylstannyl)-6-(trifluoromethyl)pyridine (15.98 mg, 0.037 mmol) and copper(I) iodide (0.465 mg, 2.443 µmol) in DMF (1 mL) was added tetrakis(triphenylphosphine)palladium(0) (2.82 mg, 2.443 µmοl). The mixture was then degassed (vacuum evacuation and refill with argon, repeated three times) and then was heated at 100 °C for 16 h. The mixture was then cooled to RT, filtered and concentrated in vacuo. The resulting residue was taken up in TFA (1 mL) and stirred at room temp for 16 h. The reaction mixture was then concentrated in vacuo and the resulting residue was purified by prep-HPLC to afford the desired product N-((S)-1-((1P)-1-(4-chloro-1-methyl-3-(methylsulfonamido)-1H-indazol-7-yl)-6-oxo-4-(6-(trifluoromethyl)pyridin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (13 mg, 0.014 mmol, 56.2 % yield). ¹H NMR (500 MHz, METHANOL-*d*₄) δ ppm 8.79 (d, *J*=7.75 Hz, 1 H), 8.28 (t, *J*=7.75 Hz, 1 H), 7.99 (dd, *J*=8.05, 0.89 Hz, 1 H), 7.60 (s, 1 H), 7.20 - 7.34 (m, 2 H), 6.52 - 6.85 (m, 4 H), 4.45 - 4.57 (m, 2 H), 3.67 (s, 3 H), 3.50 (dd, *J*=14.01, 4.47 Hz, 1 H), 3.23 (s, 3 H), 3.16 (dd, *J*=14.16, 9.69 Hz, 1 H), 2.35 - 2.43 (m, 2 H), 1.31 - 1.39 (m, 1 H), 0.94 - 1.00 (m, 1 H). LCMS Method A: retention time = 1.51 min; *m*/*z* = 900.3 [M+H]⁺.

### Preparation of 1-(2,2-difluoropropyl)-3-(tributylstannyl)-1H-pyrazole

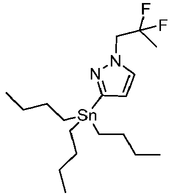

To a sealed tubed charged with 3-bromo-1-(2,2-difluoropropyl)-1H-pyrazole (300 mg, 1.333 mmol), 1,1,1,2,2,2-hexabutyldistannane (2.021 mL, 4.00 mmol), and tetrakis(triphenylphosphine)palladium(0) (154 mg, 0.133 mmol) under Ar was added toluene (12 mL). The mixture was degassed (brief high vacuum, then refilled with Ar) and heated at 110 °C for 16 h. The reaction was diluted with EtOAc and washed with water and then brine. The organic phase was concentrated, adsorbed onto Celite, and the resulting powder was subjected to silica gel chromatography (0-15%, EtOAc in hexanes) to afford the title compound 1-(2,2-difluoropropyl)-3-(tributylstannyl)-1H-pyrazole (250 mg, 0.574 mmol, 43.1 % yield) as a clear viscous oil contaminated with triphenylphosphine. The product was in the next step without further purification. LCMS (M+H) = 437.05

### Preparation of Example 8: N-((S)-1-((1P)-1-(4-chloro-1-methyl-3-(methylsulfonamido)-1H-indazol-7-yl)-4-(1-(2,2-difluoropropyl)-1H-pyrazol-3-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

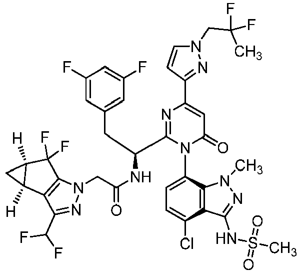

To a mixture of (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (25 mg, 0.024 mmol), 1-(2,2-difluoropropyl)-3-(tributylstannyl)-1H-pyrazole (15.95 mg, 0.037 mmol) and copper(I) iodide (0.465 mg, 2.443 µmol) in DMF (1 mL) was added tetrakis(triphenylphosphine)palladium(0) (2.82 mg, 2.443 µmοl). The mixture was then degassed (vacuum evacuation and refill with argon, repeated three times) and then was heated at 100 °C for 16 h. The mixture was then cooled to RT, filtered and concentrated in vacuo. The residue was taken up in TFA (1 mL) and stirred at room temp for 16 h. The reaction mixture was then concentrated and the resulting residue was purified by prep-HPLC to afford the title compound (13 mg, 0.014 mmol, 56.2 % yield). ¹H NMR (500 MHz, METHANOL-*d*₄) δ ppm 7.86 (d, *J*=2.38 Hz, 1 H), 7.27 (d, *J*=7.75 Hz, 1 H), 7.12 - 7.15 (m, 2 H), 7.10 (s, 1 H), 6.54 - 6.82 (m, 4 H), 4.72 (t, *J*=12.82 Hz, 2 H), 4.49 - 4.57 (m, 2 H), 3.66 (s, 2 H), 3.41 - 3.46 (m, 1 H), 3.22 (s, 3 H), 3.05 - 3.11 (m, 1 H), 2.35 - 2.44 (m, 2 H), 1.67 (t, *J*=18.78 Hz, 3 H), 1.31 - 1.38 (m, 1 H), 0.97 - 1.01 (m, 1 H). LCMS Method A: retention time = 1.40 min; *m*/*z=* 899.4 [M+H]⁺.

### Preparation of tert-butyl (S)-(1-(1-(4-chloro-3-(N-(4-methoxybenzyl)methylsulfonamido)-1-methyl-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)carbamate

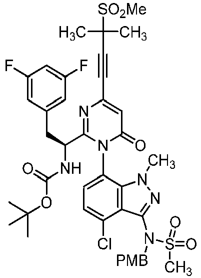

A vial was charged with (S)-2-(1-((tert-butoxycarbonyl)amino)-2-(3,5-difluorophenyl)ethyl)-1-(4-chloro-3-(N-(4-methoxybenzyl)methylsulfonamido)-1-methyl-1H-indazol-7-yl)-6-oxo-1,6-dihydropyrimidin-4-yl trifluoromethanesulfonate (150 mg, 0.171 mmol), 3-methyl-3-(methylsulfonyl)but-1-yne (30.0 mg, 0.205 mmol), DMF (2 mL), triethylamine (0.071 mL, 0.513 mmol), copper(I) iodide (3.26 mg, 0.017 mmol) and bis(triphenylphosphine)palladium(II) chloride (12.00 mg, 0.017 mmol). The mixture was degassed (brief high vacuum, then refilled with Ar) and then heated at 60 °C for 1h. The mixture was then cooled to room temperature; diluted with water; extracted with ethyl acetate; dried (Na₂SO₄); filtered and the filtrate was concentrated in vacuo. The residue was then subjected to silica chromatography eluting with 5-100% EtOAc in hexanes to afford tert-butyl (S)-(1-(1-(4-chloro-3-(N-(4-methoxybenryl)methylsulfonamido)-1-methyl-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)carbamate (100 mg, 67% yield). LCMS m/z = 817.20 (M-tBu).

### Preparation of (S)-N-((6P) -7-(2-(1-amino-2-(3,5-difluorophenyl)ethyl)-4-(3-methyl-3-(methylsulfonyl)but-1-yn-1-yl)-6-oxopyrimidin-1(6H)-yl)-4-chloro-1-methyl-1H-indazol-3-yl)methanesulfonamide

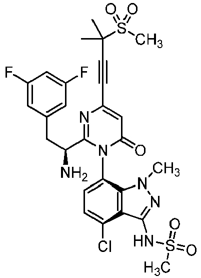

To a stirred solution of tert-butyl (5)-(1-(1-(4-chloro-3-(N-(4-methoxybenryl)methylsulfonamido)-1-methyl-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)carbamate (90 mg, 0.103 mmol) in DCM (2 mL) was added TFA (1 mL, 12.98 mmol) followed by triflic acid (0.018 mL, 0.206 mmol). The resulting solution was stirred at room temp for 2h. The mixture was concentrated under reduced pressure and the residue was taken up in EtOAc (50 mL) and then washed with aq. 1N NaOH (5 mL). The organic layer was collected; dried over Na₂SO₄; filtered and the filtrate was concentrated under reduced pressure. The residue was subjected to C18 column chromatography (50 g RediSep Gold column) eluting with 10-60% Mobile Phase A in Mobile Phase B over 30 minutes; Mobile Phase A = 5:95 acetonitrile:water with 0.1 % Formic acid; Mobile Phase B = 95:5 acetonitrile:water with 0.1 % Formic acid. This purification separated the two diastereomers (atropisomers) present in the sample: Fractions containing the second-eluting diastereomer were combined and the solution was adjusted to pH 8 using 1N NaOH. The aq. mixture was extracted with ethyl acetate; washed with brine; dried (Na₂SO₄); filtered and the filtrate was concentrated under reduced pressure to afford (S)-N-((6P)-7-(2-(1-amino-2-(3,5-difluorophenyl)ethyl)-4-(3-methyl-3-(methylsulfonyl)but-1-yn-1-yl)-6-oxopyrimidin-1(6H)-yl)-4-chloro-1-methyl-1H-indazol-3-yl)methanesulfonamide and its enantiomer (26 mg). ¹H NMR (500 MHz, METHANOL-*d*₄) δ ppm 7.17 (d, *J*=8.05 Hz, 1 H), 6.81 (d, *J*=7.75 Hz, 1 H), 6.71 - 6.76 (m, 1 H), 6.70 (s, 1 H), 6.46 - 6.51 (m, 2 H), 3.70 (s, 3 H), 3.56 (dd, *J*=7.60, 5.81 Hz, 1 H), 3.18 (s, 3 H), 3.14 - 3.17 (m, 1 H), 3.09 (s, 3 H), 2.76 (dd, *J*=13.41, 7.75 Hz, 1 H), 1.67 (s, 6 H). LCMS (M+H)+ = 653.10

### Preparation of tert-butyl (1-((4-chloro-1-(2,2-difluoroethyl)-3-(N-(4-methoxybenzyl)cyclopropanesulfonamido)-1H-indazol-7-yl)amino)-3-( 3,5-difluorophenyl)-1-iminopropan-2-yl)carbamate

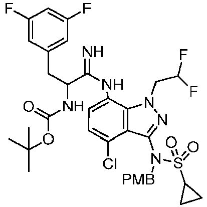

A solution of ethyl (S)-2-((tert-butoxycarbonyl)amino)-3-(3,5-difluorophenyl)propanimidate (7.64 g, 23.25 mmol) and N-(7-amino-4-chloro-1-(2,2-difluoroethyl)-1H-indazol-3-yl)-N-(4-methoxybenryl)cyclopropanesulfonamide (7.3 g, 15.50 mmol) in acetonitrile (100 mL) and acetic acid (1.331 mL, 23.25 mmol) was stirred at room temperature for 24 h. To the solution was added ethyl (S)-2-((tert-butoxycarbonyl)amino)-3-(3,5-difluorophenyl)propanimidate (2.0 g) and acetic acid (0.35 mL) and the mixture was stirred at room temperature for 16 h. The mixture was diluted with ethyl acetate and then washed with aq.1N Na₂CO₃ solution followed by brine; dried (Na₂SO₄); filtered and the filtrate was concentrated in vacuo. The residue was subjected to silica gel chromatography eluting with 5-70% EtOAc in hexanes to afford the title compound tert-butyl (1-((4-chloro-1-(2,2-difluoroethyl)-3-(N-(4-methoxybenzyl)cyclopropanesulfonamido)-1H-indazol-7-yl)amino)-3-(3,5-difluorophenyl)-1-iminopropan-2-yl)carbamate (7.5 g, 64% yield). LC/MS m/z = 753.15 [M+H]⁺.

### Preparation of tert-butyl (1-(1-(4-chloro-1-(2,2-difluoroethyl)-3-(N-(4-methoxybenzyl)cyclopropanesulfonamido)-1H-indazol-7-yl)-4-hydroxy-6-ox o-1,6-dihydropyrimidin-2-yl)-2-(3,5-difluorophenyl)ethyl)carbamate

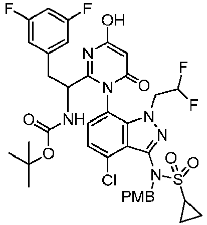

To a solution of tert-butyl (1-((4-chloro-1-(2,2-difluoroethyl)-3-(N-(4-methoxybenryl)cyclopropanesulfonamido)-1H-indazol-7-yl)amino)-3-(3,5-difluorophenyl)-1-iminopropan-2-yl)carbamate (6.6 g, 8.76 mmol) in THF (100 mL) was added bis(2,4,6-trichlorophenyl) malonate (6.08 g, 13.14 mmol). The resulting mixture was stirred at room temp for 48 h. The mixture was then concentrated under reduced pressure and the residue was subjected to silica gel chromatography eluting with 0-100% EtOAC in hexanes to afford the title compound tert-butyl (1-(1-(4-chloro-1-(2,2-difluoroethyl)-3-(N-(4-methoxybenzyl)cyclopropanesulfonamido)-1H-indazol-7-yl)-4-hydroxy-6-oxo-1,6-dihydropyrimidin-2-yl)-2-(3,5-difluorophenyl)ethyl)carbamate (4.3 g, 60% yield) as a mixture of enantiomers. LCMS m/z = 764.98 (M-tBu).

### Preparation of tert-butyl (1-(4-(benzyloxy)-1-(4-chloro-1-(2,2-difluoroethyl)-3-(N-(4-methoxybenzyl)cyclopropanesulfonamido)-1H-indazol-7-yl)-6-oxo-1,6-dihydropyrimidi n-2-yl)-2-(3,5-difluorophenyl)ethyl)carbamate

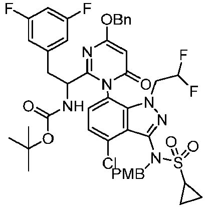

To a solution of tert-butyl (1-(1-(4-chloro-1-(2,2-difluoroethyl)-3-(N-(4-methoxybenzyl)cyclopropanesulfonamido)-1H-indazol-7-yl)-4-hydroxy-6-oxo-1,6-dihydropyrimidin-2-yl)-2-(3,5-difluorophenyl)ethyl)carbamate (4.3 g, 5.24 mmol) in DMF (40 ml) was added K₂CO₃ (1.085 g, 7.85 mmol) followed by (bromomethyl)benzene (0.934 mL, 7.85 mmol) and the resulting mixture was stirred at room temp for 5 h. The mixture was diluted with water and then was extracted with ethyl acetate; washed with brine; dried (Na₂SO₄); filtered and the filtrate was concentrated in vacuo. The resulting residue was subjected to silica gel chromatography eluting with 5-70% EtOAc in hexanes to afford the title compound tert-butyl (1-(4-(benzyloxy)-1-(4-chloro-1-(2,2-difluoroethyl)-3-(N-(4-methoxybenzyl)cyclopropanesulfonamido)-1H-indazol-7-yl)-6-oxo-1,6-dihydropyrimidin-2-yl)-2-(3,5-difluorophenyl)ethyl)carbamate (4.0 g, 84% yield) as a mixture of enantiomers. LC/MS m/z = 911.10 [M+H]⁺.

### Preparation of (S)-N-((6P)-7-((1P)-2-(1-amino-2-(3,5-difluorophenyl)ethyl)-4-(benzyloxy)-6-oxopyrimidin-1(6H)-yl)-4-chloro-1-(2,2-difluoroethyl)-1H-indazol-3-yl)-N-(4-methoxybenzyl)cyclopropanesulfonamide

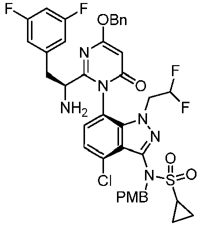

A mixture of tert-butyl (1-(4-(benryloxy)-1-(4-chloro-1-(2,2-difluoroethyl)-3-(N-(4-methoxybenryl)cyclopropanesulfonamido)-1H-indazol-7-yl)-6-oxo-1,6-dihydropyrimidin-2-yl)-2-(3,5-difluorophenyl)ethyl)carbamate (3.9 g, 4.28 mmol) and HCl (4M in dioxane, 20 mL, 80 mmol) was stirred at room temp for 2 h. The mixture was concentrated under reduced pressure and the residue was taken up in EtOAc (200 mL) and then washed with aq. 1N NaOH (20 mL). The organic layer was isolated; dried over Na₂SO₄; filtered and the filtrate was concentrated in vacuo. The residue was then purified by chiral SFC using the following method: Column = ChiralPak AD-H 21 × 250 mm; Mobile Phase = 40% 2-propanol in CO₂; Flow Rate = 70 mL/min.; Detection = 220 nm; Injection = 2 mL of ~60 mg/mL in MeOH. This purification separated two stereoisomers (enantiomers) to provide two isolates of homochiral material:
First stereoisomer to elute: (S)-N-((6P)-7-((1P)-2-(1-amino-2-(3,5-difluorophenyl)ethyl)-4-(benzyloxy)-6-oxopyrimidin-1(6H)-yl)-4-chloro-1-(2,2-difluoroethyl)-1H-indazol-3-yl)-N-(4-methoxybenzyl)cyclopropanesulfonamide (2.31 g, 2.85 mmol, 66.5 % yield). ¹H NMR (500 MHz, DMSO-*d*₆) δ ppm 7.49 - 7.55 (m, 2 H), 7.45 (br t, *J*=7.45 Hz, 2 H), 7.37 - 7.42 (m, 1 H), 7.34 (d, *J*=8.05 Hz, 1 H), 7.00 - 7.28 (m, 4 H), 6.76 - 6.84 (m, 1 H), 6.58 - 6.72 (m, 3 H), 6.16 - 6.48 (m, 1 H), 5.85 - 5.98 (m, 1 H), 5.42 - 5.46 (m, 1 H), 5.34 - 5.40 (m, 1 H), 4.85 (br d, *J*=14.31 Hz, 2 H), 4.32 - 4.52 (m, 2 H), 3.68 (br s, 1 H), 3.57 (br s, 2 H), 3.12 (br dd, *J*=13.11, 4.17 Hz, 1 H), 2.79 - 2.87 (m, 1 H), 2.70 - 2.78 (m, 1 H), 1.73 - 2.01 (m, 2 H), 0.83 - 1.09 (m, 5 H). LC/MS: m/z = 811.0 [M+H]⁺.
Second stereoisomer to elute, (R)-N-((6P)-7-((1P)-2-(1-amino-2-(3,5-difluorophenyl)ethyl)-4-(benzyloxy)-6-oxopyrimidin-1(6H)-yl)-4-chloro-1-(2,2-difluoroethyl)-1H-indazol-3-yl)-N-(4-methoxybenzyl)cyclopropanesulfonamide (1.79 g, 2.206 mmol, 51.6 % yield). ¹H NMR (500 MHz, DMSO-*d*₆) δ ppm 7.49 - 7.55 (m, 2 H), 7.45 (br t, *J*=7.45 Hz, 2 H), 7.37 - 7.42 (m, 1 H), 7.34 (d, *J*=8.05 Hz, 1 H), 7.00 - 7.28 (m, 4 H), 6.76 - 6.84 (m, 1 H), 6.58 - 6.72 (m, 3 H), 6.16 - 6.48 (m, 1 H), 5.85 - 5.98 (m, 1 H), 5.42 - 5.46 (m, 1 H), 5.34 - 5.40 (m, 1 H), 4.85 (br d, *J*=14.31 Hz, 2 H), 4.32 - 4.52 (m, 2 H), 3.68 (br s, 1 H), 3.57 (br s, 2 H), 3.12 (br dd, *J*=13.11, 4.17 Hz, 1 H), 2.79 - 2.87 (m, 1 H), 2.70 - 2.78 (m, 1 H), 1.73 - 2.01 (m, 2 H), 0.83 - 1.09 (m, 5 H). LC/MS: m/z = 811.0 [M+H]⁺.

### Preparation of N-((S)-1-((1P,1P)-4-(benzyloxy)-1-(4-chloro-1-(2,2-difluoroethyl)-3-(N-(4-methoxybenzyl)cyclopropanesulfonamido)-1H-indazol-7-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

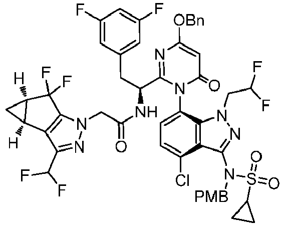

To a solution of (S)-N-((6P)-7-((1P)-2-(1-amino-2-(3,5-difluorophenyl)ethyl)-4-(benzyloxy)-6-oxopyrimidin-1(6H)-yl)-4-chloro-1-(2,2-difluoroethyl)-1H-indazol-3-yl)-N-(4-methoxybenzyl)cyclopropanesulfonamide (1 g, 1.180 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (0.374 g, 1.416 mmol) and HATU (0.538 g, 1.416 mmol) in DMF (8 mL) was added DIEA (0.618 mL, 3.54 mmol). The resulting mixture was stirred at room temperature for 3.5 hrs. The mixture was diluted with water and then was extracted with ethyl acetate; washed with brine; dried (Na₂SO₄); filtered and the filtrate was concentrated in vacuo. The residue was subjected to silica gel chromatography eluting with 5-100% EtOAc in hexanes to afford the title compound N-((S)-1-((1P,1P)-4-(benzyloxy)-1-(4-chloro-1-(2,2-difluoroethyl)-3-(N-(4-methoxybenryl)cyclopropanesulfonamido)-1H-indazol-7-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.2 g, 96% yield) as a white solid. LC/MS m/z = 1057.0 [M+H]⁺.

### Preparation of N-((S)-1-((1P)-1-(4-chloro-3-(cyclopropanesulfonamido)-1-(2,2-difluoroethyl)-1H-indazol-7-yl)-4-hydroxy-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

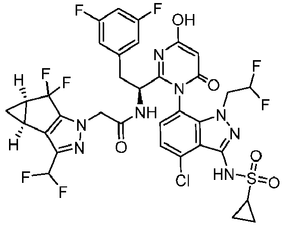

To a stirring solution of N-((S)-1-((1P,1P)-4-(benzyloxy)-1-(4-chloro-1-(2,2-difluoroethyl)-3-(N-(4-methoxybenzyl)cyclopropanesulfonamido)-1H-indazol-7-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (200 mg, 0.189 mmol) in DCM (2 mL) was added TFA (0.291 mL, 3.78 mmol) followed by triflic acid (0.050 mL, 0.567 mmol). The dark red solution was stirred at room temp for 1 h, then the solution was concentrated under reduced pressure. The resulting residue was subjected to silica gel chromatography eluting with 5-100% EtOAc in hexanes to afford the title compound N-((S)-1-((1P)-1-(4-chloro-3-(cyclopropanesulfonamido)-1-(2,2-difluoroethyl)-1H-indazol-7-yl)-4-hydroxy-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (120 mg, 75% yield) as white solid. ¹H NMR (500 MHz, METHANOL-*d*₄) δ ppm 8.68 (br d, *J*=8.05 Hz, 1 H), 7.30 (d, *J*=7.75 Hz, 1 H), 7.02 (d, *J*=7.75 Hz, 1 H), 6.51 - 6.84 (m, 4 H), 5.95 - 6.24 (m, 1 H), 5.69 (s, 1 H), 4.60 - 4.66 (m, 1 H), 4.49 - 4.59 (m, 2 H), 4.32 - 4.43 (m, 1 H), 3.93 - 4.04 (m, 1 H), 3.00 (br dd, *J*=13.71, 9.54 Hz, 1 H), 2.82 - 2.92 (m, 1 H), 2.43 (br s, 2 H), 1.36 (q, *J*=6.85 Hz, 1 H), 1.04 - 1.11 (m, 2 H), 0.91 - 1.03 (m, 3 H). LC/MS *m*/*z=* 847.95 [M+H]⁺.

### Preparation of (1P)-1-(4-chloro-3-(cyclopropanesulfonamido)-1-(2,2-difluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate

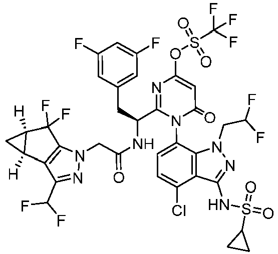

To a solution of N-((S)-1-((1P)-1-(4-chloro-3-(cyclopropanesulfonamido)-1-(2,2-difluoroethyl)-1H-indazol-7-yl)-4-hydroxy-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (110 mg, 0.130 mmol) in DCM (2 mL) at -25 °C was added pyridine (0.053 mL, 0.649 mmol) followed by trifluoromethanesulfonic anhydride (0.027 mL, 0.156 mmol). The mixture was allowed to warm to room temp with stirring for 1 h. The mixture was diluted with water and then was extracted with DCM; washed with aq. 1N HCl; dried (Na₂SO₄); filtered and the filtrate was concentrated in vacuo to afford the title compound (1P)-1-(4-chloro-3-(cyclopropanesulfonamido)-1-(2,2-difluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (110 mg, 87% yield) as a brown solid . The material was used in the next step without further purification. The sample was analyzed by LCMS Method B: retention time = 3.48 min; *m*/*z=* 979.10 [M+H]⁺.

### Preparation of N-((S)-1-((1P,1P)-4-(benzyloxy)-1-(4-chloro-1-(2,2-difluoroethyl)-3-(N-(4-methoxybenzyl)cyclopropanesulfonamido)-1H-indazol-7-yl)-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide

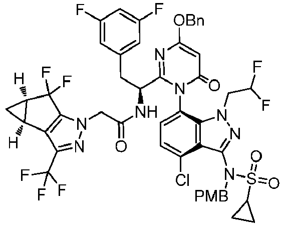

To a solution of (S)-N-((6P)-7-((1P)-2-(1-amino-2-(3,5-difluorophenyl)ethyl)-4-(benzyloxy)-6-oxopyrimidin-1(6H)-yl)-4-chloro-1-(2,2-difluoroethyl)-1H-indazol-3-yl)-N-(4-methoxybenzyl)cyclopropanesulfonamide (200 mg, 0.236 mmol), 2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetic acid (80 mg, 0.283 mmol) and HATU (108 mg, 0.283 mmol) in DMF (3 mL) was added DIEA (0.124 mL, 0.708 mmol). The resulting mixture was stirred at room temperature for 3.5 hrs. The mixture was diluted with water was then was extracted with ethyl acetate; washed with brine; dried (Na₂SO₄); filtered and the filtrate was concentrated in vacuo. The residue was subjected to silica gel chromatography eluting with 5-100% EtOAc in hexanes to afford the title compound N-((S)-1-((1P,1P)-4-(benzyloxy)-1-(4-chloro-1-(2,2-difluoroethyl)-3-(N-(4-methoxybenzyl)cyclopropanesulfonamido)-1H-indazol-7-yl)-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide (240 mg, 95% yield). The sample was analyzed by LCMS Method B: retention time = 3.80 min; *m*/*z* = 1075.0 [M+H]⁺.

### Preparation of N-((S)-1-((1P)-1-(4-chloro-1-(2,2-difluoroethyl)-3-(N-(4-methoxybenzyl)cyclopropanesulfonamido)-1H-indazol-7-yl)-4-hydroxy-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide

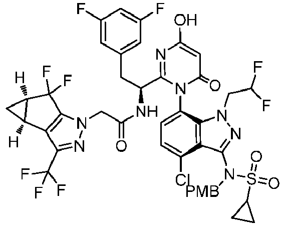

To a solution of N-((S)-1-((1P,1P)-4-(benzyloxy)-1-(4-chloro-1-(2,2-difluoroethyl)-3-(N-(4-methoxybenzyl)cyclopropanesulfonamido)-1H-indazol-7-yl)-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide (240 mg, 0.223 mmol) in methanol (5 mL) was added 10% palladium on carbon (23.75 mg, 0.022 mmol). The mixture was purged with hydrogen and then stirred under balloon-pressure hydrogen atmosphere for 30 min. The mixture was filtered through a pad of Celite and the filtrate was concentrated under reduced pressure to afford the title compound N-((S)-1-((1P)-1-(4-chloro-1-(2,2-difluoroethyl)-3-(N-(4-methoxybenzyl)cyclopropanesulfonamido)-1H-indazol-7-yl)-4-hydroxy-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide (220 mg, 100% yield). The product was used in the next step without additional purification. The product was analyzed by LCMS Method B: retention time = 3.56 min; *m*/*z* = 985.05 [M+H]⁺.

### Preparation of (1P)-1-(4-chloro-1-(2,2-difluoroethyl)-3-(N-(4-methoxybenryl)cyclopropanesulfonamido)-1H-indazol-7-yl)-2-((S)-1-(2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate

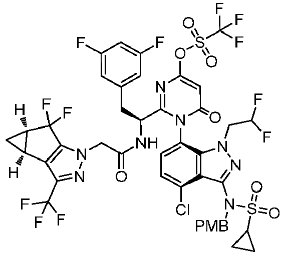

To a solution of N-((S)-1-((1P)-1-(4-chloro-1-(2,2-difluoroethyl)-3-(N-(4-methoxybenzyl)cydopropanesulfonamido)-1H-indazol-7-yl)-4-hydroxy-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide (160 mg, 0.162 mmol) in DCM (3 mL) at -25 °C was added pyridine (0.066 mL, 0.812 mmol) followed by trifluoromethanesulfonic anhydride (0.031 mL, 0.179 mmol). The mixture was allowed to warm to room temp with stirring for 1 h. The mixture was diluted with water then was extracted with DCM; washed with aq. 1N HCl; dried (Na₂SO₄); filtered and the filtrate was concentrated in vacuo to afford the title (1P)-1-(4-chloro-1-(2,2-difluoroethyl)-3-(N-(4-methoxybenryl)cyclopropanesulfonamido)-1H-indazol-7-yl)-2-((S)-1-(2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (170 mg, 94% yield) as a brown solid. The product was used in the next step without additional purification. The product was analyzed by LCMS Method B: retention time = 3.87 min; *m*/*z=* 1117.2 [M+H]⁺.

### Preparation of (1P)-1-(4-chloro-1-(2,2-difluoroethyl)-3-(methylsulfonamido)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate

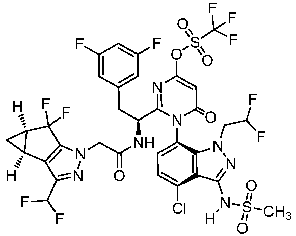

The title compound was prepared following the route and procedures used to prepare (1P)-1-(4-chloro-3-(cyclopropanesulfonamido)-1-(2,2-difluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate substituting N-(7-amino-4-chloro-1-(2,2-difluoroethyl)-1H-indazol-3-yl)-N-(4-methoxybenzyl)methanesulfonamide for N-(7-amino-4-chloro-1-(2,2-difluoroethyl)-1H-indazol-3-yl)-N-(4-methoxybenzyl)cyclopropanesulfonamide.

### Preparation of (1P)-1-(4-chloro-3-(methylsulfonamido)-1-(2,2,2-trifluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate

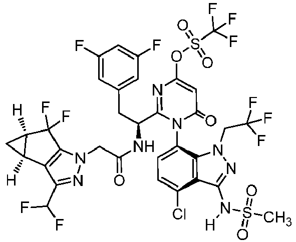

The title compound was prepared following the route and procedures used to prepare (1P)-1-(4-chloro-3-(cyclopropanesulfonamido)-1-(2,2-difluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate substituting N-(7-amino-4-chloro-1-(2,2,2-trifluoroethyl)-1H-indazol-3-yl)-N-(4-methoxybenzyl)methanesulfonamide for N-(7-amino-4-chloro-1-(2,2-difluoroethyl)-1H-indazol-3-yl)-N-(4-methoxybenzyl)cyclopropanesulfonamide.

### Preparation of 5-fluoro-2-(tributylstannyl)pyrimidine

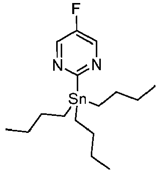

To a sealed tube was added 2-bromo-5-fluoropyrimidine (500 mg, 2.83 mmol), 1,1,1,2,2,2-hexabutyldistannane (2.86 mL, 5.65 mmol), tetrakis(triphenylphosphine)palladium(0) (326 mg, 0.283 mmol) toluene (12 mL). The mixture was degassed (brief high vacuum, then refilled with Ar) and then heated at 110 ⁰C for 16 h. The reaction mixture was diluted with EtOAc and washed with water followed by brine. The organic phase was concentrated in vacuo. The residue was adsorbed onto Celite and then was purified by silica gel chromatography eluting with 0-15% EtOAc in hexane to afford the title compound 5-fluoro-2-(tributylstannyl)pyrimidine (550 mg, 50% yield) as a clear viscous oil contaminated with triphenylphosphine. The material was used in subsequent reactions without further purification. LCMS m/z = 389.0 (M+H).

### Preparation of 5-ethoxy-2-(tributylstannyl)pyrimidine

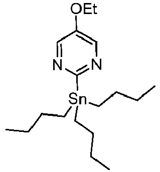

To a sealed tube was added 2-bromo-5-ethoxypyrimidine (250 mg, 1.231 mmol),1,1,1,2,2,2-hexabutyldistannane (1.244 mL, 2.463 mmol), tetrakis(triphenylphosphine)palladium(0) (142 mg, 0.123 mmol and toluene (8 mL). The mixture was degassed (brief high vacuum, then refilled with Ar) and then heated at 110 ⁰C for 16 h. The reaction mixture was diluted with EtOAc and washed with water and followed by brine. The organic phase was concentrated in vacuo. The residue was adsorbed onto Celite and then was purified by silica gel chromatography eluting with 0-15% EtOAc in hexane to afford the title compound 5-ethoxy-2-(tributylstannyl)pyrimidine (300 mg, 59% yield) as a clear viscous oil contaminated with triphenylphosphine. The material was used in subsequent reaction without further purification. LCMS m/z = 415.05 (M+H).

### Preparation of 5-ethyl-2-(tributylstannyl)pyrimidine

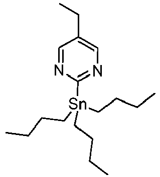

To a sealed tube was added 2-chloro-5-ethylpyrimidine (500 mg, 3.51 mmol),1,1,1,2,2,2-hexabutyldistannane (3.54 mL, 7.01 mmol), tetrakis(triphenylphosphine)palladium(0) (405 mg, 0.351 mmol) and toluene (12 mL). The mixture was degassed (brief high vacuum, then refilled with Ar) and then heated at 110 ⁰C for 16 h. The reaction mixture was diluted with EtOAc and washed with water followed by brine. The organic phase was concentrated in vacuo. The residue was adsorbed onto Celite and then was purified by silica gel chromatography eluting with 0-15% EtOAc in hexane to afford the title compound 5-ethyl-2-(tributylstannyl)pyrimidine (700 mg, 50% yield) as a clear viscous oil contaminated with triphenylphosphine. The material was used in subsequent reaction without further purification. LCMS m/z = 399.0 (M+H).

### General Procedure A:

To a mixture of (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (20 mg, 0.020 mmol), the indicated stannane (0.029 mmol) and copper(I) iodide (0.372 mg, 1.954 µmol) in N,N-Dimethylformamide (DMF) (1 mL) was added tetrakis(triphenylphosphine)palladium(0) (2.259 mg, 1.954 µmol). The mixture was degassed (brief high vacuum, then refilled with Ar) and then heated at 100 °C for 6 h. The mixture was cooled to room temperature; diluted with ethyl acetate; washed with water; dried (Na₂SO₄); and filtered. The filtrate was concentrated under reduced pressure and the resulting residue was taken up in DCM (1 mL). To the solution was added triflic acid (0.05 mL) and TFA (1 mL). The solution was stirred at rt for 1 h and then concentrated under reduced pressure. The residue was dissolved in DMF and then subjected to HPLC purification to afford the indicated product.

### General procedure B:

To a mixture of (1P)-1-(4-chloro-3-(cyclopropanesulfonamido)-1-(2,2-difluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (20 mg, 0.020 mmol), the indicated stannane (0.029 mmol), and copper(I) iodide (0.372 mg, 1.954 µmol) in DMF (1 mL) was added tetrakis(triphenylphosphine)palladium(0) (2.259 mg, 1.954 µmol). The mixture was degassed (brief high vacuum, then refilled with Ar) and then heated at 100 °C for 4 h. The mixture was cooled to room temperature and filtered, and the filtrate was subjected to HPLC purification to afford the indicated product.

### General Procedure C:

General Procedure B was followed substituting (1P)-1-(4-chloro-1-(2,2-difluoroethyl)-3-(methylsulfonamido)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate as the triflate coupling partner.

### General Procedure D:

General Procedure B was followed substituting (1P)-1-(4-chloro-3-(methylsulfonamido)-1-(2,2,2-trifluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate as the triflate coupling partner.

### Preparation of Example 9: N-((S)-1-((1P)-1-(4-chloro-1-methyl-3-(methylsulfonamido)-1H-indazol-7-yl)-4-(3-methoxy-3-methylbut-1-yn-1-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

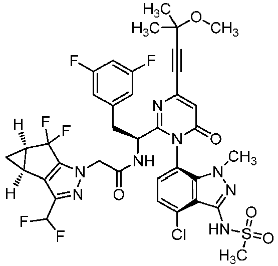

A vial was charged with (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (50 mg, 0.049 mmol), 3-methoxy-3-methylbut-1-yne (23.98 mg, 0.244 mmol), copper(I) iodide (0.931 mg, 4.89 µmol), N,N-Dimethylformamide (DMF) (1.5 mL) and triethylamine (0.020 mL, 0.147 mmol). To the vial was added bis(triphenylphosphine)palladium(II) chloride (3.43 mg, 4.89 µmol). The mixture was stirred at room temperature for 1 h upon which LCMS analysis indicated completion of the reaction. The was diluted with ethyl acetate, washed with water, dried (Na₂SO₄), and filtered. The filtrate was concentrated under reduced pressure. The residue was taken up in TFA (1.5 mL) and the resulting solution was stirred at room temp for 16 h. The solution was concentrated under reduced pressure. The resulting residue was dissolved in methanol (2 mL) and half of the volume was used to prepare Example 10. The remaining solution was subjected to HPLC purification to afford the title compound, N-((S)-1-((1P)-1-(4-chloro-1-methyl-3-(methylsulfonamido)-1H-indazol-7-yl)-4-(3-methoxy-3-methylbut-1-yn-1-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.45 min.; observed ion = 851.4 (M+H). 1H NMR (500 MHz, METHANOL-d4) δ ppm 7.30 (d, J=8.05 Hz, 1 H), 7.20 (d, J=8.05 Hz, 1 H), 6.53 - 6.85 (m, 5 H), 4.74 (dd, J=9.84, 4.77 Hz, 1 H), 4.49 - 4.60 (m, 2 H), 3.65 (s, 3 H), 3.47 (s, 3 H), 3.38 (dd, J=14.01, 4.77 Hz, 1 H), 3.24 (s, 3 H), 3.05 (dd, J=13.86, 9.69 Hz, 1 H), 2.45 (ddd, J=11.25, 7.53, 3.87 Hz, 2 H), 1.59 (s, 6 H), 1.34 - 1.43 (m, 1 H), 0.97 - 1.04 (m, 1 H).

### Preparation of Example 10: N-((S)-1-((1P)-1-(4-chloro-1-methyl-3-(methylsulfonamido)-1H-indazol-7-yl)-4-(3-methoxy-3-methylbutyl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

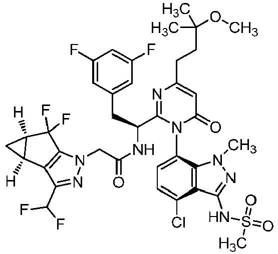

One half of the volume of the crude reaction solution produced in the preparation of Example 9 as described above (1 mL) was treated with 10% palladium on carbon (11.15 mg, 10.48 µmol). The mixture was and stirred under balloon-pressure hydrogen atmosphere for 1 h. The mixture was filtered and the filtrate was subjected to HPLC purification to afford the title compound, N-((S)-1-((1P)-1-(4-chloro-1-methyl-3-(methylsulfonamido)-1H-indazol-7-yl)-4-(3-methoxy-3-methylbutyl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.45 min.; observed ion = 855.2 (M+H). 1H NMR (500 MHz, METHANOL-d4) δ ppm 7.27 (d, J=7.75 Hz, 1 H), 7.10 (d, J=7.75 Hz, 1 H), 6.58 - 6.85 (m, 4 H), 6.50 (s, 1 H), 4.76 (dd, J=9.09, 5.22 Hz, 1 H), 4.54 (s, 2 H), 3.62 (s, 3 H), 3.37 - 3.42 (m, 1 H), 3.29 (s, 3 H), 3.23 (s, 3 H), 3.03 (dd, J=14.01, 9.24 Hz, 1 H), 2.71 - 2.78 (m, 2 H), 2.43 - 2.51 (m, 2 H), 1.94 - 2.01 (m, 2 H), 1.37 - 1.43 (m, 1 H), 1.30 (s, 6 H), 1.00 - 1.06 (m, 1 H).

### Preparation of Example 11: N-((S)-1-((1P)-1-(4-chloro-1-methyl-3-(methylsulfonamido)-1H-indazol-7-yl)-2'-ethoxy-6-oxo-1,6-dihydro-[4,5'-bipyrimidin]-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

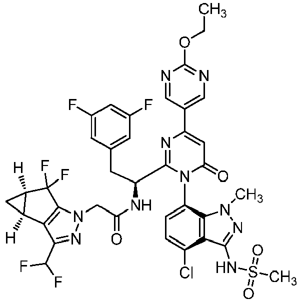

The title compound was prepared according to General Procedure A using 2-ethoxy-5-(tributylstannyl)pyrimidine as the coupling partner. The experiment afforded the title compound, N-((S)-1-((1P)-1-(4-chloro-1-methyl-3-(methylsulfonamido)-1H-indazol-7-yl)-2'-ethoxy-6-oxo-1,6-dihydro-[4,5'-bipyrimidin]-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.37 min.; observed ion = 877.4 (M+H). 1H NMR (500 MHz, METHANOL-d4) δ ppm 9.37 (s, 2 H), 7.31 (d, J=8.05 Hz, 1 H), 7.21 (d, J=7.75 Hz, 1 H), 7.17 (s, 1 H), 6.55 - 6.83 (m, 4 H), 4.75 - 4.79 (m, 1 H), 4.50 - 4.63 (m, 4 H), 3.68 (s, 3 H), 3.42 - 3.49 (m, 1 H), 3.23 (s, 3 H), 3.05 - 3.13 (m, 1 H), 2.34 - 2.47 (m, 2 H), 1.47 (t, J=7.00 Hz, 3 H), 1.33 - 1.37 (m, 1 H), 0.92 - 1.00 (m, 1 H).

### Preparation of Example 12: N-((S)-1-((1'P)-1'-(4-chloro-1-methyl-3-(methylsulfonamido)-1H-indazol-7-yl)-5-ethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

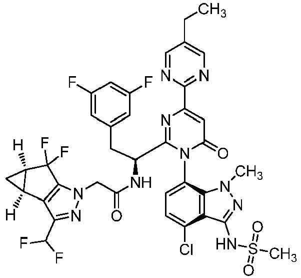

The title compound was prepared according to General Procedure A using 5-ethyl-2-(tributylstannyl)pyrimidine as the coupling partner. The experiment afforded the title compound, N-((S)-1-((1'P)-1'-(4-chloro-1-methyl-3-(methylsulfonamido)-1H-indazol-7-yl)-5-ethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.37 min.; observed ion = 861.4 (M+H). 1H NMR (500 MHz, METHANOL-d4) δ ppm 8.96 (s, 2 H), 7.70 (s, 1 H), 7.33 (d, J=8.05 Hz, 1 H), 7.20 (d, J=8.05 Hz, 1 H), 6.46 - 6.84 (m, 4 H), 4.81 - 4.85 (m, 1 H), 4.60 - 4.72 (m, 2 H), 3.62 (s, 3 H), 3.36 - 3.40 (m, 1 H), 3.23 (s, 3 H), 3.07 - 3.12 (m, 1 H), 2.86 (q, J=7.75 Hz, 2 H), 2.35 - 2.43 (m, 2 H), 1.38 (t, J=7.60 Hz, 3 H), 1.32 - 1.36 (m, 1 H), 0.95 - 1.01 (m, 1 H).

### Preparation of Example 13: N-((S)-1-((1P)-1-(4-chloro-1-methyl-3-(methylsulfonamido)-1H-indazol-7-yl)-4-(5-methoxypyrazin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

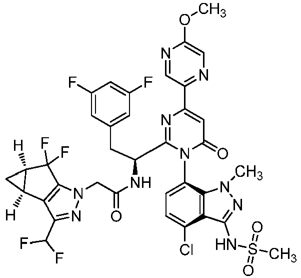

The title compound was prepared according to General Procedure A using 2-methoxy-5-(tributylstannyl)pyrazine as the coupling partner. The experiment afforded the title compound, N-((S)-1-((1P)-1-(4-chloro-1-methyl-3-(methylsulfonamido)-1H-indazol-7-yl)-4-(5-methoxypyrazin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.4 min.; observed ion = 863.4 (M+H). 1H NMR (500 MHz, METHANOL-d4) δ ppm 9.28 (d, J=1.19 Hz, 1 H), 8.37 (d, J=1.49 Hz, 1 H), 7.40 (s, 1 H), 7.29 (d, J=7.75 Hz, 1 H), 7.20 (d, J=7.75 Hz, 1 H), 6.55 - 6.84 (m, 4 H), 4.82 - 4.84 (m, 1 H), 4.50 - 4.57 (m, 2 H), 4.10 (s, 3 H), 3.67 (s, 3 H), 3.47 (dd, J=14.31, 4.77 Hz, 1 H), 3.23 (s, 3 H), 3.12 (dd, J=14.16, 9.69 Hz, 1 H), 2.34 - 2.46 (m, 2 H), 1.32 - 1.39 (m, 1 H), 0.94 - 1.01 (m, 1 H).

### Preparation of Example 14: N-((S)-1-((1'P)-1'-(4-chloro-1-methyl-3-(methylsulfonamido)-1H-indazol-7-yl)-5-ethoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

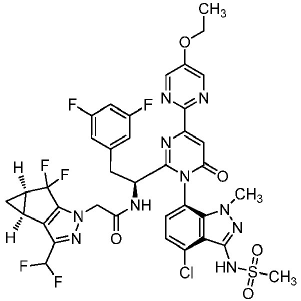

The title compound was prepared according to General Procedure A using 5-ethoxy-2-(tributylstannyl)pyrimidine as the coupling partner. The experiment afforded the title compound, N-((S)-1-((1'P)-1'-(4-chloro-1-methyl-3-(methylsulfonamido)-1H-indazol-7-yl)-5-ethoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.37 min.; observed ion = 877.4 (M+H). 1H NMR (500 MHz, METHANOL-d4) δ ppm 8.74 (s, 2 H), 7.61 (s, 1 H), 7.31 (d, J=7.75 Hz, 1 H), 7.18 (d, J=7.75 Hz, 1 H), 6.49 - 6.82 (m, 4 H), 4.82 - 4.84 (m, 1 H), 4.61 - 4.70 (m, 2 H), 4.37 (q, J=6.85 Hz, 2 H), 3.61 (s, 3 H), 3.35 - 3.40 (m, 1 H), 3.22 (s, 3 H), 3.09 (dd, J=14.16, 9.39 Hz, 1 H), 2.33 - 2.45 (m, 2 H), 1.52 (t, J=7.00 Hz, 3 H), 1.32 - 1.39 (m, 1 H), 0.98 (dtd, J=5.74, 3.91, 3.91, 2.24 Hz, 1 H).

### Preparation of Example 15: N-((S)-1-((1P)-1-(4-chloro-1-methyl-3-(methylsulfonamido)-1H-indazol-7-yl)-4-(2-(1,1-difluoroethyl)thiazol-5-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

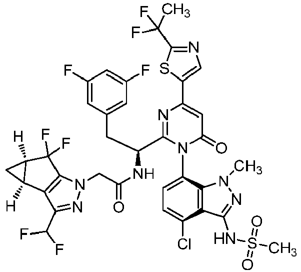

The title compound was prepared according to General Procedure A using 2-(1,1-difluoroethyl)-5-(tributylstannyl)thiazole as the coupling partner. The experiment afforded the title compound, N-((S)-1-((1P)-1-(4-chloro-1-methyl-3-(methylsulfonamido)-1H-indazol-7-yl)-4-(2-(1,1-difluoroethyl)thiazol-5-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.46 min.; observed ion = 902.3 (M+H). 1H NMR (500 MHz, METHANOL-d4) δ ppm 8.66 (t, J=1.64 Hz, 1 H), 8.16 (t, J=1.49 Hz, 1 H), 7.27 - 7.31 (m, 1 H), 7.20 - 7.24 (m, 1 H), 7.15 (s, 1 H), 6.54 - 6.82 (m, 4 H), 4.79 (dd, J=9.54, 4.47 Hz, 1 H), 4.50 (d, J=1.79 Hz, 2 H), 3.69 (s, 3 H), 3.44 (dd, J=14.16, 4.62 Hz, 1 H), 3.23 (s, 3 H), 3.08 (dd, J=14.45, 9.69 Hz, 1 H), 2.36 - 2.44 (m, 2 H), 2.14 - 2.21 (m, 3 H), 1.32 - 1.38 (m, 1 H), 0.95 - 1.00 (m, 1 H).

### Preparation of Example 16: N-((S)-1-((1P)-1-(4-chloro-1-methyl-3-(methylsulfonamido)-1H-indazol-7-yl)-4-(4-methylpyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

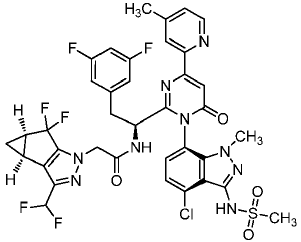

The title compound was prepared according to General Procedure A using 4-methyl-2-(tributylstannyl)pyridine as the coupling partner. The experiment afforded the title compound, N-((S)-1-((1P)-1-(4-chloro-1-methyl-3-(methylsulfonamido)-1H-indazol-7-yl)-4-(4-methylpyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.37 min.; observed ion = 846.4 (M+H). 1H NMR (500 MHz, METHANOL-d4) δ ppm 8.60 (d, J=5.07 Hz, 1 H), 8.41 (s, 1 H), 7.50 (s, 1 H), 7.42 (dd, J=4.77, 0.89 Hz, 1 H), 7.30 (d, J=8.05 Hz, 1 H), 7.19 (d, J=7.75 Hz, 1 H), 6.53 - 6.84 (m, 4 H), 4.83 - 4.84 (m, 1 H), 4.51 - 4.58 (m, 2 H), 3.66 (s, 3 H), 3.46 - 3.51 (m, 1 H), 3.23 (s, 3 H), 3.10 - 3.16 (m, 1 H), 2.54 (s, 3 H), 2.35 - 2.43 (m, 2 H), 1.32 - 1.37 (m, 1 H), 0.94 - 1.00 (m, 1 H).

### Preparation of Example 17: N-((S)-1-((1P)-1-(4-chloro-1-methyl-3-(methylsulfonamido)-1H-indazol-7-yl)-4-(2-(methylsulfonyl)thiazol-4-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

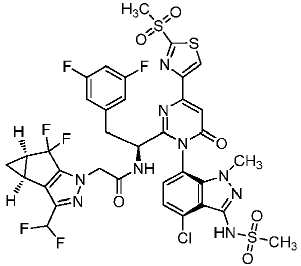

The title compound was prepared according to General Procedure A using 2-(methylsulfonyl)-4-(tributylstannyl)thiazole as the coupling partner. The experiment afforded the title compound, N-((S)-1-((1P)-1-(4-chloro-1-methyl-3-(methylsulfonamido)-1H-indazol-7-yl)-4-(2-(methylsulfonyl)thiazol-4-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.33 min.; observed ion = 916.3 (M+H). 1H NMR (500 MHz, METHANOL-d4) δ ppm 8.92 (s, 1 H), 7.32 (s, 1 H), 7.28 (d, J=7.75 Hz, 1 H), 7.18 (d, J=7.75 Hz, 1 H), 6.57 - 6.82 (m, 4 H), 4.44 - 4.55 (m, 2 H), 3.67 (s, 3 H), 3.43 - 3.52 (m, 4 H), 3.22 (s, 3 H), 3.13 (dd, J=14.31, 9.54 Hz, 1 H), 2.41 (ddd, J=11.25, 7.67, 4.02 Hz, 2 H), 1.31 - 1.40 (m, 1 H), 0.95 - 1.00 (m, 1 H).

### Preparation of Example 18: N-((S)-1-((1P)-1-(4-chloro-1-methyl-3-(methylsulfonamido)-1H-indazol-7-yl)-4-(2-(2-hydroxypropan-2-yl)thiazol-4-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

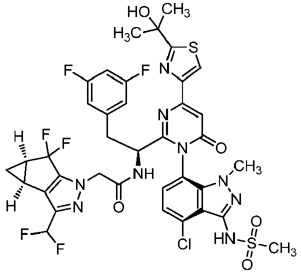

The title compound was prepared according to General Procedure A using 2-(4-(tributylstannyl)thiazol-2-yl)propan-2-ol as the coupling partner. The experiment afforded the title compound, N-((S)-1-((1P)-1-(4-chloro-1-methyl-3-(methylsulfonamido)-1H-indazol-7-yl)-4-(2-(2-hydroxypropan-2-yl)thiazol-4-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.34 min.; observed ion = 896.4 (M+H). 1H NMR (500 MHz, METHANOL-d4) δ ppm 8.44 (s, 1 H), 7.27 (d, J=7.75 Hz, 1 H), 7.21 (s, 1 H), 7.13 (d, J=8.05 Hz, 1 H), 6.53 - 6.83 (m, 4 H), 4.82 - 4.85 (m, 1 H), 4.46 - 4.56 (m, 2 H), 3.66 (s, 3 H), 3.44 - 3.49 (m, 1 H), 3.22 (s, 3 H), 3.10 (dd, J=14.16, 9.09 Hz, 1 H), 2.37 - 2.45 (m, 2 H), 1.69 (s, 6 H), 1.32 - 1.38 (m, 1 H), 0.95 - 1.03 (m, 1 H).

### Preparation of Example 19: N-((S)-1-((1P)-1-(4-chloro-1-methyl-3-(methylsulfonamido)-1H-indazol-7-yl)-4-(1-methyl-3-(trifluoromethyl)-1H-pyrazol-5-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

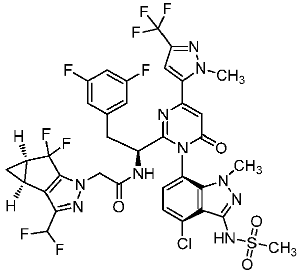

The title compound was prepared according to General Procedure A using 1-methyl-5-(tributylstannyl)-3-(trifluoromethyl)-1H-pyrazole as the coupling partner. The experiment afforded the title compound, N-((S)-1-((1P)-1-(4-chloro-1-methyl-3-(methylsulfonamido)-1H-indazol-7-yl)-4-(1-methyl-3-(trifluoromethyl)-1H-pyrazol-5-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.46 min.; observed ion = 903.4 (M+H). 1H NMR (500 MHz, METHANOL-d4) δ ppm 7.29 - 7.35 (m, 3 H), 7.06 (s, 1 H), 6.55 - 6.85 (m, 4 H), 4.79 (dd, J=10.28, 4.02 Hz, 1 H), 4.45 - 4.54 (m, 2 H), 4.39 (s, 3 H), 3.72 (s, 3 H), 3.45 - 3.51 (m, 1 H), 3.26 (s, 3 H), 3.12 - 3.17 (m, 1 H), 2.39 (s, 2 H), 1.35 - 1.41 (m, 1 H), 0.96 - 1.03 (m, 1 H).

### Preparation of Example 20: N-((S)-1-((1P)-1-(4-chloro-1-methyl-3-(methylsulfonamido)-1H-indazol-7-yl)-6-oxo-4-(pyrazin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

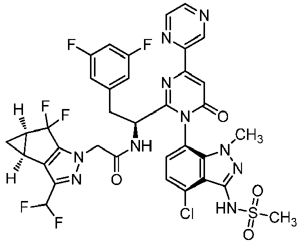

The title compound was prepared according to General Procedure A using 2-(tributylstannyl)pyrazine as the coupling partner. The experiment afforded the title compound, N-((S)-1-((1P)-1-(4-chloro-1-methyl-3-(methylsulfonamido)-1H-indazol-7-yl)-6-oxo-4-(pyrazin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.3 min.; observed ion = 833.4 (M+H). 1H NMR (500 MHz, METHANOL-d4) δ ppm 9.73 (d, J=1.49 Hz, 1 H), 8.79 - 8.87 (m, 2 H), 7.61 (s, 1 H), 7.31 - 7.36 (m, 1 H), 7.24 - 7.29 (m, 1 H), 6.53 - 6.86 (m, 4 H), 4.83 - 4.86 (m, 1 H), 4.53 - 4.61 (m, 2 H), 3.69 (s, 3 H), 3.51 (dd, J=14.31, 4.47 Hz, 1 H), 3.26 (s, 3 H), 3.14 - 3.19 (m, 1 H), 2.35 - 2.47 (m, 2 H), 1.33 - 1.39 (m, 1 H), 0.98 (dtd, J=5.81, 3.87, 3.87, 2.24 Hz, 1 H).

### Preparation of Example 21: N-((S)-1-((1P)-1-(4-chloro-1-methyl-3-(methylsulfonamido)-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

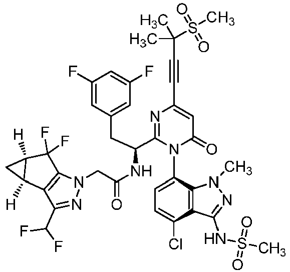

To a solution of 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (10.52 mg, 0.040 mmol) and (S)-N-((6P)-7-(2-(1-amino-2-(3,5-difluorophenyl)ethyl)-4-(3-methyl-3-(methylsulfonyl)but-1-yn-1-yl)-6-oxopyrimidin-1(6H)-yl)-4-chloro-1-methyl-1H-indazol-3-yl)methanesulfonamide (26 mg, 0.040 mmol) in N,N-Dimethylformamide (DMF) (2 mL) was added DIEA (0.021 mL, 0.119 mmol) followed by HATU (16.65 mg, 0.044 mmol). The solution was stirred at room temperature for 3 h. To the solution was added ammonia in methanol (2M, 0.5 mL) and the mixture was then stirred for 30 min. The mixture was diluted with water and then extracted with ethyl acetate; washed with brine, dried (Na2SO4), and filtered. The filtrate was concentrated under reduced pressure. The residue was then purified by silica gel chromatography eluting with 5-50% EtOAc in hexane to afford 28 mg of desired product as mixture of diastereomers (atropisomers, approximately a 60:40 ration as determined by analytical SFC). The material was then further purified by SFC using the following method: Column = Chiralpak IA, 10 x 250 mm, 5 µm; Mobile Phase = 60% MeOH in CO₂; Back pressure = 150 bar; Temperature = 40 °C; Flow rate = 3.5 mL/min. The first eluting diastereomer was collected to afford the title compound, N-((S)-1-((1P)-1-(4-chloro-1-methyl-3-(methylsulfonamido)-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 2.86 min.; observed ion = 899.1 (M+H). 1H NMR (500 MHz, METHANOL-d4) δ ppm 7.18 (d, J=8.05 Hz, 1 H), 7.07 (d, J=7.75 Hz, 1 H), 6.72 (s, 1 H), 6.65 - 6.71 (m, 1 H), 6.43 - 6.60 (m, 3 H), 4.62 (dd, J=9.83, 4.47 Hz, 1 H), 4.36 - 4.48 (m, 2 H), 3.53 (s, 3 H), 3.24 - 3.28 (m, 1 H), 3.11 (s, 3 H), 3.09 (s, 3 H), 2.93 (dd, J=14.01, 9.84 Hz, 1 H), 2.29 - 2.39 (m, 2 H), 1.67 (s, 6 H), 1.24 - 1.30 (m, 1 H), 0.86 - 0.92 (m, 1 H).

### Preparation of Example 22: N-((S)-1-((1P)-1-(4-chloro-1-methyl-3-(methylsulfonamido)-1H-indazol-7-yl)-4-(6-ethoxypyridin-3-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

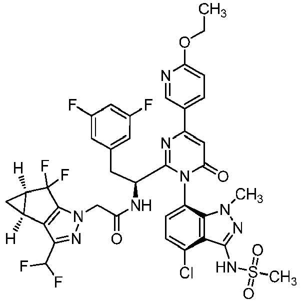

The title compound was prepared according to General Procedure A using 2-ethoxy-5-(tributylstannyl)pyridine as the coupling partner. The experiment afforded the title compound, N-((S)-1-((1P)-1-(4-chloro-1-methyl-3-(methylsulfonamido)-1H-indazol-7-yl)-4-(6-ethoxypyridin-3-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.46 min.; observed ion = 876.4 (M+H). 1H NMR (500 MHz, METHANOL-d4) δ ppm 9.06 (dd, J=2.38, 0.60 Hz, 1 H), 8.40 - 8.47 (m, 1 H), 7.27 (d, J=7.75 Hz, 1 H), 7.16 (d, J=7.75 Hz, 1 H), 7.05 (s, 1 H), 6.93 (dd, J=8.79, 0.75 Hz, 1 H), 6.55 - 6.83 (m, 4 H), 4.81 - 4.83 (m, 1 H), 4.53 (d, J=7.45 Hz, 1 H), 4.57 (s, 1 H), 4.46 (d, J=7.15 Hz, 2 H), 3.67 (s, 3 H), 3.46 (dd, J=14.45, 4.92 Hz, 1 H), 3.22 (s, 3 H), 3.11 (dd, J=14.01, 9.54 Hz, 1 H), 2.36 - 2.46 (m, 2 H), 1.42 - 1.46 (m, 2 H), 1.31 - 1.37 (m, 1 H), 0.95 - 1.01 (m, 1 H).

### Preparation of Example 23: N-((S)-1-((1P)-1-(4-chloro-1-methyl-3-(methylsulfonamido)-1H-indazol-7-yl)-4-(6-fluoropyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

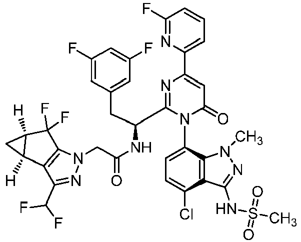

The title compound was prepared according to General Procedure A using 2-fluoro-6-(tributylstannyl)pyridine as the coupling partner. The experiment afforded the title compound, N-((S)-1-((1P)-1-(4-chloro-1-methyl-3-(methylsulfonamido)-1H-indazol-7-yl)-4-(6-fluoropyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.4 min.; observed ion = 850.4 (M+H). 1H NMR (500 MHz, METHANOL-d4) δ ppm 8.48 (dd, J=7.60, 1.94 Hz, 1 H), 8.17 (q, J=7.85 Hz, 1 H), 7.47 (s, 1 H), 7.30 (d, J=8.05 Hz, 1 H), 7.27 (dd, J=8.34, 2.09 Hz, 1 H), 7.21 (d, J=7.75 Hz, 1 H), 6.55 - 6.82 (m, 4 H), 4.81 - 4.85 (m, 1 H), 4.47 - 4.57 (m, 2 H), 3.67 (s, 3 H), 3.48 (dd, J=14.01, 4.77 Hz, 1 H), 3.24 (s, 3 H), 3.14 (dd, J=14.01, 9.54 Hz, 1 H), 2.34 - 2.43 (m, 2 H), 1.32 - 1.37 (m, 1 H), 0.94 - 1.03 (m, 1 H).

### Preparation of Example 24: N-((S)-1-((1P)-1-(4-chloro-1-methyl-3-(methylsulfonamido)-1H-indazol-7-yl)-4-(5-chloropyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

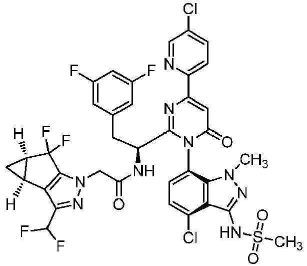

The title compound was prepared according to General Procedure A using 5-chloro-2-(tributylstannyl)pyridine as the coupling partner. The experiment afforded the title compound, N-((S)-1-((1P)-1-(4-chloro-1-methyl-3-(methylsulfonamido)-1H-indazol-7-yl)-4-(5-chloropyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.46 min.; observed ion = 866.4 (M+H). 1H NMR (500 MHz, METHANOL-d4) δ ppm 8.60 (d, J=5.07 Hz, 1 H), 8.41 (s, 1 H), 7.50 (s, 1 H), 7.42 (dd, J=4.77, 0.89 Hz, 1 H), 7.30 (d, J=8.05 Hz, 1 H), 7.19 (d, J=7.75 Hz, 1 H), 6.53 - 6.84 (m, 4 H), 4.83 - 4.84 (m, 1 H), 4.51 - 4.58 (m, 2 H), 3.66 (s, 3 H), 3.46 - 3.51 (m, 1 H), 3.23 (s, 3 H), 3.10 - 3.16 (m, 1 H), 2.54 (s, 3 H), 2.35 - 2.43 (m, 2 H), 1.32 - 1.37 (m, 1 H), 0.94 - 1.00 (m, 1 H).

### Preparation of Example 25: N-((S)-1-((1P)-1-(4-chloro-1-methyl-3-(methylsulfonamido)-1H-indazol-7-yl)-4-(2-(2-hydroxypropan-2-yl)thiazol-5-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

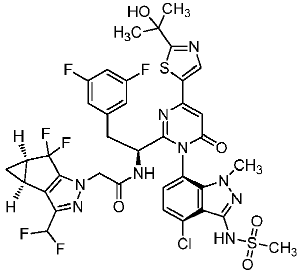

The title compound was prepared according to General Procedure A using 2-(5-(tributylstannyl)thiazol-2-yl)propan-2-ol as the coupling partner. The experiment afforded the title compound, N-((S)-1-((1P)-1-(4-chloro-1-methyl-3-(methylsulfonamido)-1H-indazol-7-yl)-4-(2-(2-hydroxypropan-2-yl)thiazol-5-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.3 min.; observed ion = 896.4 (M+H). 1H NMR (500 MHz, METHANOL-d4) δ ppm 8.50 (s, 1 H), 7.27 (d, J=7.75 Hz, 1 H), 7.16 (d, J=8.05 Hz, 1 H), 6.97 (s, 1 H), 6.53 - 6.82 (m, 4 H), 4.78 - 4.83 (m, 1 H), 4.49 (s, 2 H), 3.69 (s, 3 H), 3.45 (dd, J=14.01, 5.07 Hz, 1 H), 3.22 (s, 3 H), 3.08 (dd, J=14.01, 9.24 Hz, 1 H), 2.35 - 2.46 (m, 2 H), 1.66 (d, J=2.38 Hz, 6 H), 1.33 - 1.38 (m, 1 H), 0.94 - 1.02 (m, 1 H).

### Preparation of Example 26: N-((S)-1-((1'P)-1'-(4-chloro-3-(cyclopropanesulfonamido)-1-(2,2-difluoroethyl)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide.

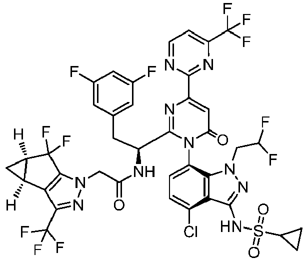

The title compound was prepared according to General Procedure A using 2-(tributylstannyl)-4-(trifluoromethyl)pyrimidine as the coupling partner modified as follows: (1P)-1-(4-chloro-1-(2,2-difluoroethyl)-3-(N-(4-methoxybenzyl)cyclopropanesulfonamido)-1H-indazol-7-yl)-2-((S)-1-(2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate was used instead of (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate. The experiment afforded the title compound, N-((S)-1-((1'P)-1'-(4-chloro-3-(cyclopropanesulfonamido)-1-(2,2-difluoroethyl)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide. The sample was analyzed using LCMS Method A: retention time = 1.49 min.; observed ion = 955.2 (M+H). 1H NMR (500 MHz, METHANOL-d4) δ ppm 9.41 (d, J=5.07 Hz, 1 H), 8.08 (d, J=5.07 Hz, 1 H), 7.75 (s, 1 H), 7.41 (d, J=7.75 Hz, 1 H), 7.28 (d, J=8.05 Hz, 1 H), 6.79 (tt, J=9.24, 2.38 Hz, 1 H), 6.48 - 6.58 (m, 2 H), 5.92 - 6.17 (m, 1 H), 4.65 - 4.78 (m, 3 H), 4.28 - 4.42 (m, 1 H), 3.90 - 4.06 (m, 1 H), 3.37 (dd, J=14.16, 4.92 Hz, 1 H), 3.09 (dd, J=14.31, 9.54 Hz, 1 H), 2.90 (tt, J=7.97, 4.69 Hz, 1 H), 2.42 (ddd, J=11.25, 7.53, 3.87 Hz, 2 H), 1.33 - 1.40 (m, 1 H), 1.07 - 1.10 (m, 2 H), 1.01 - 1.05 (m, 1 H), 0.95 - 1.01 (m, 2 H).

### Preparation of Example 27: N-((S)-1-((1'P)-1'-(4-chloro-3-(cyclopropanesulfonamido)-1-(2,2-difluoroethyl)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

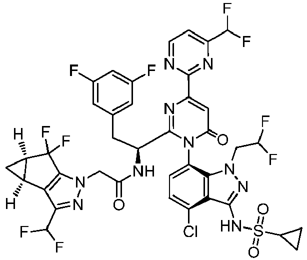

The title compound was prepared according to General Procedure B using 4-(difluoromethyl)-2-(tributylstannyl)pyrimidine as the coupling partner. The experiment afforded the title compound, N-((S)-1-((1'P)-1'-(4-chloro-3-(cyclopropanesulfonamido)-1-(2,2-difluoroethyl)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.4 min.; observed ion = 959.4 (M+H). 1H NMR (500 MHz, METHANOL-d4) δ ppm 9.31 (d, J=5.07 Hz, 1 H), 7.95 (d, J=5.07 Hz, 1 H), 7.77 (s, 1 H), 7.40 - 7.44 (m, 1 H), 7.29 (d, J=7.75 Hz, 1 H), 6.50 - 6.93 (m, 5 H), 5.93 - 6.22 (m, 1 H), 4.73 - 4.78 (m, 1 H), 4.59 - 4.70 (m, 2 H), 4.32 - 4.40 (m, 1 H), 3.94 - 4.04 (m, 1 H), 3.10 (dd, J=14.16, 9.39 Hz, 1 H), 2.85 - 2.92 (m, 1 H), 2.33 - 2.45 (m, 2 H), 1.61 - 1.67 (m, 1 H), 1.30 - 1.39 (m, 2 H), 1.17 - 1.21 (m, 1 H), 1.09 (s, 1 H), 0.96 - 1.00 (m, 2 H).

### Preparation of Example 28: N-((S)-1-((1P)-1-(4-chloro-3-(cyclopropanesulfonamido)-1-(2,2-difluoroethyl)-1H-indazol-7-yl)-4-(6-fluoropyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

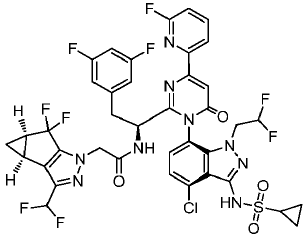

The title compound was prepared according to General Procedure B using 2-fluoro-6-(tributylstannyl)pyridine as the coupling partner. The experiment afforded the title compound, N-((S)-1-((1P)-1-(4-chloro-3-(cyclopropanesulfonamido)-1-(2,2-difluoroethyl)-1H-indazol-7-yl)-4-(6-fluoropyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.45 min.; observed ion = 926.3 (M+H). 1H NMR (500 MHz, METHANOL-d4) δ ppm 8.48 (dd, J=7.45, 1.79 Hz, 1 H), 8.17 (q, J=8.05 Hz, 1 H), 7.46 (s, 1 H), 7.38 (d, J=8.34 Hz, 1 H), 7.25 - 7.29 (m, 2 H), 6.55 - 6.84 (m, 4 H), 5.94 - 6.21 (m, 1 H), 4.73 (dd, J=9.54, 4.77 Hz, 1 H), 4.56 - 4.68 (m, 2 H), 4.32 - 4.45 (m, 1 H), 3.92 - 4.05 (m, 1 H), 3.41 (dd, J=14.31, 4.77 Hz, 1 H), 3.08 (dd, J=14.31, 9.54 Hz, 1 H), 2.86 - 2.93 (m, 1 H), 2.33 - 2.42 (m, 2 H), 1.29 - 1.36 (m, 1 H), 1.09 (dd, J=4.77, 2.38 Hz, 2 H), 0.94 - 0.99 (m, 3 H).

### Preparation of Example 29: N-((S)-1-((1'P)-1'-(4-chloro-3-(cyclopropanesulfonamido)-1-(2,2-difluoroethyl)-1H-indazol-7-yl)-5-ethoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

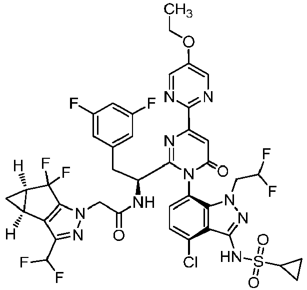

The title compound was prepared according to General Procedure B using 4-(dibutyl(5-ethoxypyrimidin-2-yl)stannyl)butan-1-ylium as the coupling partner. The experiment afforded the title compound, N-((S)-1-((1'P)-1'-(4-chloro-3-(cyclopropanesulfonamido)-1-(2,2-difluoroethyl)-1H-indazol-7-yl)-5-ethoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.43 min.; observed ion = 953.3 (M+H). 1H NMR (500 MHz, METHANOL-d4) δ ppm 8.74 (s, 2 H), 7.62 (s, 1 H), 7.39 (d, J=8.05 Hz, 1 H), 7.24 (d, J=7.75 Hz, 1 H), 6.45 - 6.81 (m, 4 H), 5.93 - 6.19 (m, 1 H), 4.72 - 4.76 (m, 1 H), 4.52 - 4.71 (m, 2 H), 4.37 (q, J=6.85 Hz, 2 H), 3.90 - 4.01 (m, 1 H), 3.06 (dd, J=14.01, 9.54 Hz, 1 H), 2.85 - 2.92 (m, 1 H), 2.29 - 2.52 (m, 2 H), 1.52 (t, J=7.00 Hz, 3 H), 1.32 - 1.38 (m, 1 H), 1.07 - 1.14 (m, 2 H), 0.89 - 1.04 (m, 3 H).

### Preparation of Example 30: N-((S)-1-((1'P)-1'-(4-chloro-3-(cyclopropanesulfonamido)-1-(2,2-difluoroethyl)-1H-indazol-7-yl)-5-fluoro-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

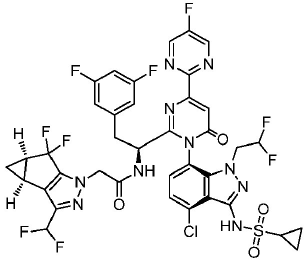

The title compound was prepared according to General Procedure B using 5-fluoro-2-(tributylstannyl)pyrimidine as the coupling partner. The experiment afforded the title compound, N-((S)-1-((1'P)-1'-(4-chloro-3-(cyclopropanesulfonamido)-1-(2,2-difluoroethyl)-1H-indazol-7-yl)-5-fluoro-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.38 min.; observed ion = 927.2 (M+H). 1H NMR (500 MHz, METHANOL-d4) δ ppm 9.06 (s, 2 H), 7.69 (s, 1 H), 7.37 - 7.44 (m, 1 H), 7.26 (br d, J=7.45 Hz, 1 H), 6.46 - 6.82 (m, 4 H), 5.91 - 6.20 (m, 1 H), 4.74 (dd, J=9.24, 4.77 Hz, 1 H), 4.57 - 4.71 (m, 2 H), 4.29 - 4.38 (m, 1 H), 3.90 - 4.03 (m, 1 H), 3.07 (dd, J=14.16, 9.69 Hz, 1 H), 2.84 - 2.92 (m, 1 H), 2.30 - 2.48 (m, 2 H), 1.32 - 1.38 (m, 1 H), 1.07 - 1.14 (m, 2 H), 0.87 - 1.02 (m, 3 H).

### Preparation of Example 31: N-((S)-1-((1P)-1-(4-chloro-3-(cyclopropanesulfonamido)-1-(2,2-difluoroethyl)-1H-indazol-7-yl)-4-(5-methoxypyrazin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

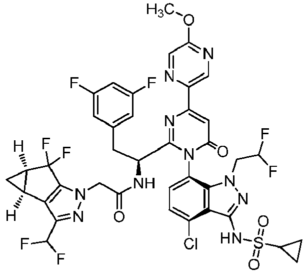

The title compound was prepared according to General Procedure B using 2-methoxy-5-(tributylstannyl)pyrazine as the coupling partner. The experiment afforded the title compound, N-((S)-1-((1P)-1-(4-chloro-3-(cyclopropanesulfonamido)-1-(2,2-difluoroethyl)-1H-indazol-7-yl)-4-(5-methoxypyrazin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.46 min.; observed ion = 939.2 (M+H). 1H NMR (500 MHz, METHANOL-d4) δ ppm 9.28 (d, J=1.19 Hz, 1 H), 8.38 (d, J=1.49 Hz, 1 H), 7.40 (s, 1 H), 7.37 (d, J=8.05 Hz, 1 H), 7.25 (d, J=7.75 Hz, 1 H), 6.54 - 6.84 (m, 4 H), 5.90 - 6.22 (m, 1 H), 4.70 (dd, J=8.94, 3.87 Hz, 1 H), 4.56 - 4.68 (m, 2 H), 4.34 - 4.44 (m, 1 H), 4.11 (s, 3 H), 3.93 - 4.04 (m, 1 H), 3.40 (dd, J=14.45, 4.62 Hz, 1 H), 3.08 (dd, J=14.01, 9.54 Hz, 1 H), 2.85 - 2.94 (m, 1 H), 2.32 - 2.43 (m, 2 H), 1.32 - 1.37 (m, 1 H), 1.06 - 1.11 (m, 2 H), 0.93 - 1.01 (m, 3 H).

### Preparation of Example 32: N-((S)-1-((1'P)-1'-(4-chloro-3-(cyclopropanesulfonamido)-1-(2,2-difluoroethyl)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide.

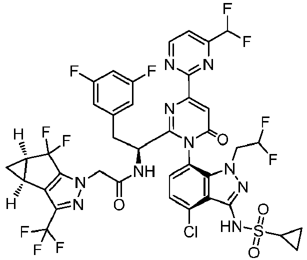

The title compound was prepared according to General Procedure A using 4-(difluoromethyl)-2-(tributylstannyl)pyrimidine as the coupling partner modified as follows: (1P)-1-(4-chloro-1-(2,2-difluoroethyl)-3-(N-(4-methoxybenzyl)cyclopropanesulfonamido)-1H-indazol-7-yl)-2-((S)-1-(2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate was used instead of (1P)-1-(4-chloro-3-(N-(4-methoxybenzyl)methylsulfonamido)-1-methyl-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate . The experiment afforded the title compound, N-((S)-1-((1'P)-1'-(4-chloro-3-(cyclopropanesulfonamido)-1-(2,2-difluoroethyl)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide. The sample was analyzed using LCMS Method A: retention time = 1.45 min.; observed ion = 977.2 (M+H). 1H NMR (500 MHz, METHANOL-d4) δ ppm 9.31 (d, J=5.36 Hz, 1 H), 7.94 (d, J=5.07 Hz, 1 H), 7.77 (s, 1 H), 7.41 (d, J=7.75 Hz, 1 H), 7.28 (d, J=8.05 Hz, 1 H), 6.74 - 6.93 (m, 2 H), 6.47 - 6.57 (m, 2 H), 5.90 - 6.20 (m, 1 H), 4.63 - 4.81 (m, 3 H), 4.29 - 4.40 (m, 1 H), 3.91 - 4.04 (m, 1 H), 3.34 - 3.38 (m, 1 H), 3.08 (dd, J=14.01, 9.54 Hz, 1 H), 2.90 (tt, J=8.01, 4.81 Hz, 1 H), 2.37 - 2.47 (m, 2 H), 1.34 - 1.41 (m, 1 H), 1.08 - 1.12 (m, 2 H), 1.01 - 1.05 (m, 1 H), 0.98 (dd, J=8.20, 1.34 Hz, 2 H).

### Preparation of Example 33: N-((S)-1-((1'P)-1'-(4-chloro-3-(cyclopropanesulfonamido)-1-(2,2-difluoroethyl)-1H-indazol-7-yl)-4-methoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

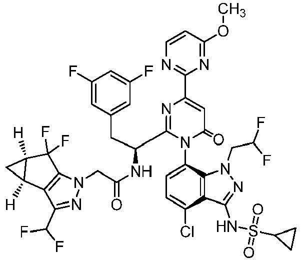

The title compound was prepared according to General Procedure B using 4-methoxy-2-(tributylstannyl)pyrimidine as the coupling partner. The experiment afforded the title compound, N-((S)-1-((1'P)-1'-(4-chloro-3-(cyclopropanesulfonamido)-1-(2,2-difluoroethyl)-1H-indazol-7-yl)-4-methoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.41 min.; observed ion = 939.2 (M+H). 1H NMR (500 MHz, METHANOL-d4) δ ppm 8.79 (d, J=5.66 Hz, 1 H), 7.72 (s, 1 H), 7.40 (br d, J=8.05 Hz, 1 H), 7.25 (d, J=8.05 Hz, 1 H), 7.04 - 7.10 (m, 1 H), 6.46 - 6.81 (m, 4 H), 5.92 - 6.21 (m, 1 H), 4.73 - 4.78 (m, 1 H), 4.58 - 4.72 (m, 2 H), 4.29 - 4.39 (m, 1 H), 4.17 (s, 3 H), 3.92 - 4.00 (m, 1 H), 3.06 (dd, J=13.41, 9.24 Hz, 1 H), 2.89 (ddd, J=8.20, 4.92, 2.98 Hz, 1 H), 2.35 - 2.48 (m, 2 H), 1.33 - 1.38 (m, 1 H), 1.11 - 1.15 (m, 1 H), 1.09 (dd, J=4.77, 1.19 Hz, 1 H), 1.02 - 1.07 (m, 1 H), 0.96 - 1.01 (m, 2 H).

### Preparation of Example 34: N-((S)-1-(1'-(4-chloro-3-(cyclopropanesulfonamido)-1-(2,2-difluoroethyl)-1H-indazol-7-yl)-4,6-dimethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

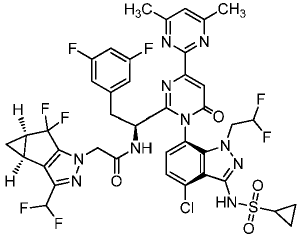

The title compound was prepared according to General Procedure B using 4,6-dimethyl-2-(tributylstannyl)pyrimidine as the coupling partner. The experiment afforded the title compound, N-((S)-1-(1'-(4-chloro-3-(cyclopropanesulfonamido)-1-(2,2-difluoroethyl)-1H-indazol-7-yl)-4,6-dimethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.42 min.; observed ion = 937.3 (M+H). 1H NMR (500 MHz, METHANOL-d4) δ ppm 7.72 (s, 1 H), 7.39 - 7.46 (m, 2 H), 7.20 (d, J=7.75 Hz, 1 H), 6.49 - 6.82 (m, 4 H), 5.91 - 6.15 (m, 1 H), 4.77 - 4.81 (m, 1 H), 4.63 - 4.72 (m, 2 H), 4.32 - 4.39 (m, 1 H), 3.93 - 4.00 (m, 1 H), 3.02 - 3.08 (m, 1 H), 2.86 - 2.92 (m, 1 H), 2.67 (s, 6 H), 2.37 - 2.46 (m, 2 H), 1.33 - 1.39 (m, 1 H), 1.12 - 1.14 (m, 1 H), 1.07 - 1.10 (m, 2 H), 0.92 - 1.01 (m, 3 H).

### Preparation of Example 35: N-((S)-1-((1P)-1-(4-chloro-3-(cyclopropanesulfonamido)-1-(2,2-difluoroethyl)-1H-indazol-7-yl)-4-(5-chloropyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

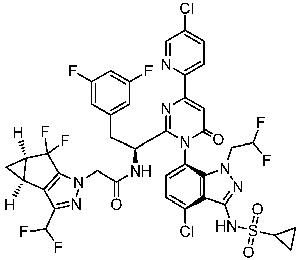

The title compound was prepared according to General Procedure B using 5-chloro-2-(tributylstannyl)pyridine as the coupling partner. The experiment afforded the title compound, N-((S)-1-((1P)-1-(4-chloro-3-(cyclopropanesulfonamido)-1-(2,2-difluoroethyl)-1H-indazol-7-yl)-4-(5-chloropyridin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.51 min.; observed ion = 942.2 (M+H). 1H NMR (500 MHz, METHANOL-d4) δ ppm 8.76 (d, J=2.38 Hz, 1 H), 8.63 (br s, 1 H), 8.55 (d, J=8.34 Hz, 1 H), 8.38 (d, J=8.94 Hz, 1 H), 7.54 (s, 1 H), 7.38 (d, J=7.75 Hz, 1 H), 7.25 (d, J=7.75 Hz, 1 H), 6.53 - 6.83 (m, 4 H), 5.95 - 6.21 (m, 1 H), 4.71 - 4.76 (m, 1 H), 4.56 - 4.68 (m, 2 H), 4.32 - 4.46 (m, 1 H), 3.95 - 4.05 (m, 1 H), 3.38 - 3.46 (m, 1 H), 3.04 - 3.12 (m, 1 H), 2.85 - 2.91 (m, 1 H), 2.35 - 2.42 (m, 2 H), 1.31 - 1.36 (m, 1 H), 1.09 (br dd, J=4.32, 2.53 Hz, 2 H), 0.98 (br d, J=4.17 Hz, 3 H).

### Preparation of Example 36: N-((S)-1-((1P)-1-(4-chloro-3-(cyclopropanesulfonamido)-1-(2,2-difluoroethyl)-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide.

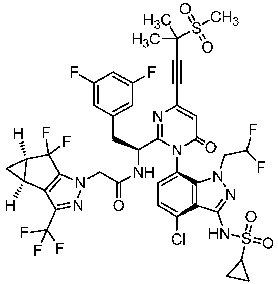

A vial was charged with (1P)-1-(4-chloro-1-(2,2-difluoroethyl)-3-(N-(4-methoxybenzyl)cyclopropanesulfonamido)-1H-indazol-7-yl)-2-((5)-1-(2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (40 mg, 0.036 mmol), 3-methyl-3-(methylsulfonyl)but-1-yne (15.70 mg, 0.107 mmol), copper(I) iodide (0.682 mg, 3.58 µmol), N,N-Dimethylformamide (DMF) (1.5 mL) and triethylamine (0.015 mL, 0.107 mmol). To the mixture was added bis(triphenylphosphine)palladium(II) chloride (2.51 mg, 3.58 µmol). The mixture was stirred 60 °C for 2 h upon which LCMS analysis indicated the reaction was complete. The mixture was cooled to room temperature and then was diluted with ethyl acetate; then washed with water; dried (Na2SO4); and filtered. The filtrate was concentrated under reduced pressure. The residue was taken up in DCM (1 mL) and to the solution was added triflic acid (0.05 mL) and TFA (1 mL). The mixture was stirred at rt for 1 h and then concentrated under reduced pressure. The residue was dissolved in DMF and then subjected to HPLC purification to afford the title compound, N-((S)-1-((1P)-1-(4-chloro-3-(cyclopropanesulfonamido)-1-(2,2-difluoroethyl)-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide. The sample was analyzed using LCMS Method A: retention time = 1.43 min.; observed ion = 993.2 (M+H). 1H NMR (500 MHz, METHANOL-d4) δ ppm 7.38 (d, J=8.05 Hz, 1 H), 7.26 (d, J=7.75 Hz, 1 H), 6.82 (s, 1 H), 6.76 - 6.81 (m, 1 H), 6.49 - 6.54 (m, 2 H), 5.90 - 6.19 (m, 1 H), 4.59 - 4.73 (m, 2 H), 4.56 (dd, J=10.13, 4.17 Hz, 1 H), 4.29 - 4.39 (m, 1 H), 3.86 - 3.98 (m, 1 H), 3.19 (s, 3 H), 3.00 (dd, J=14.31, 10.13 Hz, 1 H), 2.89 (tt, J=7.90, 4.77 Hz, 1 H), 2.41 - 2.54 (m, 2 H), 1.77 (d, J=1.19 Hz, 6 H), 1.36 - 1.43 (m, 1 H), 1.02 - 1.09 (m, 3 H), 0.94 - 1.00 (m, 2 H).

### Preparation of Example 37: N-((S)-1-((1P)-1-(4-chloro-3-(cyclopropanesulfonamido)-1-(2,2-difluoroethyl)-1H-indazol-7-yl)-4-(6-ethoxypyridin-3-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

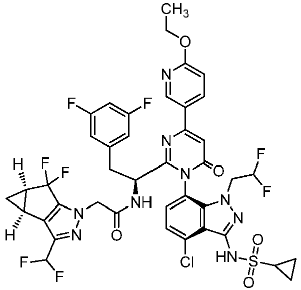

The title compound was prepared according to General Procedure B using 4-(dibutyl(6-ethoxypyridin-3-yl)stannyl)butan-1-ylium as the coupling partner. The experiment afforded the title compound, N-((S)-1-((1P)-1-(4-chloro-3-(cyclopropanesulfonamido)-1-(2,2-difluoroethyl)-1H-indazol-7-yl)-4-(6-ethoxypyridin-3-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.51 min.; observed ion = 952.4 (M+H). 1H NMR (500 MHz, METHANOL-d4) δ ppm 9.03 - 9.08 (m, 1 H), 8.41 - 8.46 (m, 1 H), 7.37 (d, J=8.05 Hz, 1 H), 7.22 (d, J=8.05 Hz, 1 H), 7.06 (s, 1 H), 6.91 - 6.95 (m, 1 H), 6.54 - 6.84 (m, 4 H), 5.97 - 6.22 (m, 1 H), 4.68 - 4.71 (m, 1 H), 4.57 - 4.65 (m, 2 H), 4.45 - 4.49 (m, 2 H), 4.34 - 4.41 (m, 1 H), 3.98 - 4.06 (m, 1 H), 3.40 (dd, J=14.01, 4.77 Hz, 1 H), 3.07 (dd, J=14.16, 9.39 Hz, 1 H), 2.86 - 2.92 (m, 1 H), 2.34 - 2.42 (m, 2 H), 1.44 (t, J=7.00 Hz, 3 H), 1.32 - 1.36 (m, 1 H), 1.08 (dd, J=4.77, 2.38 Hz, 2 H), 0.94 - 1.01 (m, 3 H).

### Preparation of Example 38: N-((S)-1-((1P)-1-(4-chloro-3-(cyclopropanesulfonamido)-1-(2,2-difluoroethyl)-1H-indazol-7-yl)-6-oxo-4-(6-(trifluoromethyl)pyridin-3-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

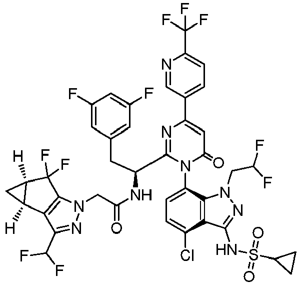

The title compound was prepared according to General Procedure B using 5-(tributylstannyl)-2-(trifluoromethyl)pyridine as the coupling partner. The experiment afforded the title compound, N-((S)-1-((1P)-1-(4-chloro-3-(cyclopropanesulfonamido)-1-(2,2-difluoroethyl)-1H-indazol-7-yl)-6-oxo-4-(6-(trifluoromethyl)pyridin-3-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.5 min.; observed ion = 976.2 (M+H). 1H NMR (500 MHz, METHANOL-d4) δ ppm 9.56 (d, J=2.09 Hz, 1 H), 8.77 - 8.84 (m, 1 H), 8.00 (dd, J=8.20, 0.75 Hz, 1 H), 7.39 (d, J=8.05 Hz, 1 H), 7.33 (s, 1 H), 7.30 (d, J=8.05 Hz, 1 H), 6.55 - 6.84 (m, 4 H), 5.93 - 6.24 (m, 1 H), 4.68 - 4.72 (m, 1 H), 4.57 - 4.67 (m, 2 H), 4.35 - 4.46 (m, 1 H), 3.96 - 4.07 (m, 1 H), 3.38 - 3.44 (m, 1 H), 3.09 (dd, J=14.31, 9.84 Hz, 1 H), 2.90 (tt, J=8.05, 4.77 Hz, 1 H), 2.31 - 2.41 (m, 2 H), 1.30 - 1.35 (m, 1 H), 1.07 - 1.10 (m, 2 H), 0.93 - 1.02 (m, 3 H).

### Preparation of Example 39: N-((S)-1-((1'P)-1'-(4-chloro-3-(cyclopropanesulfonamido)-1-(2,2-difluoroethyl)-1H-indazol-7-yl)-5-ethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

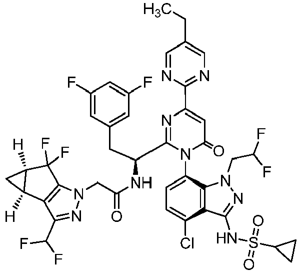

The title compound was prepared according to General Procedure A using 5-ethyl-2-(tributylstannyl)pyrimidine as the coupling partner. The experiment afforded the title compound, N-((S)-1-((1'P)-1'-(4-chloro-3-(cyclopropanesulfonamido)-1-(2,2-difluoroethyl)-1H-indazol-7-yl)-5-ethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.43 min.; observed ion = 937.3 (M+H). 1H NMR (500 MHz, METHANOL-d4) δ ppm 8.97 (s, 2 H), 7.71 (s, 1 H), 7.41 (d, J=8.05 Hz, 1 H), 7.27 (d, J=7.75 Hz, 1 H), 6.47 - 6.82 (m, 4 H), 5.95 - 6.20 (m, 1 H), 4.74 - 4.77 (m, 1 H), 4.53 - 4.71 (m, 2 H), 4.28 - 4.39 (m, 1 H), 3.91 - 4.02 (m, 1 H), 3.07 (dd, J=14.16, 9.69 Hz, 1 H), 2.81 - 2.91 (m, 3 H), 2.33 - 2.46 (m, 2 H), 1.37 - 1.40 (m, 3 H), 1.32 - 1.36 (m, 1 H), 1.07 - 1.14 (m, 2 H), 0.95 - 1.03 (m, 3 H).

### Preparation of Example 40: N-((S)-1-((1P)-1-(4-chloro-3-(cyclopropanesulfonamido)-1-(2,2-difluoroethyl)-1H-indazol-7-yl)-4-(6-methoxypyrazin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

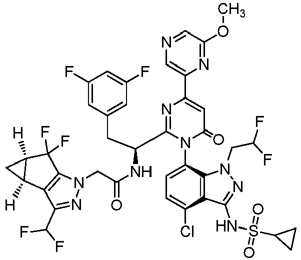

The title compound was prepared according to General Procedure B using 2-methoxy-6-(tributylstannyl)pyrazine as the coupling partner. The experiment afforded the title compound, N-((S)-1-((1P)-1-(4-chloro-3-(cyclopropanesulfonamido)-1-(2,2-difluoroethyl)-1H-indazol-7-yl)-4-(6-methoxypyrazin-2-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.45 min.; observed ion = 939.3 (M+H). 1H NMR (500 MHz, METHANOL-d4) δ ppm 9.27 (s, 1 H), 8.42 (s, 1 H), 7.54 (s, 1 H), 7.39 (d, J=7.75 Hz, 1 H), 7.26 (d, J=7.75 Hz, 1 H), 6.53 - 6.84 (m,4 H), 5.95 - 6.22 (m, 1 H), 4.69 - 4.73 (m, 1 H), 4.56 - 4.68 (m, 2 H), 4.35 - 4.45 (m, 1 H), 4.13 (s, 3 H), 3.96 - 4.04 (m, 1 H), 3.39 - 3.43 (m, 1 H), 3.08 (dd, J=14.31, 9.54 Hz, 1 H), 2.86 - 2.93 (m, 1 H), 2.34 - 2.45 (m, 2 H), 1.32 - 1.37 (m, 1 H), 1.06 - 1.11 (m, 2 H), 0.92 - 1.01 (m, 3 H).

### Preparation of Example 41: N-((S)-1-((1P)-1-(4-chloro-3-(cyclopropanesulfonamido)-1-(2,2-difluoroethyl)-1H-indazol-7-yl)-4-(3-methoxy-3-methylbut-1-yn-1-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

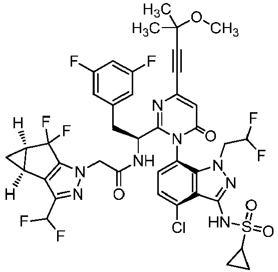

A vial was charged with (1P)-1-(4-chloro-3-(cyclopropanesulfonamido)-1-(2,2-difluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (30 mg, 0.031 mmol), 3-methoxy-3-methylbut-1-yne (15.03 mg, 0.153 mmol), copper(I) iodide (0.583 mg, 3.06 µmol), N,N-Dimethylformamide (DMF) (1.5 mL) and triethylamine (0.013 mL, 0.092 mmol). To the mixture was added bis(triphenylphosphine)palladium(II) chloride (2.150 mg, 3.06 µmol). The mixture was stirred at room temp for 1 h upon which time LCMS analysis indicated completion of the reaction. The mixture was diluted with ethyl acetate; washed with water; dried (Na2SO4); and filtered. The filtrate was concentrated under reduced pressure and the resulting residue was then diluted with 2 mL of DMF subjected to HPLC purification to afford the title compound, N-((S)-1-((1P)-1-(4-chloro-3-(cyclopropanesulfonamido)-1-(2,2-difluoroethyl)-1H-indazol-7-yl)-4-(3-methoxy-3-methylbut-1-yn-1-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.48 min.; observed ion = 927.2 (M+H). 1H NMR (500 MHz, METHANOL-d4) δ ppm 7.36 (d, J=8.05 Hz, 1 H), 7.23 (d, J=8.05 Hz, 1 H), 6.47 - 6.83 (m, 5 H), 5.93 - 6.21 (m, 1 H), 4.52 - 4.67 (m, 3 H), 4.31 - 4.41 (m, 1 H), 3.86 - 3.98 (m, 1 H), 3.45 (s, 3 H), 2.97 - 3.04 (m, 1 H), 2.84 - 2.91 (m, 1 H), 2.38 - 2.48 (m, 2 H), 1.57 (s, 6 H), 1.34 - 1.39 (m, 1 H), 1.27 - 1.31 (m, 1 H), 1.05 - 1.09 (m, 2 H), 0.93 - 1.01 (m, 3 H).

### Preparation of Example 42: N-((S)-1-((1P)-1-(4-chloro-3-(cyclopropanesulfonamido)-1-(2,2-difluoroethyl)-1H-indazol-7-yl)-4-(1-(2,2-difluoropropyl)-1H-pyrazol-3-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

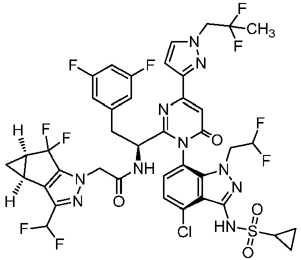

The title compound was prepared according to General Procedure B using 1-(2,2-difluoropropyl)-3-(tributylstannyl)-1H-pyrazole as the coupling partner. The experiment afforded the title compound, N-((S)-1-((1P)-1-(4-chloro-3-(cyclopropanesulfonamido)-1-(2,2-difluoroethyl)-1H-indazol-7-yl)-4-(1-(2,2-difluoropropyl)-1H-pyrazol-3-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.42 min.; observed ion = 975.3 (M+H). 1H NMR (500 MHz, METHANOL-d4) δ ppm 7.87 (d, J=2.38 Hz, 1 H), 7.36 (d, J=7.75 Hz, 1 H), 7.19 (d, J=7.75 Hz, 1 H), 7.15 (d, J=2.68 Hz, 1 H), 7.10 (s, 1 H), 6.52 - 6.83 (m, 4 H), 5.93 - 6.21 (m, 1 H), 4.68 - 4.78 (m, 3 H), 4.55 - 4.68 (m, 2 H), 4.34 - 4.44 (m, 1 H), 3.93 - 4.05 (m, 1 H), 3.35 - 3.40 (m, 1 H), 3.05 (dd, J=13.86, 9.09 Hz, 1 H), 2.89 (tt, J=8.01, 4.81 Hz, 1 H), 2.32 - 2.45 (m, 2 H), 1.67 (t, J=18.63 Hz, 3 H), 1.32 - 1.37 (m, 1 H), 1.06 - 1.11 (m, 2 H), 0.93 - 1.02 (m, 3 H).

### Preparation of Example 43: N-((S)-1-((1'P)-1'-(4-chloro-3-(cyclopropanesulfonamido)-1-(2,2-difluoroethyl)-1H-indazol-7-yl)-4-methyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

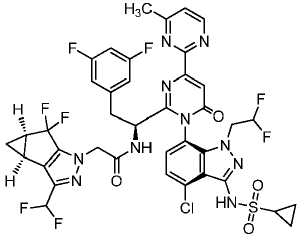

The title compound was prepared according to General Procedure B using 4-methyl-2-(tributylstannyl)pyrimidine as the coupling partner. The experiment afforded the title compound, N-((S)-1-((1'P)-1'-(4-chloro-3-(cyclopropanesulfonamido)-1-(2,2-difluoroethyl)-1H-indazol-7-yl)-4-methyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.38 min.; observed ion = 923.3 (M+H). 1H NMR (500 MHz, METHANOL-d4) δ ppm 8.92 (d, J=5.36 Hz, 1 H), 7.73 (s, 1 H), 7.55 (d, J=5.07 Hz, 1 H), 7.40 (d, J=7.75 Hz, 1 H), 7.25 (d, J=7.75 Hz, 1 H), 6.48 - 6.82 (m, 4 H), 5.93 - 6.21 (m, 1 H), 4.77 (dd, J=9.24, 4.77 Hz, 1 H), 4.57 - 4.71 (m, 2 H), 4.28 - 4.38 (m, 1 H), 3.91 - 4.02 (m, 1 H), 3.06 (dd, J=14.31, 9.24 Hz, 1 H), 2.84 - 2.93 (m, 1 H), 2.72 (s, 3 H), 2.35 - 2.45 (m, 2 H), 1.32 - 1.38 (m, 1 H), 1.04 - 1.15 (m, 3 H), 0.94 - 1.02 (m, 3 H).

### Preparation of Example 44: N-((S)-1-((1P)-1-(4-chloro-3-(cyclopropanesulfonamido)-1-(2,2-difluoroethyl)-1H-indazol-7-yl)-6-oxo-4-(6-(trifluoromethyl)pyridin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

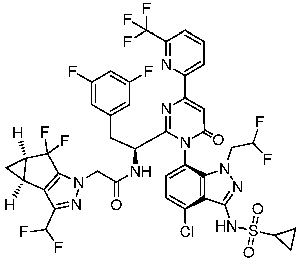

The title compound was prepared according to General Procedure B using 2-(tributylstannyl)-6-(trifluoromethyl)pyridine as the coupling partner. The experiment afforded the title compound, N-((S)-1-((1P)-1-(4-chloro-3-(cyclopropanesulfonamido)-1-(2,2-difluoroethyl)-1H-indazol-7-yl)-6-oxo-4-(6-(trifluoromethyl)pyridin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.52 min.; observed ion = 976.2 (M+H). 1H NMR (500 MHz, METHANOL-d4) δ ppm 8.79 (d, J=7.75 Hz, 1 H), 8.26 - 8.31 (m, 1 H), 7.99 (dd, J=7.75, 0.89 Hz, 1 H), 7.59 (s, 1 H), 7.39 (d, J=7.75 Hz, 1 H), 7.29 (d, J=7.75 Hz, 1 H), 6.53 - 6.85 (m, 4 H), 5.91 - 6.21 (m, 1 H), 4.74 (dd, J=9.69, 4.32 Hz, 1 H), 4.63 (q, J=16.59 Hz, 2 H), 4.34 - 4.44 (m, 1 H), 3.94 - 4.06 (m, 1 H), 3.40 - 3.44 (m, 1 H), 3.10 (dd, J=14.16, 9.69 Hz, 1 H), 2.90 (tt, J=7.97, 4.84 Hz, 1 H), 2.33 - 2.42 (m, 2 H), 1.31 - 1.36 (m, 1 H), 1.09 (dd, J=4.77, 2.38 Hz, 2 H), 0.95 - 1.01 (m, 3 H).

### Preparation of Example 45: N-((S)-1-((1P)-1-(4-chloro-3-(cyclopropanesulfonamido)-1-(2,2-difluoroethyl)-1H-indazol-7-yl)-4-(3-methoxy-3-methylbut-1-yn-1-yl)-6-oxo-1,6-dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide.

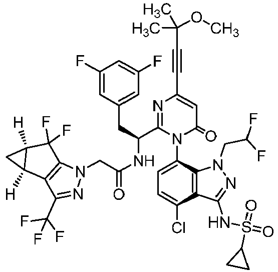

A vial was charged with (1P)-1-(4-chloro-1-(2,2-difluoroethyl)-3-(N-(4-methoxybenzyl)cydopropanesulfonamido)-1H-indazol-7-yl)-2-((S)-1-(2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (40 mg, 0.036 mmol), 3-methoxy-3-methylbut-1-yne (17.57 mg, 0.179 mmol), copper(I) iodide (0.682 mg, 3.58 µmol), N,N-Dimethylformamide (DMF) (1.5 mL) and triethylamine (0.015 mL, 0.107 mmol). To the mixture was added bis(triphenylphosphine)palladium(II) chloride (2.51 mg, 3.58 µmol). The mixture was stirred at room temp for 5 h upon which time LCMS analysis indicated the reaction was complete.

The mixture was diluted with ethyl acetate; washed with water; dried (Na2SO4); and filtered. The filtrate was concentrated under reduced pressure. The resulting residue was taken up in DCM (1 mL) and to the resulting solution was added triflic acid (0.05 mL) and TFA (1 mL). The solution was stirred at rt for 1 h and then concentrated under reduced pressure. The residue was dissolved in DMF (2 mL) and then subjected to HPLC purification to afford the title compound, N-((S)-1-((1P)-1-(4-chloro-3-(cyclopropanesulfonamido)-1-(2,2-difluoroethyl)-1H-indazol-7-yl)-4-(3-methoxy-3-methylbut-1-yn-1-yl)-6-oxo-1,6-d dihydropyrimidin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide. The sample was analyzed using LCMS Method A: retention time = 1.52 min.; observed ion = 945.3 (M+H). 1H NMR (500 MHz, METHANOL-d4) δ ppm 7.37 (d, J=7.75 Hz, 1 H), 7.24 (d, J=8.05 Hz, 1 H), 6.76 - 6.81 (m, 1 H), 6.72 (s, 1 H), 6.52 (dd, J=8.05, 2.38 Hz, 2 H), 5.92 - 6.18 (m, 1 H), 4.59 - 4.74 (m, 2 H), 4.55 - 4.59 (m, 1 H), 4.28 - 4.40 (m, 1 H), 3.86 - 4.00 (m, 1 H), 3.45 (s, 3 H), 3.00 (dd, J=14.16, 9.98 Hz, 1 H), 2.85 - 2.93 (m, 1 H), 2.39 - 2.53 (m, 2 H), 1.57 (s, 6 H), 1.36 - 1.43 (m, 1 H), 1.03 - 1.09 (m, 3 H), 0.93 - 0.99 (m, 2 H).

### Preparation of Example 46: N-((S)-1-((1'P)-1'-(4-chloro-3-(cyclopropanesulfonamido)-1-(2,2-difluoroethyl)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

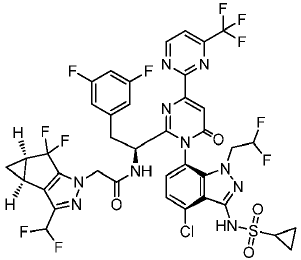

The title compound was prepared according to General Procedure B using 2-(tributylstannyl)-4-(trifluoromethyl)pyrimidine as the coupling partner. The experiment afforded the title compound, N-((S)-1-((1'P)-1'-(4-chloro-3-(cyclopropanesulfonamido)-1-(2,2-difluoroethyl)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.45 min.; observed ion = 977.3 (M+H). 1H NMR (500 MHz, METHANOL-d4) δ ppm 9.41 (d, J=5.07 Hz, 1 H), 8.08 (d, J=5.07 Hz, 1 H), 7.75 (s, 1 H), 7.41 (d, J=7.75 Hz, 1 H), 7.27 (d, J=8.05 Hz, 1 H), 6.49 - 6.83 (m, 4 H), 5.94 - 6.22 (m, 1 H), 4.76 (dd, J=9.54, 4.77 Hz, 1 H), 4.57 - 4.70 (m, 2 H), 4.33 - 4.44 (m, 1 H), 3.93 - 4.05 (m, 1 H), 3.35 - 3.39 (m, 1 H), 3.09 (dd, J=14.31, 9.54 Hz, 1 H), 2.89 (tt, J=8.01, 4.81 Hz, 1 H), 2.32 - 2.44 (m, 2 H), 1.31 - 1.37 (m, 1 H), 1.07 - 1.11 (m, 2 H), 0.94 - 1.03 (m, 3 H).

### Preparation of Example 47: N-((S)-1-((1P)-1-(4-chloro-3-(cyclopropanesulfonamido)-1-(2,2-difluoroethyl)-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

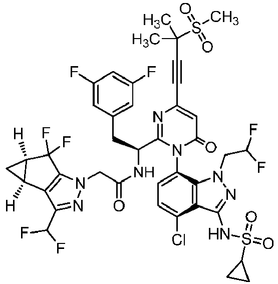

A vial was charged with (1P)-1-(4-chloro-3-(cyclopropanesulfonamido)-1-(2,2-difluoroethyl)-1H-indazol-7-yl)-2-((S)-1-(2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamido)-2-(3,5-difluorophenyl)ethyl)-6-oxo-1,6-dihydropyrimidin-4-yl trifluoromethanesulfonate (30 mg, 0.031 mmol), 3-methyl-3-(methylsulfonyl)but-1-yne (13.44 mg, 0.092 mmol), copper(I) iodide (0.583 mg, 3.06 µmol), N,N-Dimethylformamide (DMF) (1.5 mL) and triethylamine (0.013 mL, 0.092 mmol). To the mixture was added bis(triphenylphosphine)palladium(II) chloride (2.150 mg, 3.06 µmol). The mixture was degassed (brief high vacuum, then refilled with Ar) and then heated at 60 °C for 3 h. The mixture was cooled to room temperature; diluted with ethyl acetate; washed with water; dried (Na2SO4); and then filtered. The filtrate was concentrated under reduced pressure and the resulting residue was dissolved in DMF (2 mL) and then subjected to HPLC purification to afford the title compound, N-((S)-1-((1P)-1-(4-chloro-3-(cyclopropanesulfonamido)-1-(2,2-difluoroethyl)-1H-indazol-7-yl)-4-(3-methyl-3-(methylsulfonyl)but-1-yn-1-yl)-6-oxo-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.38 min.; observed ion = 975.3 (M+H). 1H NMR (500 MHz, METHANOL-d4) δ ppm 7.37 (d, J=7.75 Hz, 1 H), 7.24 (d, J=8.05 Hz, 1 H), 6.82 (s, 1 H), 6.48 - 6.81 (m, 4 H), 5.93 - 6.20 (m, 1 H), 4.53 - 4.67 (m, 3 H), 4.31 - 4.43 (m, 1 H), 3.86 - 3.99 (m, 1 H), 3.19 (s, 3 H), 2.99 (dd, J=14.31, 9.84 Hz, 1 H), 2.88 (tt, J=7.94, 4.73 Hz, 1 H), 2.39 - 2.51 (m, 2 H), 1.78 (s, 6 H), 1.33 - 1.39 (m, 1 H), 1.05 - 1.10 (m, 2 H), 0.94 - 1.02 (m, 3 H).

### Preparation of Example 48: N-((S)-1-((1'P)-1'-(4-chloro-3-(cyclopropanesulfonamido)-1-(2,2-difluoroethyl)-1H-indazol-7-yl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

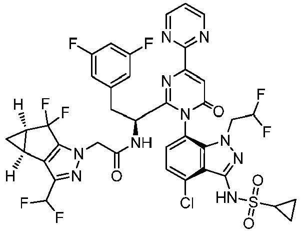

The title compound was prepared according to General Procedure B using 2-(tributylstannyl)pyrimidine as the coupling partner. The experiment afforded the title compound, N-((S)-1-((1'P)-1'-(4-chloro-3-(cyclopropanesulfonamido)-1-(2,2-difluoroethyl)-1H-indazol-7-yl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.34 min.; observed ion = 909.4 (M+H). 1H NMR (500 MHz, METHANOL-d4) δ ppm 9.10 (d, J=4.77 Hz, 2 H), 7.75 (s, 1 H), 7.68 (t, J=4.92 Hz, 1 H), 7.41 (d, J=8.05 Hz, 1 H), 7.28 (d, J=8.05 Hz, 1 H), 6.46 - 6.83 (m, 4 H), 5.91 - 6.20 (m, 1 H), 4.73 - 4.78 (m, 1 H), 4.57 - 4.72 (m, 2 H), 4.30 - 4.40 (m, 1 H), 3.92 - 4.03 (m, 1 H), 3.07 (dd, J=14.01, 9.54 Hz, 1 H), 2.89 (tt, J=8.01, 4.81 Hz, 1 H), 2.32 - 2.49 (m, 2 H), 1.32 - 1.38 (m, 1 H), 1.06 - 1.11 (m, 2 H), 0.94 - 1.02 (m, 3 H).

### Preparation of Example 49: N-((S)-1-((1P)-1-(4-chloro-3-(cyclopropanesulfonamido)-1-(2,2-difluoroethyl)-1H-indazol-7-yl)-2'-ethoxy-6-oxo-1,6-dihydro-[4,5'-bipyrimidin]-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

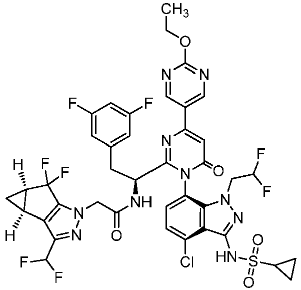

The title compound was prepared according to General Procedure B using 4-(dibutyl(2-ethoxypyrimidin-5-yl)stannyl)butan-1-ylium as the coupling partner. The experiment afforded the title compound, N-((S)-1-((1P)-1-(4-chloro-3-(cyclopropanesulfonamido)-1-(2,2-difluoroethyl)-1H-indazol-7-yl)-2'-ethoxy-6-oxo-1,6-dihydro-[4,5'-bipyrimidin]-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.43 min.; observed ion = 953.4 (M+H). 1H NMR (500 MHz, METHANOL-d4) δ ppm 9.37 (s, 2 H), 7.38 (d, J=8.05 Hz, 1 H), 7.26 (d, J=8.05 Hz, 1 H), 7.19 (s, 1 H), 6.53 - 6.84 (m, 4 H), 5.94 - 6.22 (m, 1 H), 4.65 - 4.68 (m, 1 H), 4.56 - 4.61 (m, 2 H), 4.34 - 4.44 (m, 1 H), 3.95 - 4.04 (m, 1 H), 3.38 (dd, J=14.31, 4.17 Hz, 1 H), 3.06 (dd, J=14.16, 9.69 Hz, 1 H), 2.86 - 2.93 (m, 1 H), 2.33 - 2.43 (m, 2 H), 1.46 - 1.49 (m, 3 H), 1.30 - 1.36 (m, 1 H), 1.07 - 1.13 (m, 2 H), 0.90 - 1.01 (m, 3 H).

### Preparation of Example 50: N-((S)-1-((1P)-1-(4-chloro-3-(cyclopropanesulfonamido)-1-(2,2-difluoroethyl)-1H-indazol-7-yl)-6-oxo-4-(pyrazin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

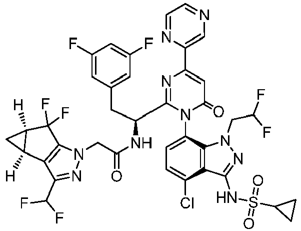

The title compound was prepared according to General Procedure B using 2-(tributylstannyl)pyrazine as the coupling partner. The experiment afforded the title compound, N-((S)-1-((1P)-1-(4-chloro-3-(cyclopropanesulfonamido)-1-(2,2-difluoroethyl)-1H-indazol-7-yl)-6-oxo-4-(pyrazin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method A: retention time = 1.37 min.; observed ion = 909.3 (M+H). 1H NMR (500 MHz, METHANOL-d4) δ ppm 9.71 (d, J=1.49 Hz, 1 H), 8.77 - 8.84 (m, 2 H), 7.58 (s, 1 H), 7.40 (d, J=8.05 Hz, 1 H), 7.30 (d, J=7.75 Hz, 1 H), 6.54 - 6.83 (m, 4 H), 5.94 - 6.22 (m, 1 H), 4.69 - 4.72 (m, 1 H), 4.56 - 4.69 (m, 2 H), 4.34 - 4.42 (m, 1 H), 3.92 - 4.02 (m, 1 H), 3.39 - 3.44 (m, 1 H), 3.09 (dd, J=14.31, 9.83 Hz, 1 H), 2.86 - 2.93 (m, 1 H), 2.35 - 2.46 (m, 2 H), 1.31 - 1.36 (m, 1 H), 1.07 - 1.14 (m, 2 H), 0.93 - 1.01 (m, 3 H).

### Preparation of Example 51: N-((S)-1-((1'P)-1'-(4-chloro-1-(2,2-difluoroethyl)-3-(methylsulfonamido)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

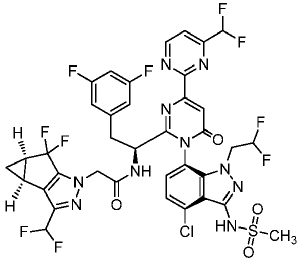

The title compound was prepared according to General Procedure C using 4-(difluoromethyl)-2-(tributylstannyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.02 min.; observed ion = 933.0 (M+H).

### Preparation of Example 52: N-((S)-1-((1'P)-1'-(4-chloro-1-(2,2-difluoroethyl)-3-(methylsulfonamido)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

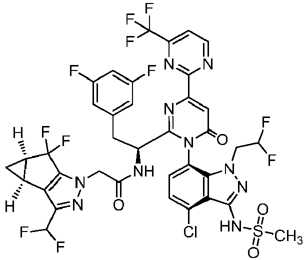

The title compound was prepared according to General Procedure C using 2-(tributylstannyl)-4-(trifluoromethyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.11 min.; observed ion = 950.9 (M+H).

### Preparation of Example 53: N-((S)-1-((1'P)-1'-(4-chloro-1-(2,2-difluoroethyl)-3-(methylsulfonamido)-1H-indazol-7-yl)-5-ethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

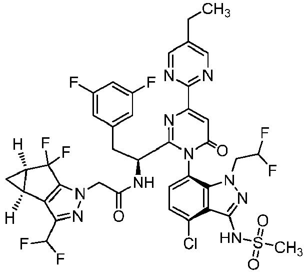

The title compound was prepared according to General Procedure C using 5-ethyl-2-(tributylstannyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-5-ethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.07 min.; observed ion = 911.2 (M+H).

### Preparation of Example 54: N-((S)-1-((1'P)-1'-(4-chloro-1-(2,2-difluoroethyl)-3-(methylsulfonamido)-1H-indazol-7-yl)-5-fluoro-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

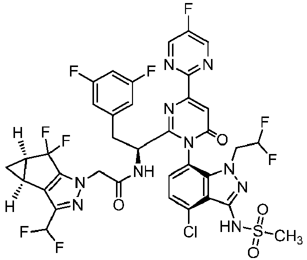

The title compound was prepared according to General Procedure C using 5-fluoro-2-(tributylstannyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-5-fluoro-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 2.95 min.; observed ion = 901 (M+H).

### Preparation of Example 55: N-((S)-1-((1'P)-1'-(4-chloro-1-(2,2-difluoroethyl)-3-(methylsulfonamido)-1H-indazol-7-yl)-5-ethoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

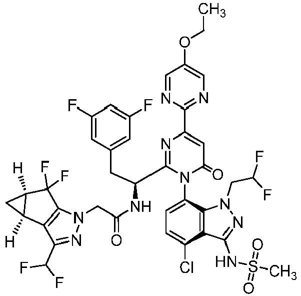

The title compound was prepared according to General Procedure C using 5-ethoxy-2-(tributylstannyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-5-ethoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.07 min.; observed ion = 927.1 (M+H).

### Preparation of Example 56: N-((S)-1-((1'P)-1'-(4-chloro-1-(2,2-difluoroethyl)-3-(methylsulfonamido)-1H-indazol-7-yl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

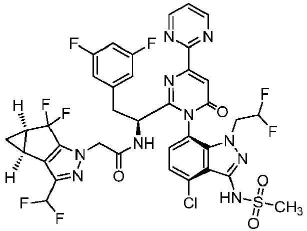

The title compound was prepared according to General Procedure C using 2-(tributylstannyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 2.87 min.; observed ion = 883 (M+H).

### Preparation of Example 57: N-((S)-1-((1'P)-1'-(4-chloro-1-(2,2-difluoroethyl)-3-(methylsulfonamido)-1H-indazol-7-yl)-4,6-dimethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

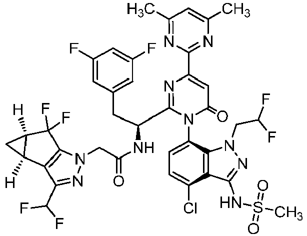

The title compound was prepared according to General Procedure C using 4,6-dimethyl-2-(tributylstannyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-4,6-dimethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.05 min.; observed ion = 911 (M+H).

### Preparation of Example 58: N-((S)-1-((1'P)-1'-(4-chloro-1-(2,2-difluoroethyl)-3-(methylsulfonamido)-1H-indazol-7-yl)-4-methyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

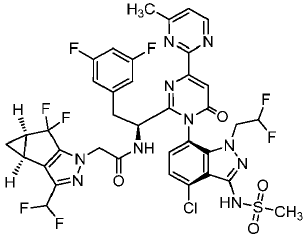

The title compound was prepared according to General Procedure C using 4-methyl-2-(tributylstannyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-4-methyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 2.96 min.; observed ion = 897 (M+H).

### Preparation of Example 59: N-((S)-1-((1'P)-1'-(4-chloro-1-(2,2-difluoroethyl)-3-(methylsulfonamido)-1H-indazol-7-yl)-4-methoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

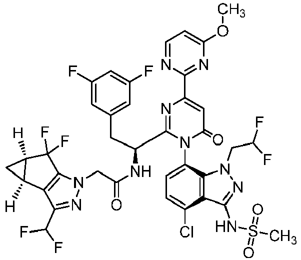

The title compound was prepared according to General Procedure C using 4-methoxy-2-(tributylstannyl)pyrimidine as the coupling partner. The experiment afforded the title compound, N-((S)-1-((1'P)-1'-(4-chloro-1-(2,2-difluoroethyl)-3-(methylsulfonamido)-1H-indazol-7-yl)-4-methoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.03 min.; observed ion = 913.1 (M+H).

### Preparation of Example 60: N-((S)-1-((1P)-1-(4-chloro-1-(2,2-difluoroethyl)-3-(methylsulfonamido)-1H-indazol-7-yl)-6-oxo-4-(6-(trifluoromethyl)pyridin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

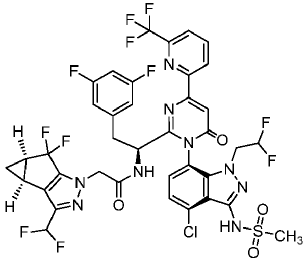

The title compound was prepared according to General Procedure C using 2-(tributylstannyl)-6-(trifluoromethyl)pyridine as the coupling partner. The experiment afforded the title compound, N-((S)-1-((1P)-1-(4-chloro-1-(2,2-difluoroethyl)-3-(methylsulfonamido)-1H-indazol-7-yl)-6-oxo-4-(6-(trifluoromethyl)pyridin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.26 min.; observed ion = 950.1 (M+H).

### Preparation of Example 61: N-((S)-1-((1P)-1-(4-chloro-1-(2,2-difluoroethyl)-3-(methylsulfonamido)-1H-indazol-7-yl)-6-oxo-4-(6-(trifluoromethyl)pyridin-3-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

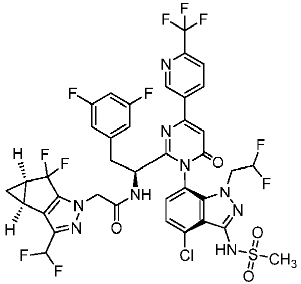

The title compound was prepared according to General Procedure C using 5-(tributylstannyl)-2-(trifluoromethyl)pyridine as the coupling partner. The experiment afforded the title compound, N-((S)-1-((1P)-1-(4-chloro-1-(2,2-difluoroethyl)-3-(methylsulfonamido)-1H-indazol-7-yl)-6-oxo-4-(6-(trifluoromethyl)pyridin-3-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.23 min.; observed ion = 949.9 (M+H).

### Preparation of Example 62: N-((S)-1-((1P)-1-(4-chloro-1-(2,2-difluoroethyl)-3-(methylsulfonamido)-1H-indazol-7-yl)-6-oxo-4-(pyrazin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

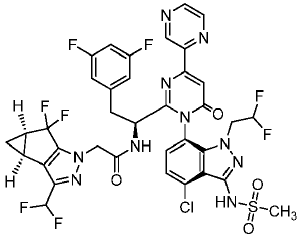

The title compound was prepared according to General Procedure C using 2-(tributylstannyl)pyrazine as the coupling partner. The experiment afforded the title compound, N-((S)-1-((1P)-1-(4-chloro-1-(2,2-difluoroethyl)-3-(methylsulfonamido)-1H-indazol-7-yl)-6-oxo-4-(pyrazin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 2.94 min.; observed ion = 883 (M+H).

### Preparation of Example 63: N-((S)-1-((1'P)-1'-(4-chloro-3-(methylsulfonamido)-1-(2,2,2-trifluoroethyl)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

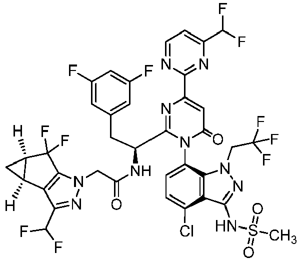

The title compound was prepared according to General Procedure D using 4-(difluoromethyl)-2-(tributylstannyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-4-(difluoromethyl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.04 min.; observed ion = 950.9 (M+H).

### Preparation of Example 64: N-((S)-1-((1'P)-1'-(4-chloro-3-(methylsulfonamido)-1-(2,2,2-trifluoroethyl)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

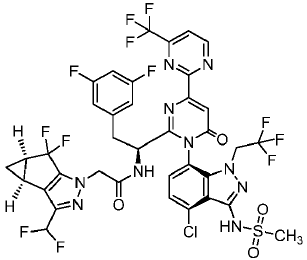

The title compound was prepared according to General Procedure D using 2-(tributylstannyl)-4-(trifluoromethyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-6'-oxo-4-(trifluoromethyl)-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.13 min.; observed ion = 969.1 (M+H).

### Preparation of Example 65: N-((S)-1-((1'P)-1'-(4-chloro-3-(methylsulfonamido)-1-(2,2,2-trifluoroethyl)-1H-indazol-7-yl)-5-ethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

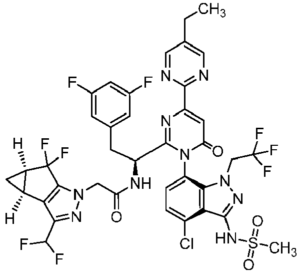

The title compound was prepared according to General Procedure D using 5-ethyl-2-(tributylstannyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-5-ethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.11 min.; observed ion = 929 (M+H).

### Preparation of Example 66: N-((S)-1-((1'P)-1'-(4-chloro-3-(methylsulfonamido)-1-(2,2,2-trifluoroethyl)-1H-indazol-7-yl)-5-fluoro-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

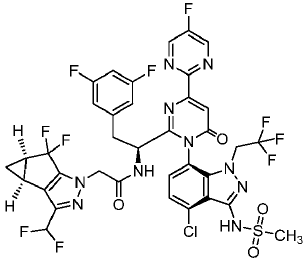

The title compound was prepared according to General Procedure D using 5-fluoro-2-(tributylstannyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-5-fluoro-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 2.98 min.; observed ion = 919.1 (M+H).

### Preparation of Example 67: N-((S)-1-((1'P)-1'-(4-chloro-3-(methylsulfonamido)-1-(2,2,2-trifluoroethyl)-1H-indazol-7-yl)-5-ethoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

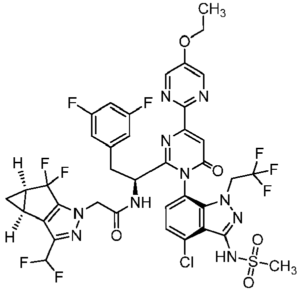

The title compound was prepared according to General Procedure D using 5-ethoxy-2-(tributylstannyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-5-ethoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.1 min.; observed ion = 945 (M+H).

### Preparation of Example 68: N-((S)-1-((1'P)-1'-(4-chloro-3-(methylsulfonamido)-1-(2,2,2-trifluoroethyl)-1H-indazol-7-yl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

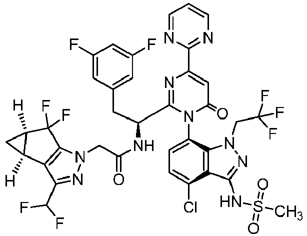

The title compound was prepared according to General Procedure D using 2-(tributylstannyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 2.91 min.; observed ion = 900.9 (M+H).

### Preparation of Example 69: N-((S)-1-((1'P)-1'-(4-chloro-3-(methylsulfonamido)-1-(2,2,2-trifluoroethyl)-1H-indazol-7-yl)-4,6-dimethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

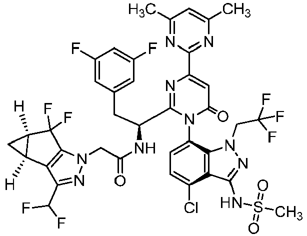

The title compound was prepared according to General Procedure D using 4,6-dimethyl-2-(tributylstannyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-4,6-dimethyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.1 min.; observed ion = 929.2 (M+H).

### Preparation of Example 70: N-((S)-1-((1'P)-1'-(4-chloro-3-(methylsulfonamido)-1-(2,2,2-trifluoroethyl)-1H-indazol-7-yl)-4-methyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

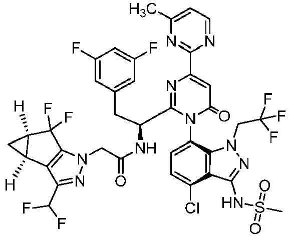

The title compound was prepared according to General Procedure D using 4-methyl-2-(tributylstannyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-4-methyl-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3 min.; observed ion = 914.9 (M+H).

### Preparation of Example 71: N-((S)-1-((1'P)-1'-(4-chloro-3-(methylsulfonamido)-1-(2,2,2-trifluoroethyl)-1H-indazol-7-yl)-4-methoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

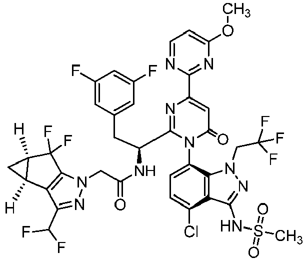

The title compound was prepared according to General Procedure D using 4-methoxy-2-(tributylstannyl)pyrimidine as the coupling partner. The experiment afforded the title compound, N-((S)-1-((1'P)-1'-(4-chloro-3-(methylsulfonamido)-1-(2,2,2-trifluoroethyl)-1H-indazol-7-yl)-4-methoxy-6'-oxo-1',6'-dihydro-[2,4'-bipyrimidin]-2'-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.06 min.; observed ion = 931 (M+H).

### Preparation of Example 72: N-((S)-1-((1P)-1-(4-chloro-3-(methylsulfonamido)-1-(2,2,2-trifluoroethyl)-1H-indazol-7-yl)-6-oxo-4-(6-(trifluoromethyl)pyridin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

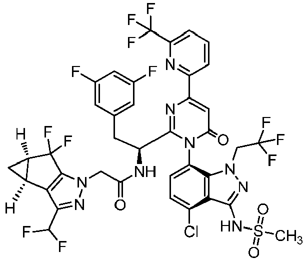

The title compound was prepared according to General Procedure D using 2-(tributylstannyl)-6-(trifluoromethyl)pyridine as the coupling partner. The experiment afforded the title compound, N-((S)-1-((1P)-1-(4-chloro-3-(methylsulfonamido)-1-(2,2,2-trifluoroethyl)-1H-indazol-7-yl)-6-oxo-4-(6-(trifluoromethyl)pyridin-2-yl)-1,6-dihydropyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method B: retention time = 3.29 min.; observed ion = 967.9 (M+H).

### IUPAC Chemical Names:

The IUPAC chemical names for each example are listed below. At this time these names are not recognized by common software such tools such as ChemDraw or JChem. Therefore, the chemical names used throughout the Examples section above were generated with ChemDraw and then appended with the correct P/M designation. The chemical names above can be converted to chemical structures using ChemDraw after the P/M nomenclature-e.g., "(1P)-"- is removed.

| **Example** | **IUPAC Name** |
|---|---|
| Example 1 | N-[(1S)-1-[(1P)-1-(4-chloro-3-methanesulfonamido-1-methyl-1H-indazol-7-yl)-6-oxo-4-phenyl-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 2 | N-[(1R)-1-[(1P)-1-(4-chloro-3-methanesulfonamido-1-methyl-1H-indazol-7-yl)-6-oxo-4-phenyl-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 3 | N-[(1S)-1-[(1P)-1-(4-chloro-3-methanesulfonamido-1-methyl-1H-indazol-7-yl)-4-(2,4-difluorophenyl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 4 | N-[(1S)-1-[(1'P)-1'-(4-chloro-3-methanesulfonamido-1-methyl-1H-indazol-7-yl)-4,6-dimethyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 5 | N-[(1S)-1-[(1'P)-1'-(4-chloro-3-methanesulfonamido-1-methyl-1H-indazol-7-yl)-4-(difluoromethyl)-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 6 | N-[(1S)-1-[(1'P)-1'-(4-chloro-3-methanesulfonamido-1-methyl-1H-indazol-7-yl)-6'-oxo-4-(trifluoromethyl)-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 7 | N-[(1S)-1-[(1P)-1-(4-chloro-3-methanesulfonamido-1-methyl-1H-indazol-7-yl)-6-oxo-4-[6-(trifluoromethyl)pyridin-2-yl]-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 8 | N-[(1S)-1-[(1P)-1-(4-chloro-3-methanesulfonamido-1-methyl-1H-indazol-7-yl)-4-[1-(2,2-difluoropropyl)-1H-pyrazol-3-yl]-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 9 | N-[(1S)-1-[(1P)-1-(4-chloro-3-methanesulfonamido-1-methyl-1H-indazol-7-yl)-4-(3-methoxy-3-methylbut-1-yn-1-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 10 | N-[(1S)-1-[(1P)-1-(4-chloro-3-methanesulfonamido-1-methyl-1H-indazol-7-yl)-4-(3-methoxy-3-methylbutyl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 11 | N-[(1S)-1-[(1P)-1-(4-chloro-3-methanesulfonamido-1-methyl-1H-indazol-7-yl)-2'-ethoxy-6-oxo-1,6-dihydro-[4,5'-bipyrimidin]-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 12 | N-[(1S)-1-[(1'P)-1'-(4-chloro-3-methanesulfonamido-1-methyl-1H-indazol-7-yl)-5-ethyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 13 | N-[(1S)-1-[(1P)-1-(4-chloro-3-methanesulfonamido-1-methyl-1H-indazol-7-yl)-4-(5-methoxypyrazin-2-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 14 | N-[(1S)-1-[(1'P)-1'-(4-chloro-3-methanesulfonamido-1-methyl-1H-indazol-7-yl)-5-ethoxy-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 15 | N-[(1S)-1-[(1P)-1-(4-chloro-3-methanesulfonamido-1-methyl-1H-indazol-7-yl)-4-[2-(1,1-difluoroethyl)-1,3-thiazol-5-yl]-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 16 | N-[(1S)-1-[(1P)-1-(4-chloro-3-methanesulfonamido-1-methyl-1H-indazol-7-yl)-4-(4-methylpyridin-2-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 17 | N-[(1S)-1-[(1P)-1-(4-chloro-3-methanesulfonamido-1-methyl-1H-indazol-7-yl)-4-(2-methanesulfonyl-1,3-thiazol-4-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 18 | N-[(1S)-1-[(1P)-1-(4-chloro-3-methanesulfonamido-1-methyl-1H-indazol-7-yl)-4-[2-(2-hydroxypropan-2-yl)-1,3-thiazol-4-yl]-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 19 | N-[(1S)-1-[(1P)-1-(4-chloro-3-methanesulfonamido-1-methyl-1H-indazol-7-yl)-4-[1-methyl-3-(trifluoromethyl)-1H-pyrazol-5-yl]-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 20 | N-[(1S)-1-[(1P)-1-(4-chloro-3-methanesulfonamido-1-methyl-1H-indazol-7-yl)-6-oxo-4-(pyrazin-2-yl)-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 21 | N-[(1S)-1-[(1P)-1-(4-chloro-3-methanesulfonamido-1-methyl-1H-indazol-7-yl)-4-(3-methanesulfonyl-3-methylbut-1-yn-1-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 22 | N-[(1S)-1-[(1P)-1-(4-chloro-3-methanesulfonamido-1-methyl-1H-indazol-7-yl)-4-(6-ethoxypyridin-3-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 23 | N-[(1S)-1-[(1P)-1-(4-chloro-3-methanesulfonamido-1-methyl-1H-indazol-7-yl)-4-(6-fluoropyridin-2-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 24 | N-[(1S)-1-[(1P)-1-(4-chloro-3-methanesulfonamido-1-methyl-1H-indazol-7-yl)-4-(5-chloropyridin-2-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 25 | N-[(1S)-1-[(1P)-1-(4-chloro-3-methanesulfonamido-1-methyl-1H-indazol-7-yl)-4-[2-(2-hydroxypropan-2-yl)-1,3-thiazol-5-yl]-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 26 | N-[(1S)-1-[(1'P)-1'-[4-chloro-3-cyclopropanesulfonamido-1-(2,2-difluoroethyl)-1H-indazol-7-yl]-6'-oxo-4-(trifluoromethyl)-1',6'-dihydro-[2,4'-bipyrimidin]-2'-yl]-2-(3,5-difluorophenyl)ethyl]-2-[(2S,4R)-5,5-difluoro-9-(trifluoromethyl)-7,8-diazatricyclo[4.3.0.0^{2,4}]nona-1(6),8-dien-7-yl]acetamide |
| Example 27 | N-[(1S)-1-[(1'P)-1'-[4-chloro-3-cyclopropanesulfonamido-1-(2,2-difluoroethyl)-1H-indazol-7-yl]-4-(difluoromethyl)-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 28 | N-[(1S)-1-[(1P)-1-[4-chloro-3-cyclopropanesulfonamido-1-(2,2-difluoroethyl)-1H-indazol-7-yl]-4-(6-fluoropyridin-2-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 29 | N-[(1S)-1-[(1'P)-1'-[4-chloro-3-cyclopropanesulfonamido-1-(2,2-difluoroethyl)-1H-indazol-7-yl]-5-ethoxy-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 30 | N-[(1S)-1-[(1'P)-1'-[4-chloro-3-cyclopropanesulfonamido-1-(2,2-difluoroethyl)-1H-indazol-7-yl]-5-fluoro-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 31 | N-[(1S)-1-[(1P)-1-[4-chloro-3-cyclopropanesulfonamido-1-(2,2-difluoroethyl)-1H-indazol-7-yl]-4-(5-methoxypyrazin-2-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 32 | N-[(1S)-1-[(1'P)-1'-[4-chloro-3-cyclopropanesulfonamido-1-(2,2-difluoroethyl)-1H-indazol-7-yl]-4-(difluoromethyl)-6'-oxo-1',6'-dihydro-[2,4'-bipyrimidin]-2'-yl]-2-(3,5-difluorophenyl)ethyl]-2-[(2S,4R)-5,5-difluoro-9-(trifluoromethyl)-7,8-diazatricyclo[4.3.0.0^{2,4}]nona-1(6),8-dien-7-yl]acetamide |
| Example 33 | N-[(1S)-1-[(1'P)-1'-[4-chloro-3-cyclopropanesulfonamido-1-(2,2-difluoroethyl)-1H-indazol-7-yl]-4-methoxy-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 34 | N-[(1S)-1-[(1'P)-1'-[4-chloro-3-cyclopropanesulfonamido-1-(2,2-difluoroethyl)-1H-indazol-7-yl]-4,6-dimethyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 35 | N-[(1S)-1-[(1P)-1-[4-chloro-3-cyclopropanesulfonamido-1-(2,2-difluoroethyl)-1H-indazol-7-yl]-4-(5-chloropyridin-2-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 36 | N-[(1S)-1-[(1P)-1-[4-chloro-3-cyclopropanesulfonamido-1-(2,2-difluoroethyl)-1H-indazol-7-yl]-4-(3-methanesulfonyl-3-methylbut-1-yn-1-yl)-6-oxo-1,6-dihydropyrimidin-2-yl]-2-(3,5-difluorophenyl)ethyl]-2-[(2S,4R)-5,5-difluoro-9-(trifluoromethyl)-7,8-diazatricydo[4.3.0.0^{2,4}]nona-1(6),8-dien-7-yl]acetamide |
| Example 37 | N-[(1S)-1-[(1P)-1-[4-chloro-3-cyclopropanesulfonamido-1-(2,2-difluoroethyl)-1H-indazol-7-yl]-4-(6-ethoxypyridin-3-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 38 | N-[(1S)-1-[(1P)-1-[4-chloro-3-cyclopropanesulfonamido-1-(2,2-difluoroethyl)-1H-indazol-7-yl]-6-oxo-4-[6-(trifluoromethyl)pyridin-3-yl]-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 39 | N-[(1S)-1-[(1'P)-1'-[4-chloro-3-cyclopropanesulfonamido-1-(2,2-difluoroethyl)-1H-indazol-7-yl]-5-ethyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 40 | N-[(1S)-1-[(1P)-1-[4-chloro-3-cyclopropanesulfonamido-1-(2,2-difluoroethyl)-1H-indazol-7-yl]-4-(6-methoxypyrazin-2-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 41 | N-[(1S)-1-[(1P)-1-[4-chloro-3-cyclopropanesulfonamido-1-(2,2-difluoroethyl)-1H-indazol-7-yl]-4-(3-methoxy-3-methylbut-1-yn-1-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 42 | N-[(1S)-1-[(1P)-1-[4-chloro-3-cyclopropanesulfonamido-1-(2,2-difluoroethyl)-1H-indazol-7-yl]-4-[1-(2,2-difluoropropyl)-1H-pyrazol-3-yl]-6-oxo-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 43 | N-[(1S)-1-[(1'P)-1'-[4-chloro-3-cyclopropanesulfonamido-1-(2,2-difluoroethyl)-1H-indazol-7-yl]-4-methyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 44 | N-[(1S)-1-[(1P)-1-[4-chloro-3-cyclopropanesulfonamido-1-(2,2-difluoroethyl)-1H-indazol-7-yl]-6-oxo-4-[6-(trifluoromethyl)pyridin-2-yl]-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 45 | N-[(1S)-1-[(1P)-1-[4-chloro-3-cyclopropanesulfonamido-1-(2,2-difluoroethyl)-1H-indazol-7-yl]-4-(3-methoxy-3-methylbut-1-yn-1-yl)-6-oxo-1,6-dihydropyrimidin-2-yl]-2-(3,5-difluorophenyl)ethyl]-2-[(2S,4R)-5,5-difluoro-9-(trifluoromethyl)-7,8-diazatricyclo[4.3.0.0^{2,4}]nona-1(6),8-dien-7-yl]acetamide |
| Example 46 | N-[(1S)-1-[(1'P)-1'-[4-chloro-3-cyclopropanesulfonamido-1-(2,2-difluoroethyl)-1H-indazol-7-yl]-6'-oxo-4-(trifluoromethyl)-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 47 | N-[(1S)-1-[(1P)-1-[4-chloro-3-cyclopropanesulfonamido-1-(2,2-difluoroethyl)-1H-indazol-7-yl]-4-(3-methanesulfonyl-3-methylbut-1-yn-1-yl)-6-oxo-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 48 | N-[(1S)-1-[(1'P)-1'-[4-chloro-3-cyclopropanesulfonamido-1-(2,2-difluoroethyl)-1H-indazol-7-yl]-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 49 | N-[(1S)-1-[(1P)-1-[4-chloro-3-cyclopropanesulfonamido-1-(2,2-difluoroethyl)-1H-indazol-7-yl]-2'-ethoxy-6-oxo-1,6-dihydro-[4,5'-bipyrimidin]-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 50 | N-[(1S)-1-[(1P)-1-[4-chloro-3-cyclopropanesulfonamido-1-(2,2-difluoroethyl)-1H-indazol-7-yl]-6-oxo-4-(pyrazin-2-yl)-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 51 | N-[(1S)-1-[(1'P)-1'-[4-chloro-1-(2,2-difluoroethyl)-3-methanesulfonamido-1H-indazol-7-yl]-4-(difluoromethyl)-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 52 | N-[(1S)-1-[(1'P)-1'-[4-chloro-1-(2,2-difluoroethyl)-3-methanesulfonamido-1H-indazol-7-yl]-6'-oxo-4-(trifluoromethyl)-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 53 | N-[(1S)-1-[(1'P)-1'-[4-chloro-1-(2,2-difluoroethyl)-3-methanesulfonamido-1H-indazol-7-yl]-5-ethyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 54 | N-[(1S)-1-[(1'P)-1'-[4-chloro-1-(2,2-difluoroethyl)-3-methanesulfonamido-1H-indazol-7-yl]-5-fluoro-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 55 | N-[(1S)-1-[(1'P)-1'-[4-chloro-1-(2,2-difluoroethyl)-3-methanesulfonamido-1H-indazol-7-yl]-5-ethoxy-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 56 | N-[(1S)-1-[(1'P)-1'-[4-chloro-1-(2,2-difluoroethyl)-3-methanesulfonamido-1H-indazol-7-yl]-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 57 | N-[(1S)-1-[(1'P)-1'-[4-chloro-1-(2,2-difluoroethyl)-3-methanesulfonamido-1H-indazol-7-yl]-4,6-dimethyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 58 | N-[(1S)-1-[(1'P)-1'-[4-chloro-1-(2,2-difluoroethyl)-3-methanesulfonamido-1H-indazol-7-yl]-4-methyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 59 | N-[(1S)-1-[(1'P)-1'-[4-chloro-1-(2,2-difluoroethyl)-3-methanesulfonamido-1H-indazol-7-yl]-4-methoxy-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 60 | N-[(1S)-1-[(1P)-1-[4-chloro-1-(2,2-difluoroethyl)-3-methanesulfonamido-1H-indazol-7-yl]-6-oxo-4-[6-(trifluoromethyl)pyridin-2-yl]-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 61 | N-[(1S)-1-[(1P)-1-[4-chloro-1-(2,2-difluoroethyl)-3-methanesulfonamido-1H-indazol-7-yl]-6-oxo-4-[6-(trifluoromethyl)pyridin-3-yl]-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 62 | N-[(1S)-1-[(1P)-1-[4-chloro-1-(2,2-difluoroethyl)-3-methanesulfonamido-1H-indazol-7-yl]-6-oxo-4-(pyrazin-2-yl)-1,6-dihydropyrimidin-2-yl]-2-(3,5-difluorophenyl)ethyl]-2-[(2S,4R)-9-(difluoromethyl)-5,5-difluoro-7,8-diazatricydo[4.3.0.0^{2,4}]nona-1(6),8-dien-7-yl]acetamide |
| Example 63 | N-[(1S)-1-[(1'P)-1'-[4-chloro-3-methanesulfonamido-1-(2,2,2-trifluoroethyl)-1H-indazol-7-yl]-4-(difluoromethyl)-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 64 | N-[(1S)-1-[(1'P)-1'-[4-chloro-3-methanesulfonamido-1-(2,2,2-trifluoroethyl)-1H-indazol-7-yl]-6'-oxo-4-(trifluoromethyl)-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 65 | N-[(1S)-1-[(1'P)-1'-[4-chloro-3-methanesulfonamido-1-(2,2,2-trifluoroethyl)-1H-indazol-7-yl]-5-ethyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 66 | N-[(1S)-1-[(1'P)-1'-[4-chloro-3-methanesulfonamido-1-(2,2,2-trifluoroethyl)-1H-indazol-7-yl]-5-fluoro-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 67 | N-[(1S)-1-[(1'P)-1'-[4-chloro-3-methanesulfonamido-1-(2,2,2-trifluoroethyl)-1H-indazol-7-yl]-5-ethoxy-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 68 | N-[(1S)-1-[(1'P)-1'-[4-chloro-3-methanesulfonamido-1-(2,2,2-trifluoroethyl)-1H-indazol-7-yl]-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 69 | N-[(1S)-1-[(1'P)-1'-[4-chloro-3-methanesulfonamido-1-(2,2,2-trifluoroethyl)-1H-indazol-7-yl]-4,6-dimethyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 70 | N-[(1S)-1-[(1'P)-1'-[4-chloro-3-methanesulfonamido-1-(2,2,2-trifluoroethyl)-1H-indazol-7-yl]-4-methyl-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 71 | N-[(1S)-1-[(1'P)-1'-[4-chloro-3-methanesulfonamido-1-(2,2,2-trifluoroethyl)-1H-indazol-7-yl]-4-methoxy-6'-oxo-1',6'-dihydro-[2,4'-bipyrimidin]-2'-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |
| Example 72 | N-[(1S)-1-[(1P)-1-[4-chloro-3-methanesulfonamido-1-(2,2,2-trifluoroethyl)-1H-indazol-7-yl]-6-oxo-4-[6-(trifluoromethyl)pyridin-2-yl]-1,6-dihydropyrimidin-2-yl]-2-(3,5-difluorophenyl)ethyl]-2-[(2S,4R)-9-(difluoromethyl)-5,5-difluoro-7,8-diazatricyclo[4.3.0.0^{2,4}]nona-1(6),8-dien-7-yl]acetamide |

### BIOLOGICAL METHODS

HIV cell culture assay - MT-2 cells, 293T cells and the proviral DNA clone of NL₄₋₃ virus were obtained from the NIH AIDS Research and Reference Reagent Program. MT-2 cells were propagated in RPMI 1640 media supplemented with 10% heat inactivated fetal bovine serum (FBS), 100 µg/ml penicillin G and up to 100 units/mL streptomycin. The 293T cells were propagated in DMEM media supplemented with 10% heat inactivated FBS, 100 µg/mL penicillin G and 100 µg/mL streptomycin. A recombinant NL₄₋₃ proviral clone, in which a section of the nef gene was replaced with the Renilla luciferase gene, was used to make the reference virus used in these studies. The recombinant virus was prepared through transfection of the recombinant NL₄₋₃ proviral clone into 293T cells using Transit-293 Transfection Reagent from Mirus Bio LLC (Madison, WI). Supernatent was harvested after 2-3 days and the amount of virus present was titered in MT-2 cells using luciferase enzyme activity as a marker by measuring luciferase enzyme activity. Luciferase was quantitated using the EnduRen Live Cell Substrate from Promega (Madison, WI). Antiviral activities of compounds toward the recombinant virus were quantified by measuring luciferase activity in MT-2 cells infected for 4-5 days with the recombinant virus in the presence of serial dilutions of the compound.

The 50% effective concentration (EC₅₀) was calculated by using the exponential form of the median effect equation where (Fa) = 1/[1+ (ED₅₀/drug conc.)^{m}] (Johnson VA, Byington RT. Infectivity Assay. In Techniques in HIV Research. ed. Aldovini A, Walker BD. 71-76. New York: Stockton Press.1990). Curve fitting and analysis were performed with ActivityBase XE Runner software version 9.1.0.4 using model 203 (ID Business Solutions, LTD, Guildford, UK).

Compound cytotoxicity and the corresponding CC₅₀ values were determined using the same protocol as described in the antiviral assay except that uninfected cells were used. Cytotoxicity was assessed on day 4 in uninfected MT2 cells by using a XTT (2,3-bis[2-Methoxy-4-nitro-5-sulfophenyl]-2H-tetrazolium-5-carboxyanilide inner salt)-based colorimetric assay (Sigma-Aldrich, St Louis, Mo).

| **Example** | **EC₅₀ nM** | **CC₅₀ µM** |
|---|---|---|
| Example 1 | 0.72 | > 0.5 |
| Example 2 | 190 | > 0.5 |
| Example 3 | 1.7 | > 0.1 |
| Example 4 | 0.044 | > 0.1 |
| Example 5 | 0.054 | > 0.1 |
| Example 6 | 0.054 | > 0.1 |
| Example 7 | 0.53 | > 0.1 |
| Example 8 | 0.28 | > 0.1 |
| Example 9 | 0.040 | > 0.1 |
| Example 10 | 0.079 | > 0.1 |
| Example 11 | 0.81 | > 0.1 |
| Example 12 | 0.019 | > 0.1 |
| Example 13 | 0.20 | > 0.1 |
| Example 14 | 0.042 | > 0.1 |
| Example 15 | 0.59 | > 0.1 |
| Example 16 | 0.31 | > 0.1 |
| Example 17 | 0.81 | > 0.1 |
| Example 18 | 0.47 | > 0.1 |
| Example 19 | 0.74 | > 0.1 |
| Example 20 | 0.16 | > 0.1 |
| Example 21 | 0.069 | >0.5 |
| Example 22 | 0.46 | > 0.1 |
| Example 23 | 0.42 | > 0.1 |
| Example 24 | 0.37 | > 0.1 |
| Example 25 | 1.5 | > 0.1 |
| Example 26 | 0.11 | > 0.1 |
| Example 27 | 0.13 | > 0.1 |
| Example 28 | 0.39 | > 0.1 |
| Example 29 | 0.052 | > 0.1 |
| Example 30 | 0.20 | > 0.1 |
| Example 31 | 0.53 | > 0.1 |
| Example 32 | 0.30 | > 0.1 |
| Example 33 | 0.13 | > 0.1 |
| Example 34 | 0.24 | > 0.1 |
| Example 35 | 0.72 | > 0.1 |
| Example 36 | 0.16 | > 0.1 |
| Example 37 | 3.7 | > 0.1 |
| Example 38 | 0.39 | > 0.1 |
| Example 39 | 0.059 | > 0.1 |
| Example 40 | 0.60 | > 0.1 |
| Example 41 | 0.064 | > 0.1 |
| Example 42 | 0.21 | > 0.1 |
| Example 43 | 0.17 | > 0.1 |
| Example 44 | 0.24 | > 0.1 |
| Example 45 | 0.12 | > 0.1 |
| Example 46 | 0.050 | > 0.1 |
| Example 47 | 0.047 | > 0.1 |
| Example 48 | 0.18 | > 0.1 |
| Example 49 | 0.58 | > 0.1 |
| Example 50 | 0.81 | > 0.1 |

## Claims

1. A compound of Formula I, or a pharmaceutically acceptable salt thereof: 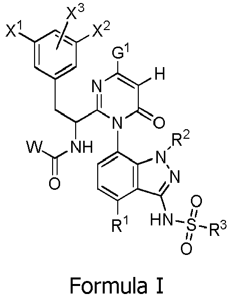 wherein:
X¹ and X² are independently selected from H, F, Cl, and -CH₃, and X³ is H, F, Cl, -CH₃, -OCH₃, - OCHF₂, or -OCF₃ with the proviso that within the group X¹, X², and X³ the substituent Cl is not used more than twice and the substituent -CH₃ is not used more than twice;
R¹ is hydrogen, Cl, or CH₃;
R² is hydrogen, C₁-C₃alkyl optionally substituted with 1-3 fluorines, or C₃-C₆cycloalkyl optionally substituted with 1-2 fluorines;
R³ is C₁-C₃alkyl or C₃-C₄ cycloalkyl;
G¹ is phenyl optionally substituted 1-3 times with a substituent independently selected from fluorine, chlorine, -CH₃, and -OCH₃, or G¹ is selected from: 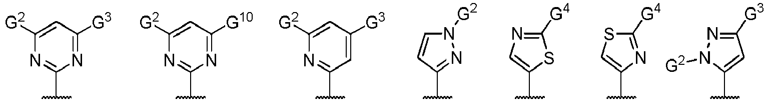 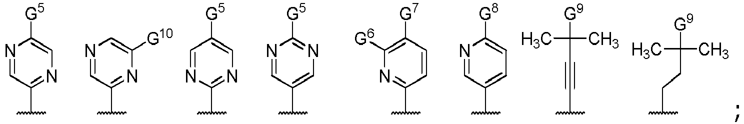
wherein G² and G³ are independently selected from hydrogen and C₁-C₃alkyl optionally substituted with 1-3 fluorines;
G⁴ is -C(CH₃)₂OH, -SO₂(C₁-C₄alkyl), or C₁-C₃alkyl optionally substituted with 1-3 fluorines;
G⁵ is H, F, C₁-C₄alkyl optionally substituted with 1-3 fluorines, or O(C₁-C₄alkyl) optionally substituted with 1-3 fluorines;
G⁶ is H, F, or Cl;
G⁷ is H, F, Cl, C₁-C₄alkyl optionally substituted with 1-3 fluorines, or O(C₁-C₄alkyl) optionally substituted with 1-3 fluorines;
G⁸ is F, Cl, -CN, C₁-C₄alkyl, or O(C₁-C₄ alkyl) optionally substituted with 1-3 fluorines;
G⁹ is -O(C₁-C₃alkyl), -O(C₃-C₄cycloalkyl), -SO₂(C₁-C₃alkyl), or -SO₂(C₃-C₄cycloalkyl);
G¹⁰ is -OCH₃, -OCHF₂, or -OCF₃;
W is selected from: 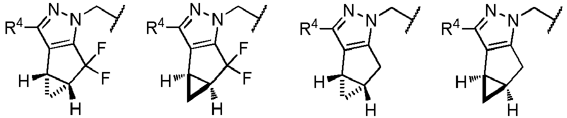 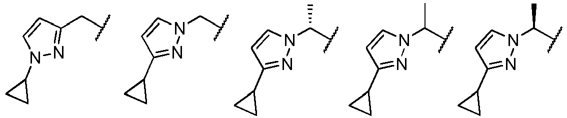
wherein R⁴ is methyl optionally substituted with 1-3 fluorines.

2. A compound or salt according to claim 1 wherein W is the following: 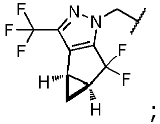 or
wherein W is the following: 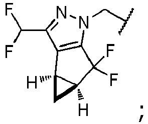 or
wherein W is one of the following: 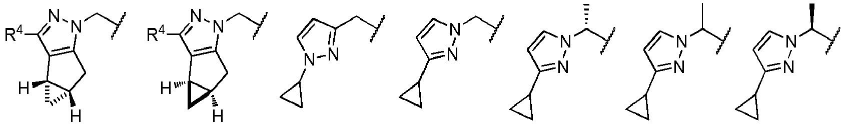
wherein R⁴ is methyl optionally substituted with 1-3 fluorines.

3. A compound or salt according to any of claims 1 or 2 wherein R¹ is Cl; R² is methyl, 2,2-difluoroethyl, or 2,2,2-trifluoroethyl; and R³ is methyl or cyclopropyl.

4. A compound or salt according to any of claims 1-3 wherein X³ is H; or
wherein X¹ is F, X² is F, and X³ is H.

5. A compound or salt according to any of claims 1-3 wherein if X³ is H then at least one of X¹ and X² is other than F.

6. A compound or salt according to any of claims 1-5 wherein G¹ is one of the following:

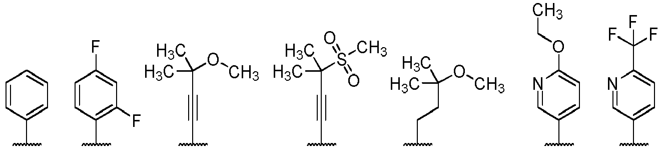

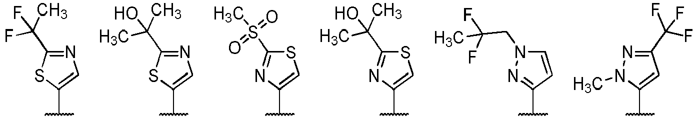

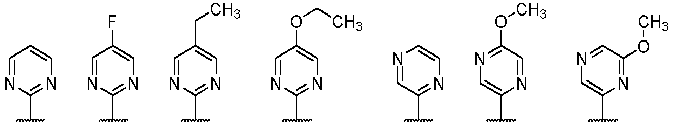

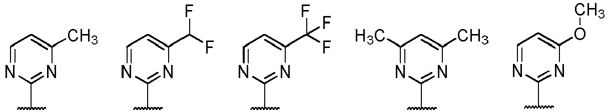

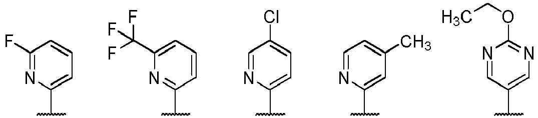

7. A compound or salt according to any of claims 1-6 wherein the stereochemistry is as depicted below: 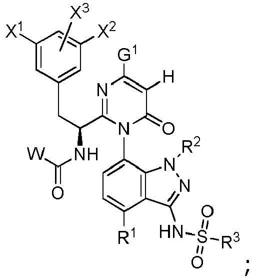 or
wherein the stereochemistry is as depicted below:

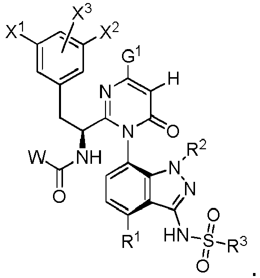

8. A compound or salt according to claim 1, selected from the group consisting of: 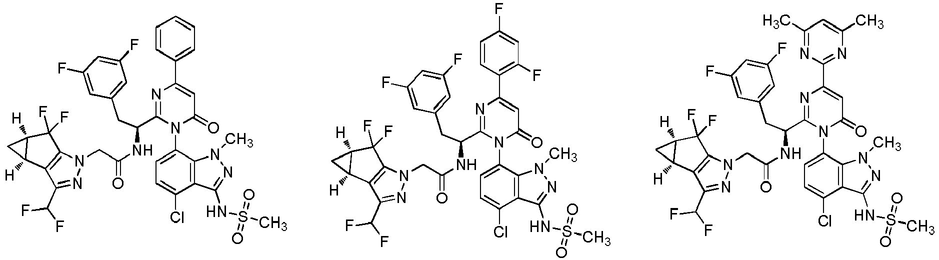 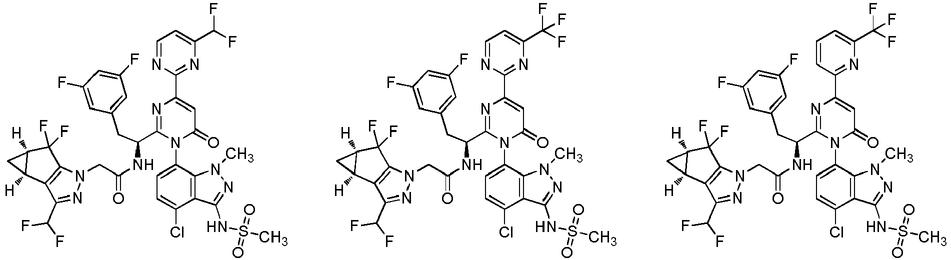 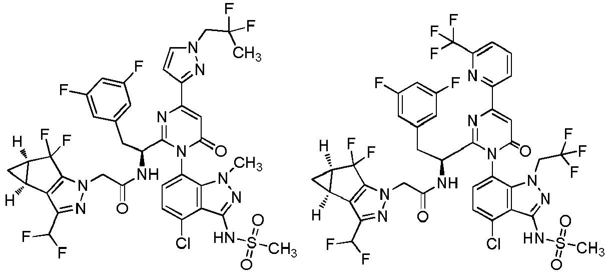 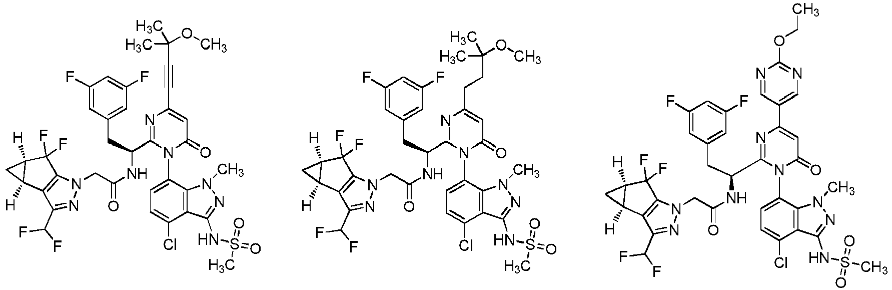 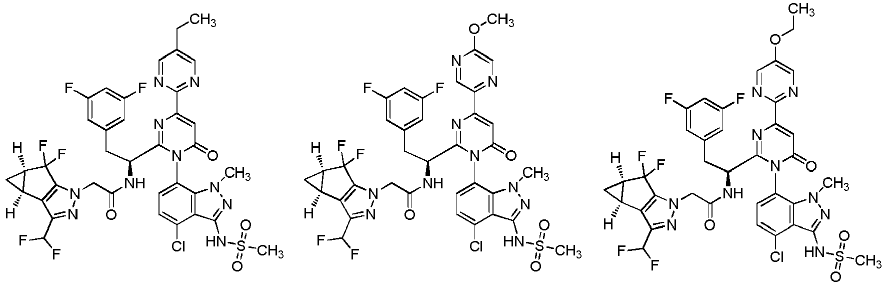 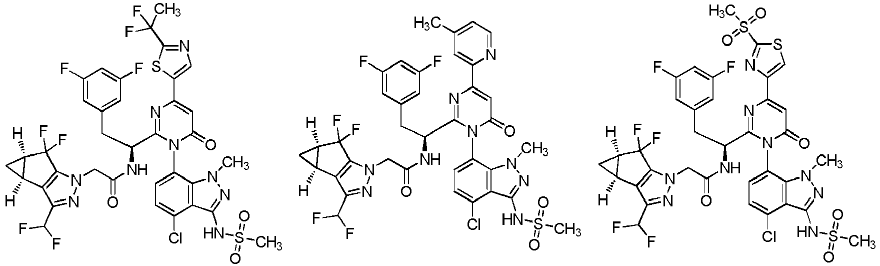 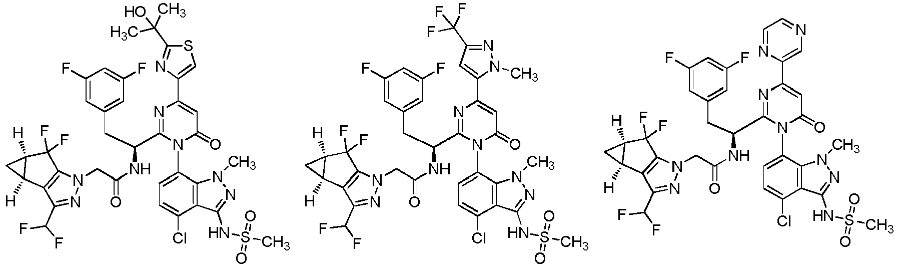 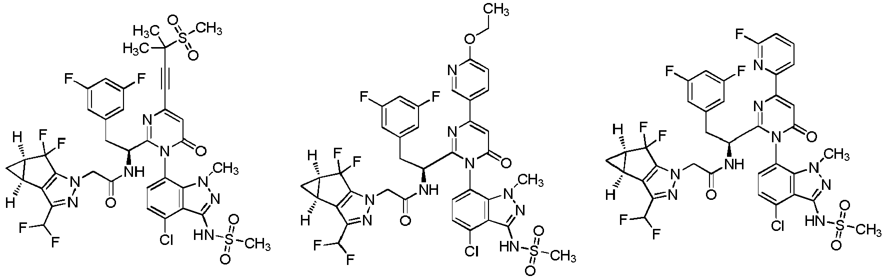 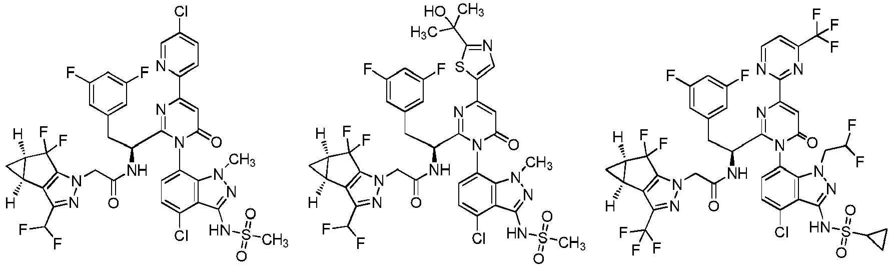 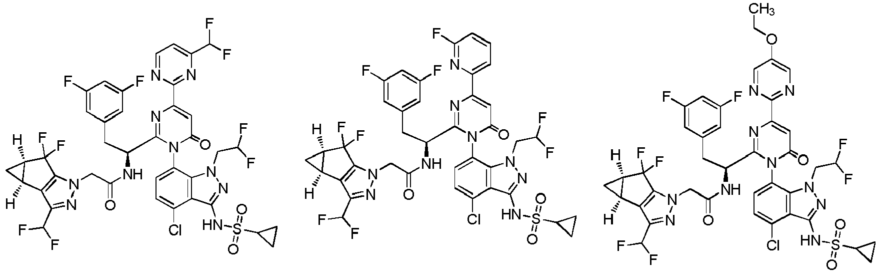 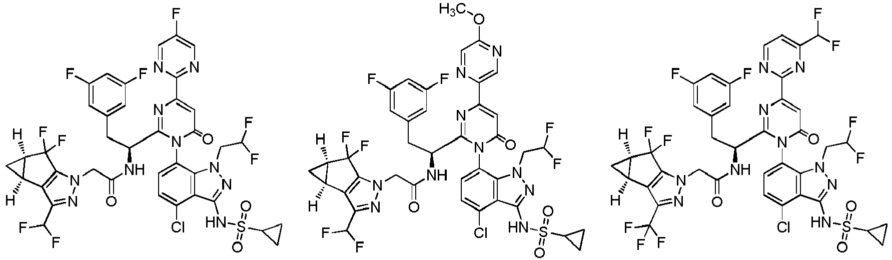 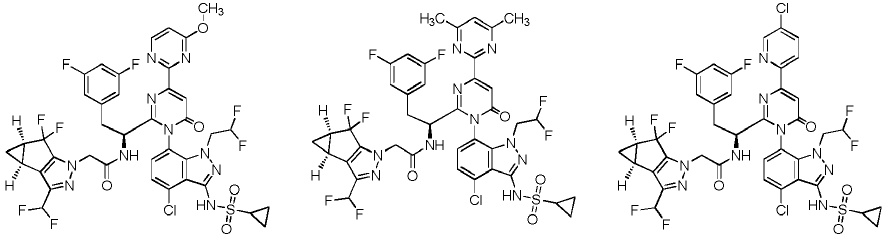 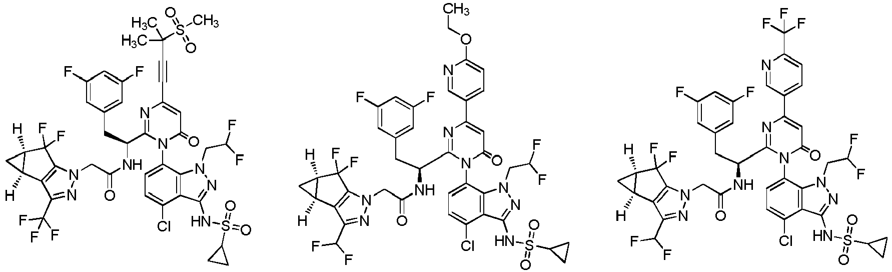 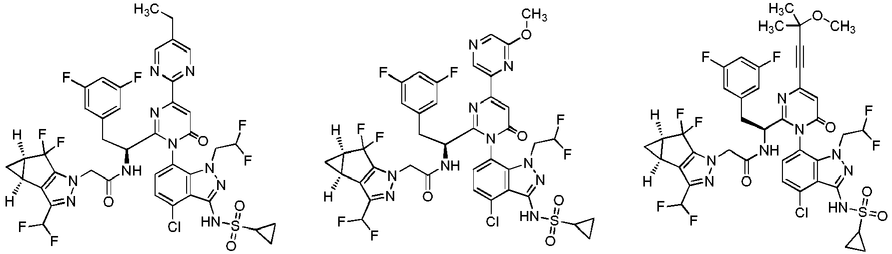 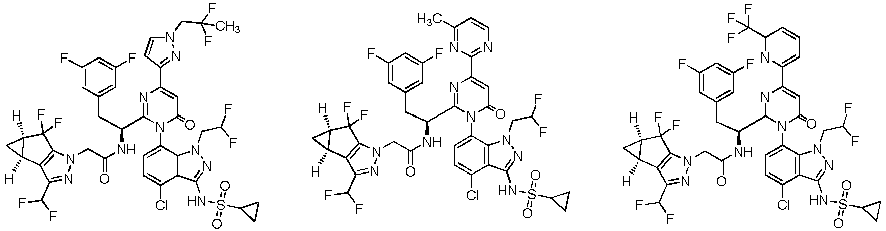 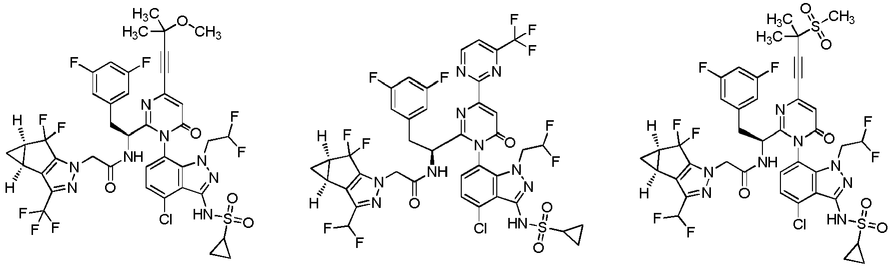 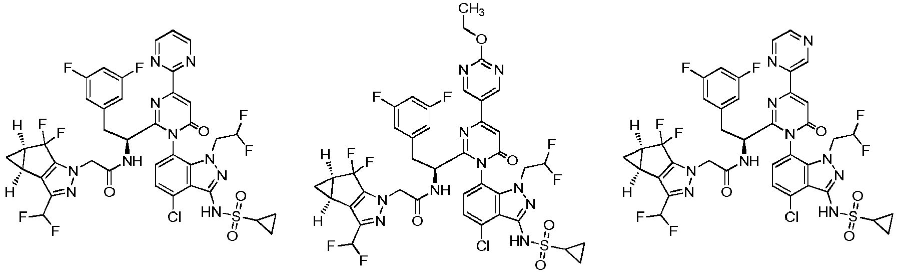 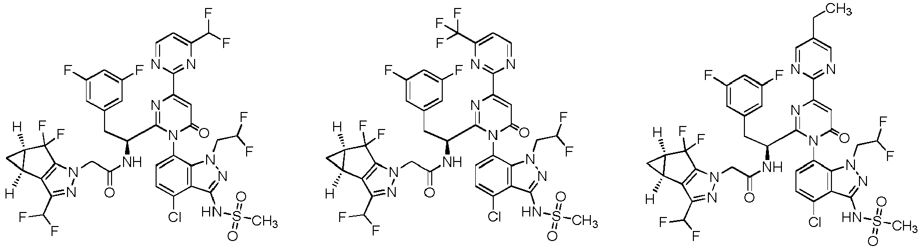 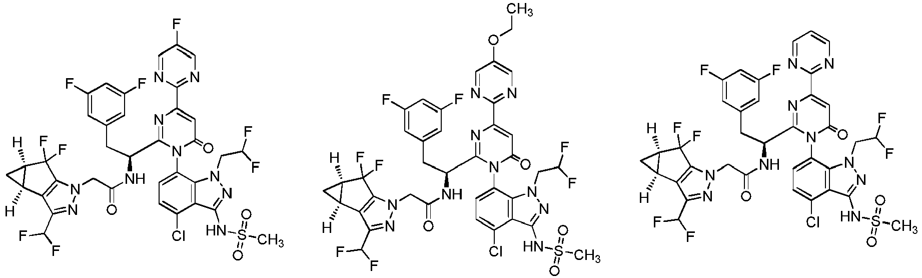 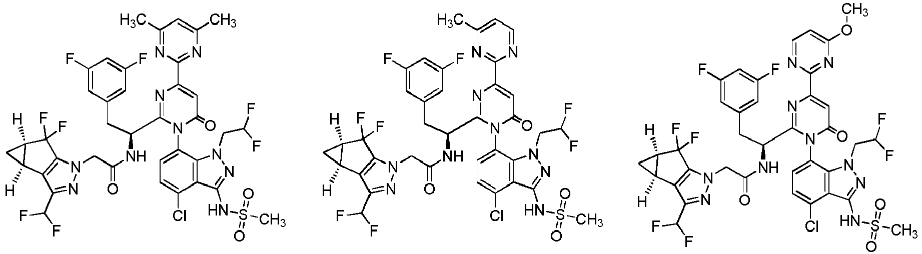 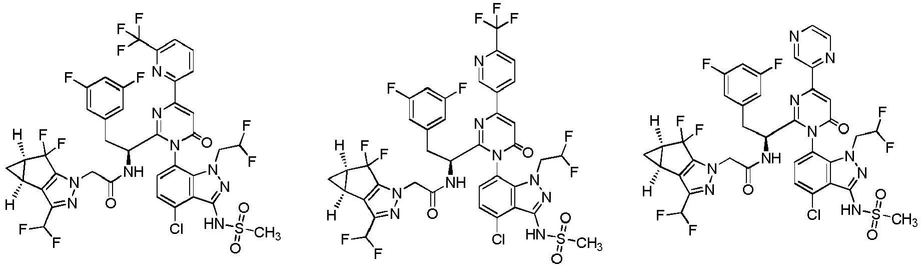 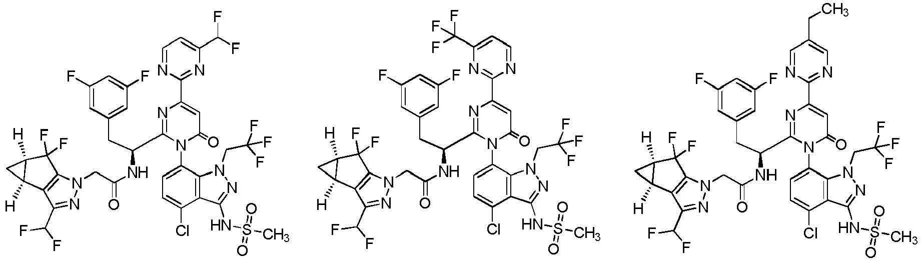 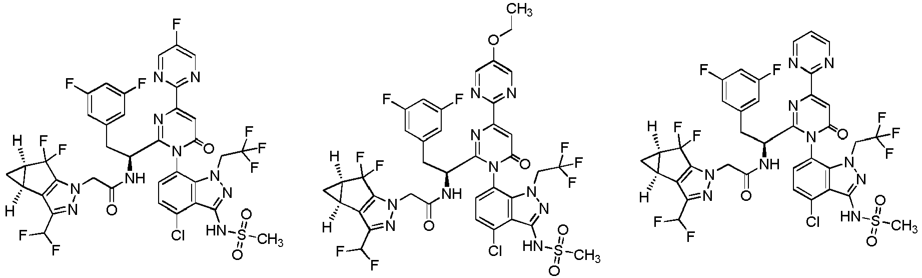 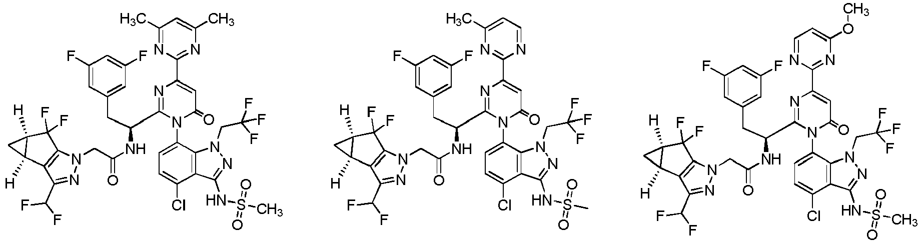 and pharmaceutically acceptable salts thereof.

9. A pharmaceutical composition comprising a compound or salt according to any of claims 1-8; or
a pharmaceutical composition comprising a compound or salt according to any of claims 1-8 and further comprising a pharmaceutically acceptable excipient.

10. A pharmaceutical composition according to claim 9 suitable for oral administration, for intramuscular injection, or for subcutaneous injection.

11. A compound or pharmaceutically acceptable salt thereof according to any of claims 1-8 for use in therapy.

12. A compound or pharmaceutically acceptable salt thereof according to any of claims 1-8 for use in treating HIV infection in a human.

13. The compound or pharmaceutically acceptable salt thereof for use according to claim 12, wherein the compound or pharmaceutically acceptable salt thereof according is administered orally; or
wherein the compound or pharmaceutically acceptable salt thereof is administered by injection intramuscularly.

14. The compound or pharmaceutically acceptable salt thereof for use according to claim 12, where the use further comprises administration of at least one other agent used for treatment of HIV infection in a human.

15. The compound or pharmaceutically acceptable salt thereof for use according to claim 14 wherein said at least one other agent is selected from the group consisting of dolutegravir, bictegravir, lamivudine, fostemsavir, and cabotegravir.

## Patentansprüche

1. Verbindung der Formel I oder pharmazeutisch akzeptables Salz davon: 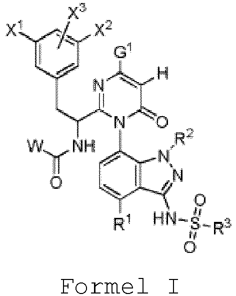 wobei:
X¹ und X² unabhängig ausgewählt sind aus H, F, Cl und -CH₃ und X³ H, F, Cl, -CH₃, -OCH₃, -OCHF₂ oder -OCF₃ ist, mit der Maßgabe, dass innerhalb der Gruppe X³, X² und X³ der Substituent Cl nicht mehr als zweimal verwendet wird und der Substituent -CH₃ nicht mehr als zweimal verwendet wird;
R¹ Wasserstoff, Cl oder CH₃ ist;
R³ Wasserstoff, C₁-C₃-Alkyl, optional substituiert mit 1-3 Fluoratomen, oder C₃-C₆-Cycloalkyl, optional substituiert mit 1-2 Fluoratomen, ist;
R³ C₁-C₃-Alkyl oder C₃-C₄-Cycloalkyl ist;
G¹ Phenyl ist, optional ein- bis dreimal substituiert mit einem Substituenten, unabhängig ausgewählt aus Fluor, Chlor, -CH₃ und -OCH₃, oder G¹ ausgewählt ist aus: 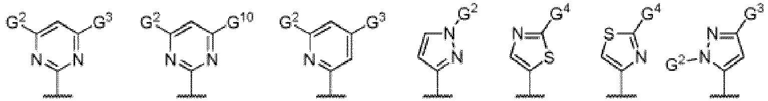 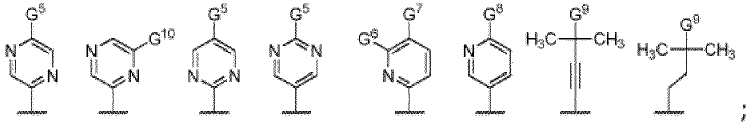
wobei G² und G³ unabhängig aus Wasserstoff und C₁-C₃-Alkyl ausgewählt sind, optional substituiert mit 1-3 Fluoratomen;
G⁴ -C(CH₃)₂OH, -SO₂(C₁-C₄-Alkyl) oder C₁-C₃-Alkyl, optional substituiert mit 1-3 Fluoratomen, ist;
G⁵ H, F, C₁-C₄-Alkyl, optional substituiert mit 1-3 Fluoratomen, oder O(C₁-C₄-Alkyl), optional substituiert mit 1-3 Fluoratomen, ist;
G⁶ H, F oder Cl ist;
G⁷ H, F, Cl, C₁-C₄-Alkyl, optional substituiert mit 1-3 Fluoratomen, oder O(C₁-C₄-Alkyl), optional substituiert mit 1-3 Fluoratomen, ist;
G⁸ F, Cl, -CN, C₁-C₄-Alkyl oder O(C₁-C₄-Alkyl), optional substituiert mit 1-3 Fluoratomen, ist;
G⁹ -O(C₁-C₃-Alkyl), -O(C₃-C₄-Cycloalkyl), -SO₂(C₁-C₃-Alkyl) oder -SO₂(C₃-C₄-Cycloalkyl) ist;
G¹⁰ -OCH₃, -OCHF₂ oder -OCF₃ ist;
W ausgewählt ist aus: 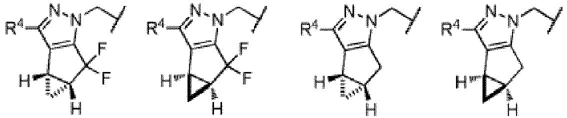 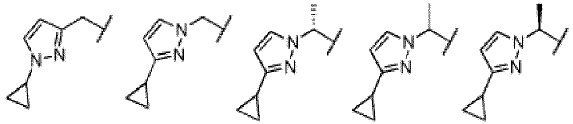
wobei R⁴ Methyl, optional substituiert mit 1-3 Fluoratomen, ist.

2. Verbindung oder Salz gemäß Anspruch 1, wobei W Folgendes ist: 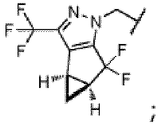 oder
wobei W Folgendes ist: 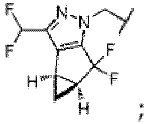 oder
wobei W eines der folgenden ist: 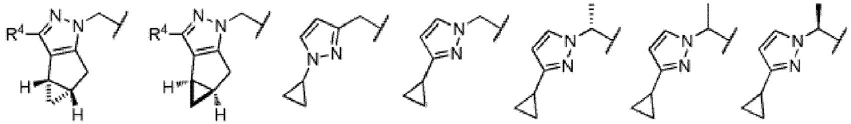
wobei R⁴ Methyl, optional substituiert mit 1-3 Fluoratomen, ist.

3. Verbindung oder Salz gemäß irgendeinem der Ansprüche 1 oder 2, wobei R¹ Cl ist; R² Methyl, 2,2-Difluorethyl oder 2,2,2-Trifluorethyl ist; und R³ Methyl oder Cyclopropyl ist.

4. Verbindung oder Salz gemäß irgendeinem der Ansprüche 1-3, wobei X³ H ist; oder wobei X¹ F ist, X² F ist und X³ H ist.

5. Verbindung oder Salz gemäß irgendeinem der Ansprüche 1-3, wobei, wenn X³ H ist, dann wenigstens eines von X¹ und X² etwas anderes ist als F.

6. Verbindung oder Salz gemäß irgendeinem der Ansprüche 1-5, wobei G¹ eines der folgenden ist:

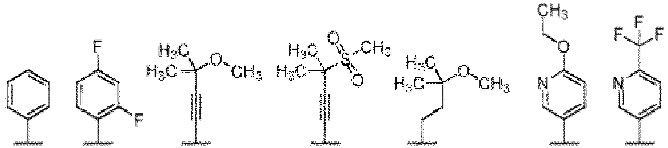

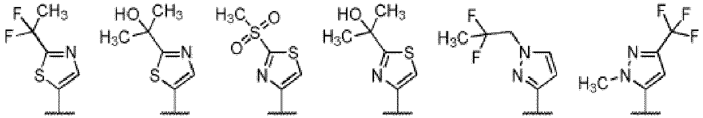

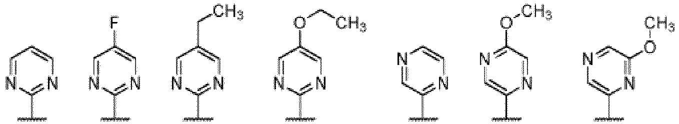

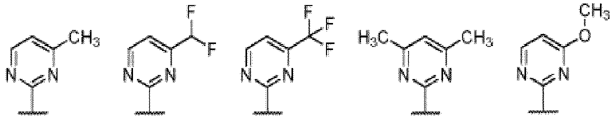

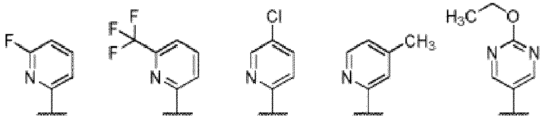

7. Verbindung oder Salz gemäß irgendeinem der Ansprüche 1-6, wobei die Stereochemie wie unten dargestellt ist: 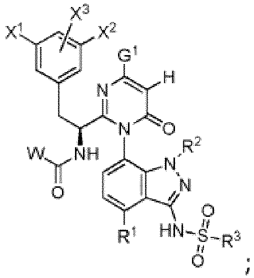 oder
wobei die Stereochemie wie unten dargestellt ist:

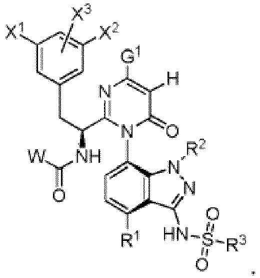

8. Verbindung oder Salz gemäß Anspruch 1, ausgewählt aus der Gruppe, bestehend aus: 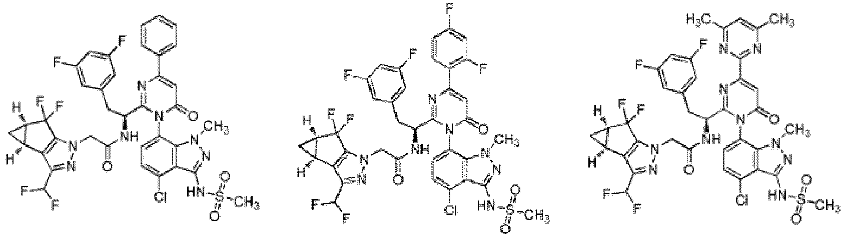 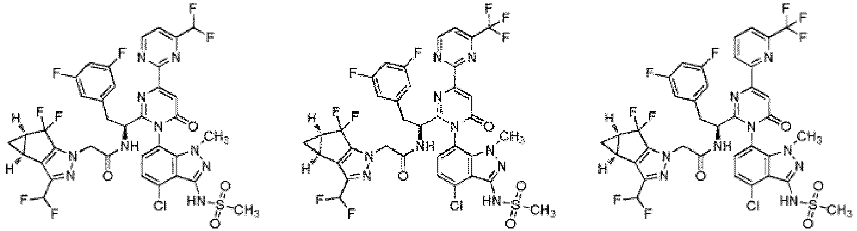 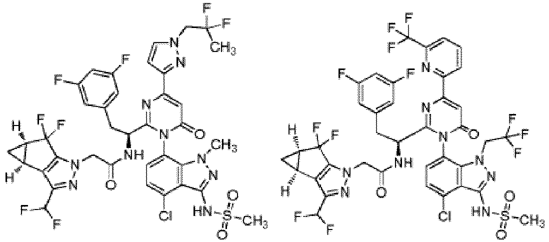 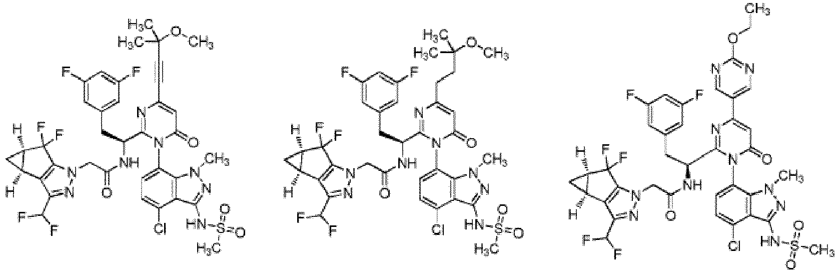 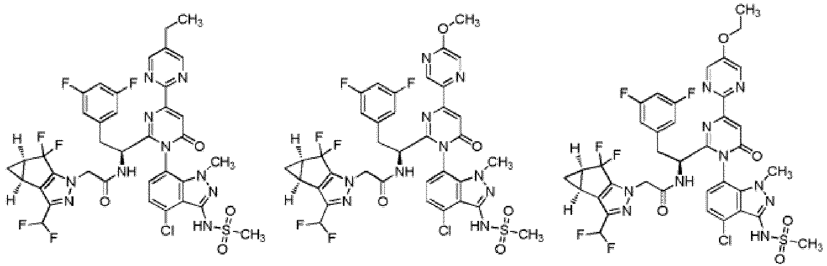 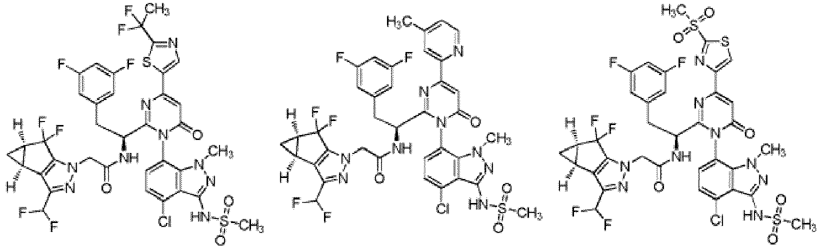 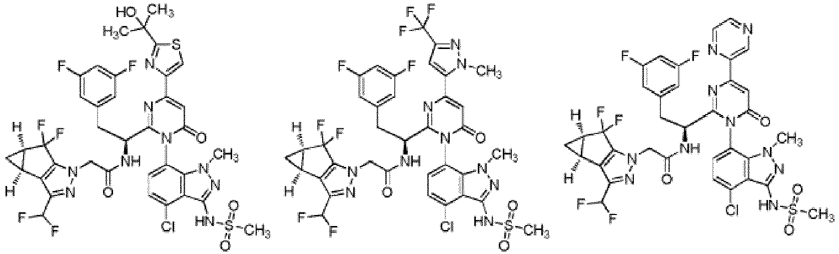 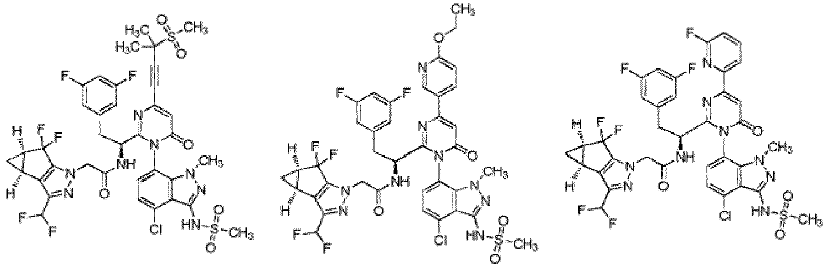 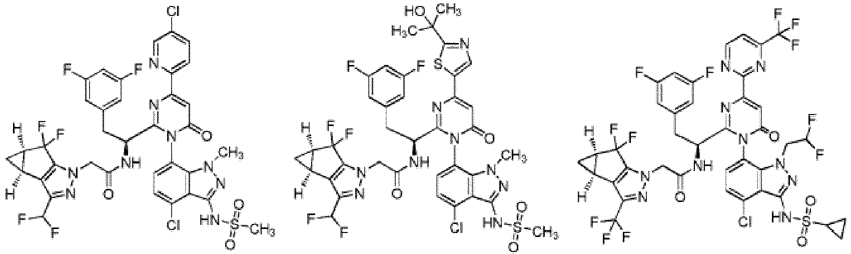 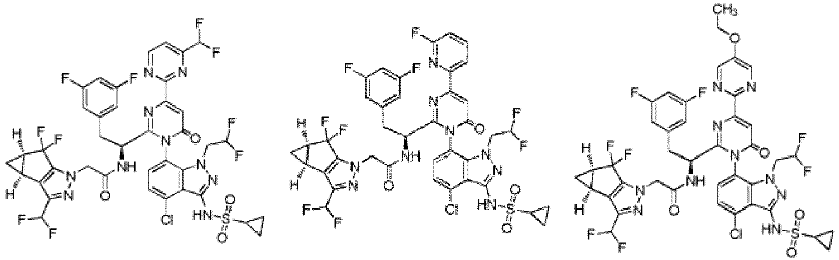 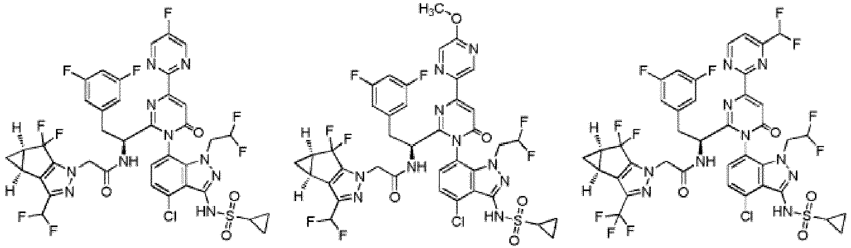 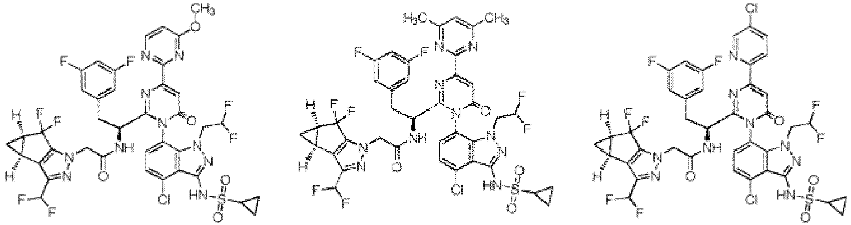 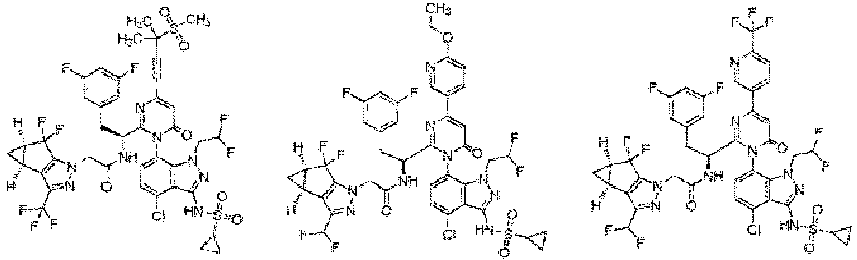 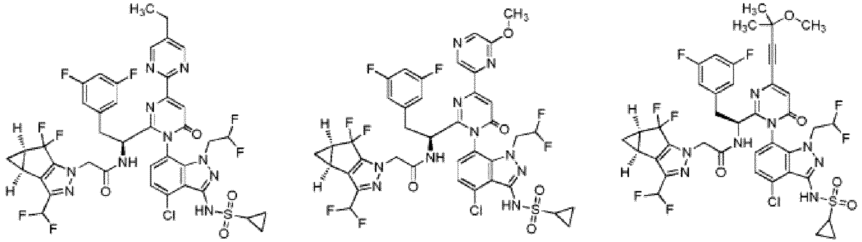 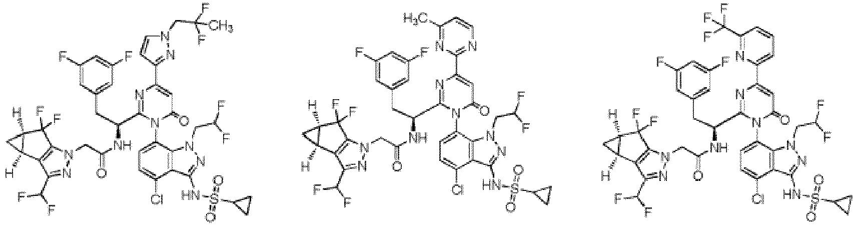 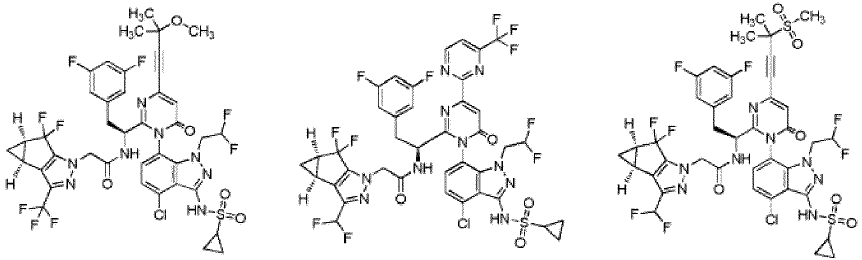 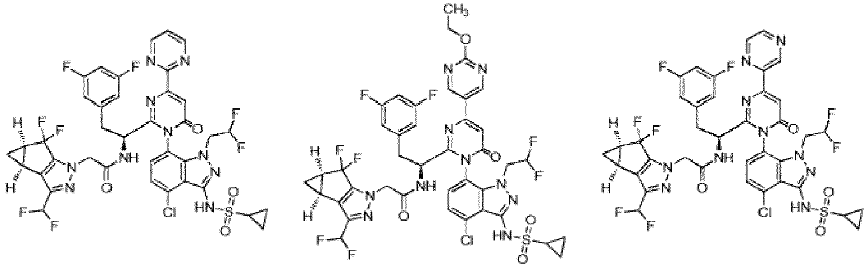 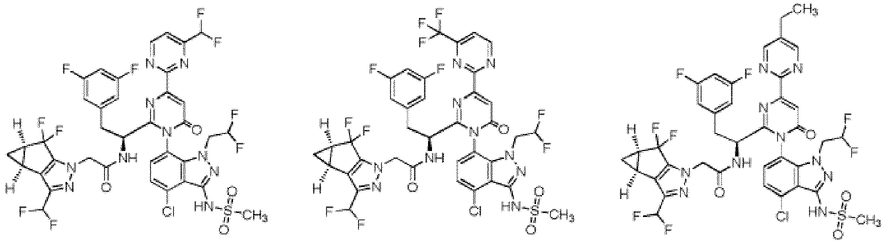 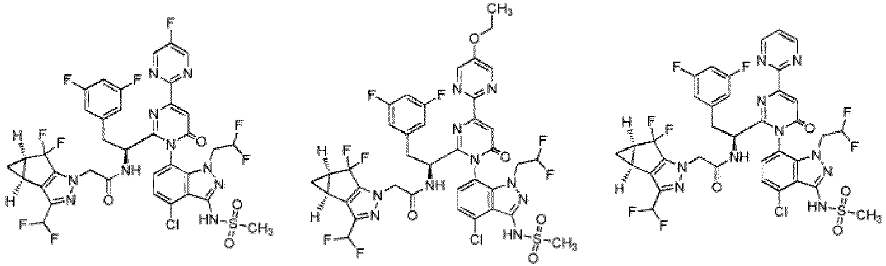 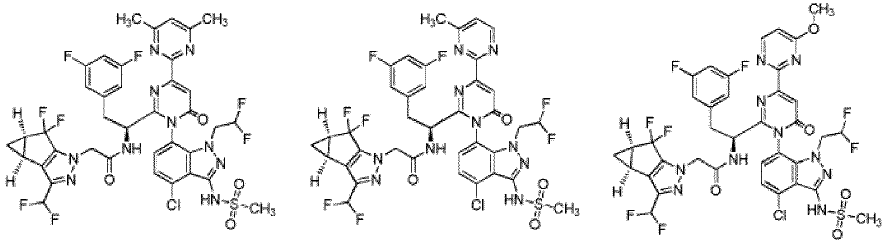 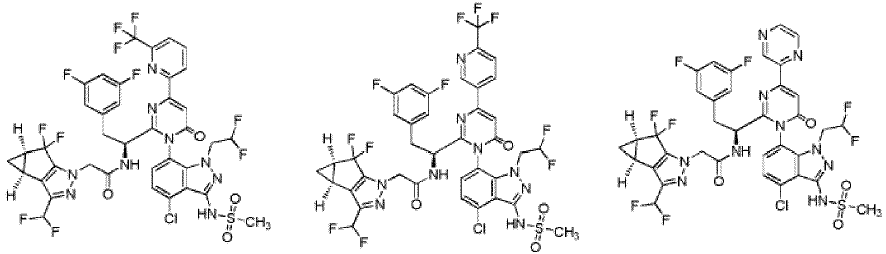 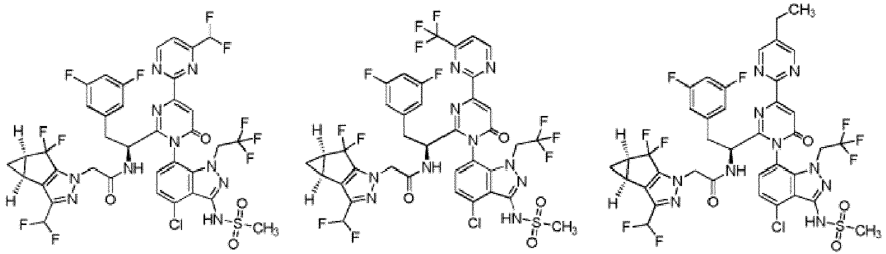 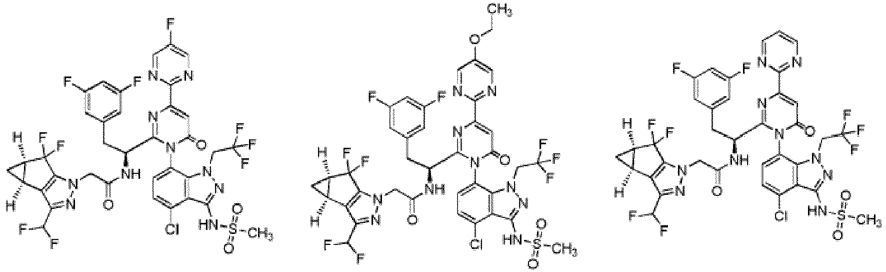 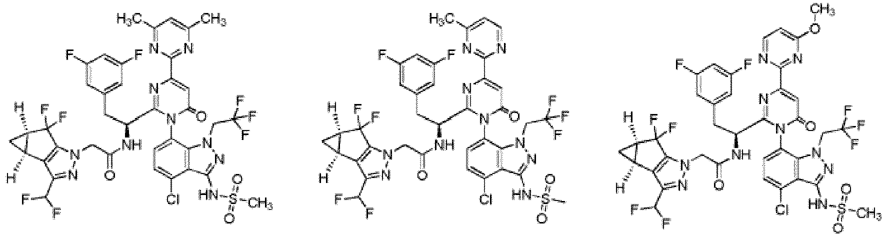 und pharmazeutisch akzeptablen Salzen davon.

9. Pharmazeutische Zusammensetzung, umfassend eine Verbindung oder ein Salz gemäß irgendeinem der Ansprüche 1-8; oder
pharmazeutische Zusammensetzung, umfassend eine Verbindung oder ein Salz gemäß irgendeinem der Ansprüche 1-8 und weiterhin umfassend einen pharmazeutisch akzeptablen Hilfsstoff.

10. Pharmazeutische Zusammensetzung gemäß Anspruch 9, die für orale Verabreichung, für intramuskuläre Injektion oder für subkutane Injektion geeignet ist.

11. Verbindung oder pharmazeutisch akzeptables Salz davon gemäß irgendeinem der Ansprüche 1-8 zur Verwendung in der Therapie.

12. Verbindung oder pharmazeutisch akzeptables Salz davon gemäß irgendeinem der Ansprüche 1-8 zur Verwendung bei der Behandlung von HIV-Infektion in einem Menschen.

13. Verbindung oder pharmazeutisch akzeptables Salz davon zur Verwendung gemäß Anspruch 12, wobei die Verbindung oder das pharmazeutisch akzeptable Salz davon oral verabreicht wird; oder
wobei die Verbindung oder das pharmazeutisch akzeptable Salz davon durch Injektion intramuskulär verabreicht wird.

14. Verbindung oder pharmazeutisch akzeptables Salz davon zur Verwendung gemäß Anspruch 12, wobei die Verwendung weiterhin die Verabreichung wenigstens eines anderen Agens umfasst, das für die Behandlung von HIV-Infektion in einem Menschen verwendet wird.

15. Verbindung oder pharmazeutisch akzeptables Salz davon zur Verwendung gemäß Anspruch 14, wobei das wenigstens eine andere Agens aus der Gruppe ausgewählt ist, bestehend aus Dolutegravir, Bictegravir, Lamivudin, Fostemsavir und Cabotegravir.

## Revendications

1. Composé de formule I, ou un sel pharmaceutiquement acceptable de celui-ci : 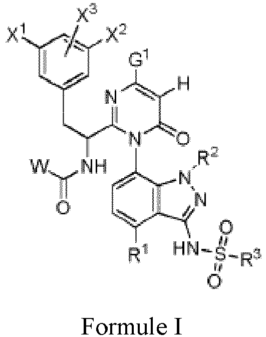 dans lequel :
X¹ et X² sont sélectionnés indépendamment parmi H, F, Cl, et -CH₃, et X³ est H, F, Cl, -CH₃, -OCH₃, - OCHF₂, ou -OCF₃ à condition que dans les groupes X¹, X², et X³, le substituant Cl ne soit pas utilisé plus de deux fois et que le substituant -CH₃ ne soit pas utilisé plus de deux fois ;
R¹ est un hydrogène, Cl, ou CH₃ ;
R² est un hydrogène, un alkyle en C₁ à C₃ facultativement substitué avec 1 à 3 fluors, ou un cycloalkyle en C₃ à C₆ facultativement substitué avec 1 à 2 fluors ;
R³ est un alkyle en C₁ à C₃ ou un cycloalkyle en C₃ à C₄ ;
G¹ est un phényle facultativement substitué 1 à 3 fois avec un substituant sélectionné indépendamment parmi un fluor, un chlore, -CH₃, et -OCH₃, ou G¹ est sélectionné parmi : 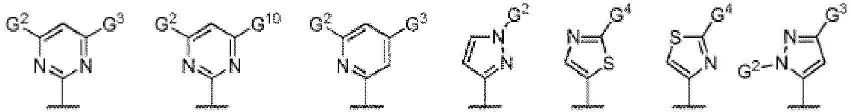 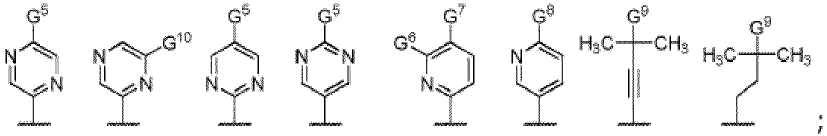
dans lequel G² et G³ sont sélectionnés indépendamment parmi un hydrogène et un alkyle en C₁ à C₃ facultativement substitué avec 1 à 3 fluors ;
G⁴ est -C(CH₃)₂OH, un -SO₂(alkyle en C₁ à C₄), ou un alkyle en C₁ à C₃ facultativement substitué avec 1 à 3 fluors ;
G⁵ est H, F, un alkyle en C₁ à C₄ facultativement substitué avec 1 à 3 fluors, ou un O(alkyle en C₁ à C₄) facultativement substitué avec 1 à 3 fluors ;
G⁶ est H, F, ou Cl ;
G⁷ est H, F, Cl, un alkyle en C₁ à C₄ facultativement substitué avec 1 à 3 fluors, ou un O(alkyle en C₁ à C₄) facultativement substitué avec 1 à 3 fluors ;
G⁸ est F, Cl, -CN, un alkyle en C₁ à C₄, ou un O(alkyle en C₁ à C₄) facultativement substitué avec 1 à 3 fluors ;
G⁹ est un -O(alkyle en C₁ à C₃), un -O(cycloalkyle en C₃ à C₄), un -SO₂(alkyle en C₁ à C₃), ou un -SO₂(cycloalkyle en C₃ à C₄) ;
G¹⁰ est -OCH₃, -OCHF₂, ou -OCF₃ ;
W est sélectionné parmi : 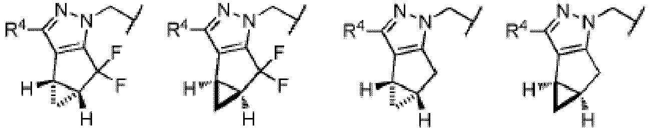 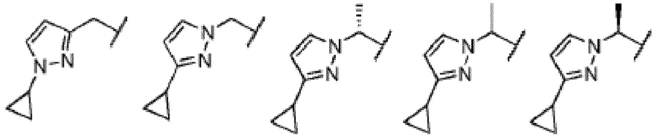
dans lequel R⁴ est un méthyle facultativement substitué avec 1 à 3 fluors.

2. Composé ou sel selon la revendication 1 dans lequel W est le suivant : 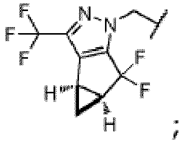 ou
dans lequel W est le suivant : 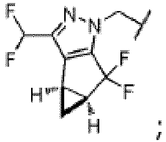 ou
dans lequel W est l'un des suivants : 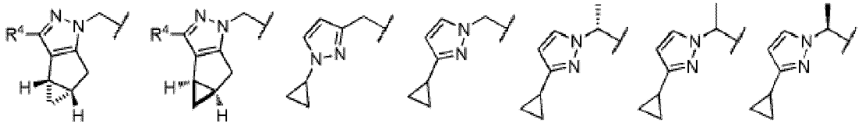
dans lequel R⁴ est un méthyle facultativement substitué avec 1 à 3 fluors.

3. Composé ou sel selon l'une quelconque des revendications 1 ou 2 dans lequel R¹ est Cl ; R² est un méthyle, un 2,2-difluoroéthyle, ou un 2,2,2-trifluoroéthyle ; et R³ est un méthyle ou un cyclopropyle.

4. Composé ou sel selon l'une quelconque des revendications 1 à 3 dans lequel X³ est H ; ou
dans lequel X¹ est F, X² est F, et X³ est H.

5. Composé ou sel selon l'une quelconque des revendications 1 à 3 dans lequel si X³ est H, alors au moins un parmi X¹ et X² est différent de F.

6. Composé ou sel selon l'une quelconque des revendications 1 à 5 dans lequel G¹ est l'un des suivants :

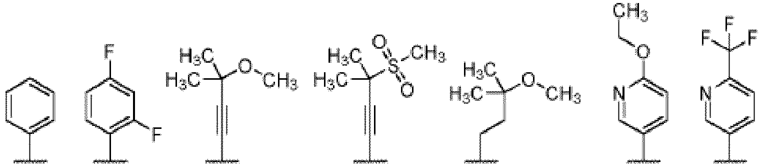

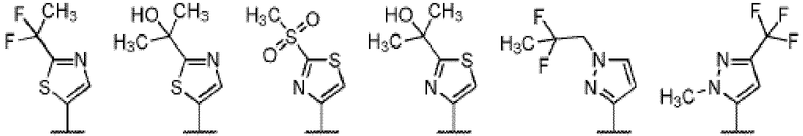

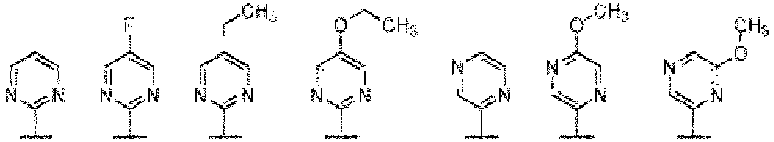

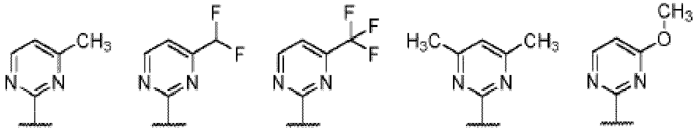

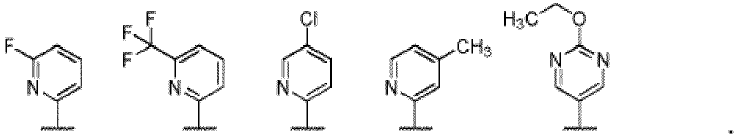

7. Composé ou sel selon l'une quelconque des revendications 1 à 6 dans lequel la stéréochimie est telle que représentée ci-dessous : 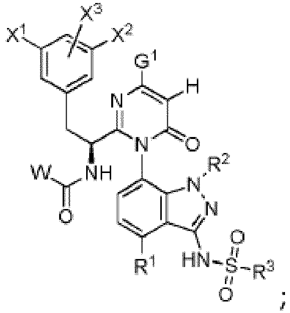 ou
dans lequel la stéréochimie est telle que représentée ci-dessous :

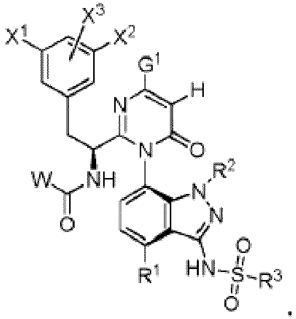

8. Composé ou sel selon la revendication 1, sélectionné dans le groupe constitué de : 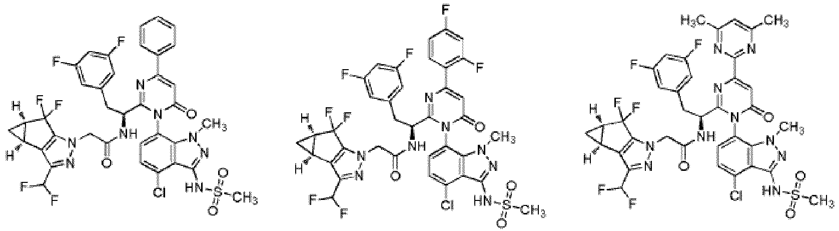 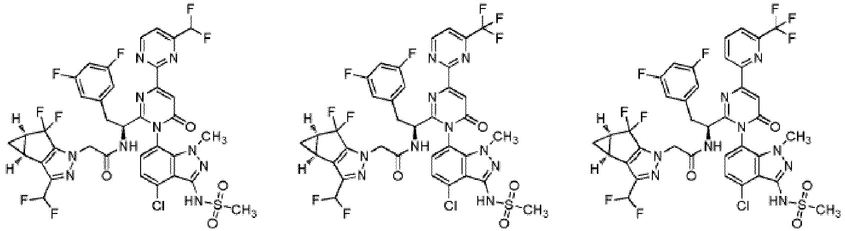 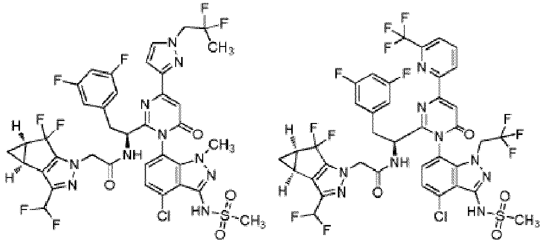 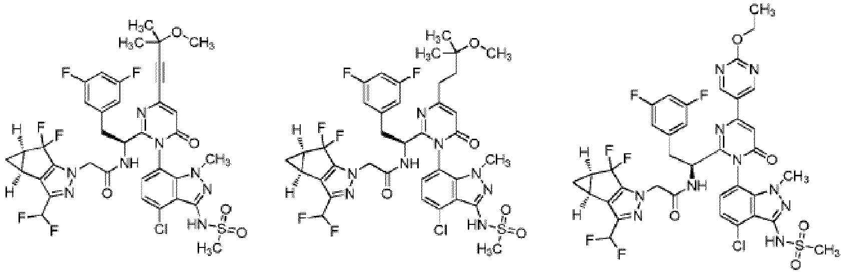 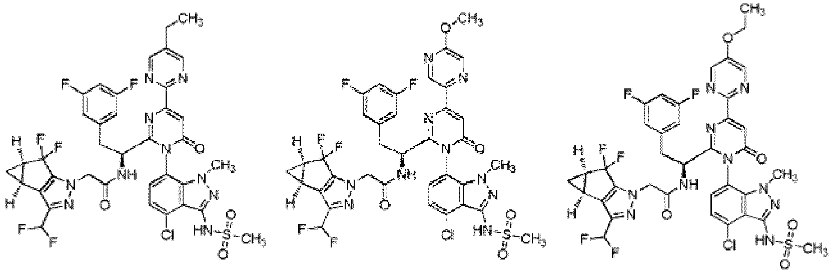 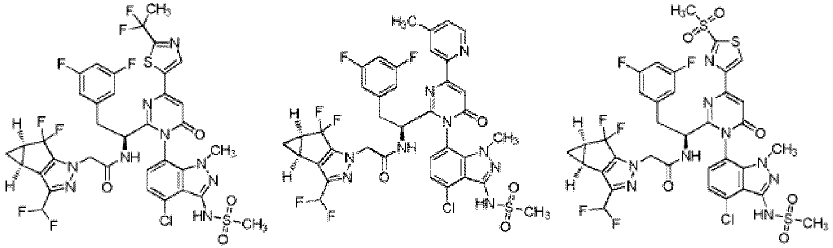 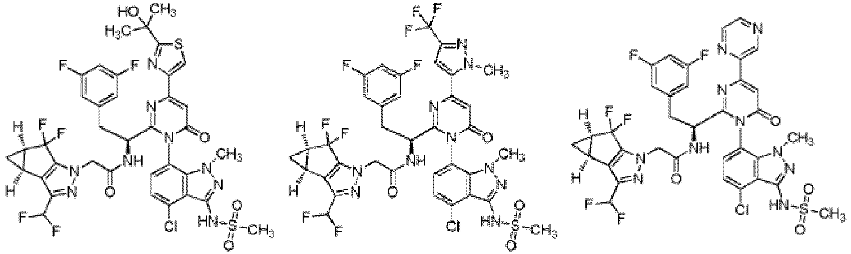 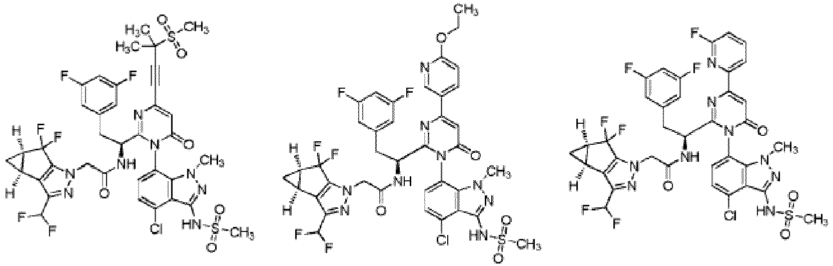 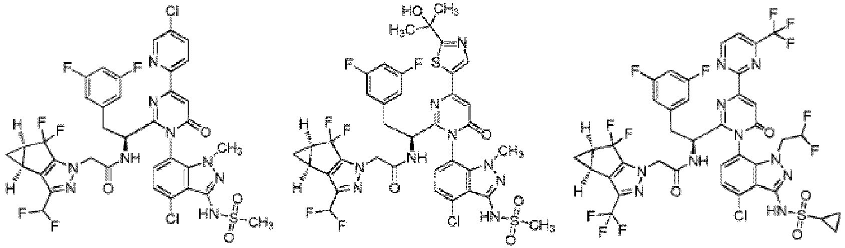 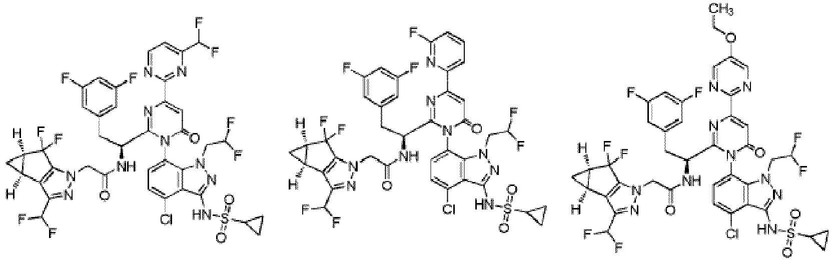 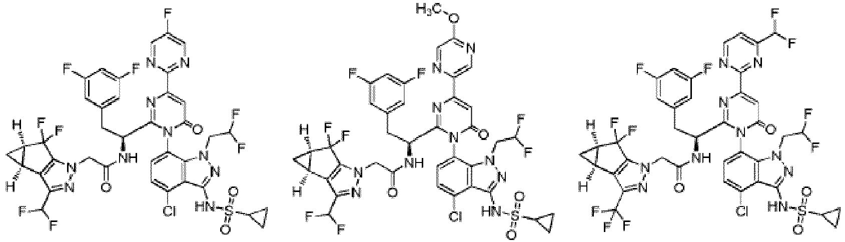 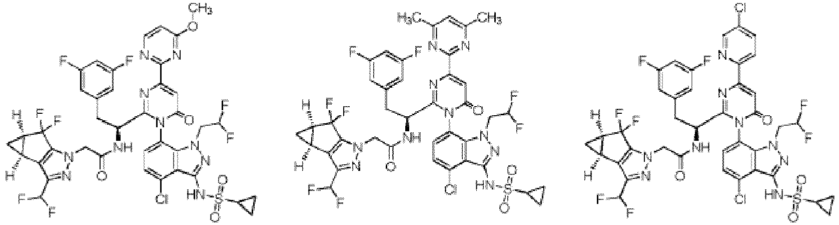 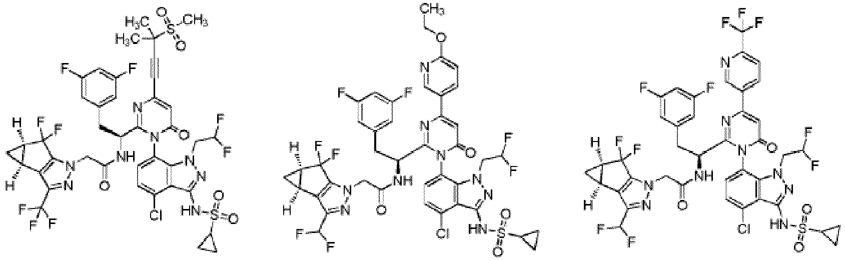 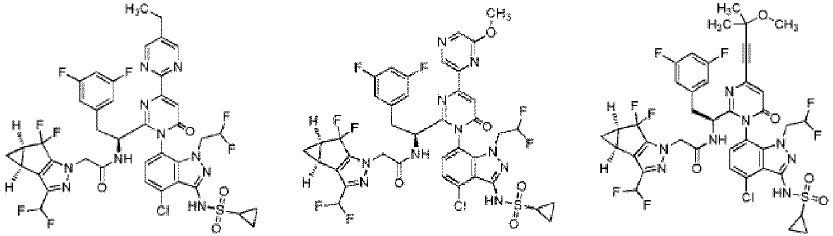 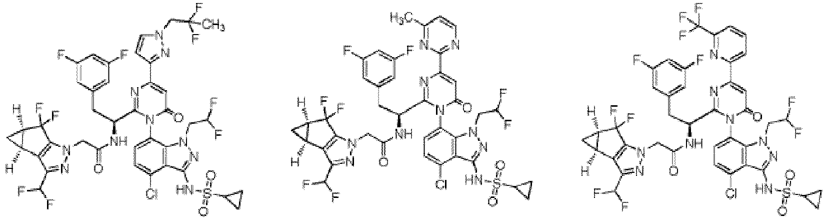 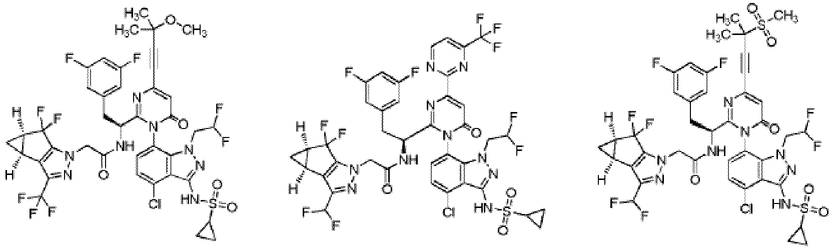 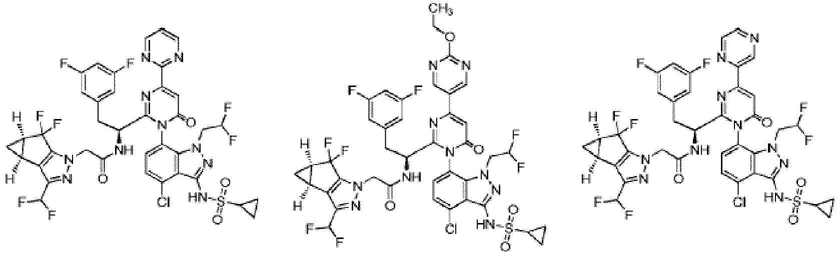 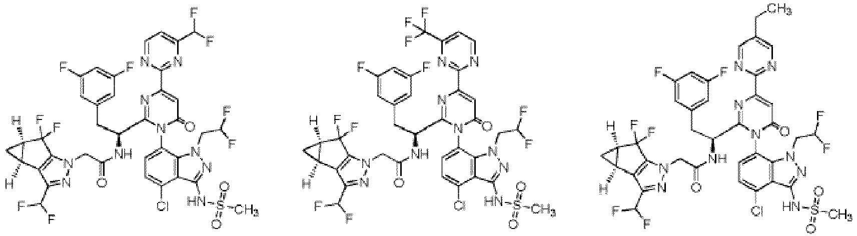 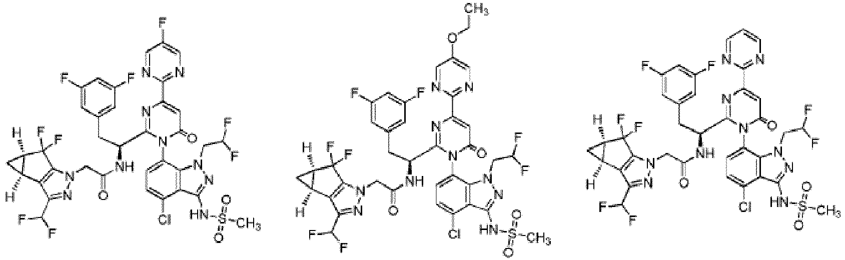 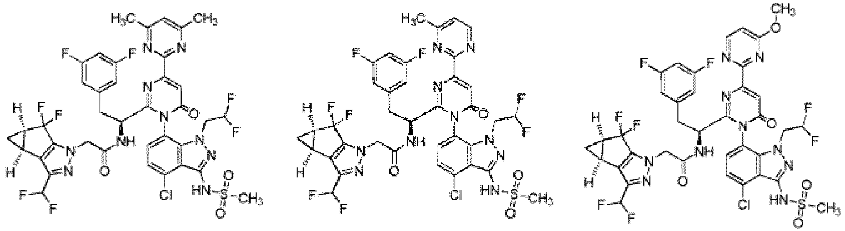 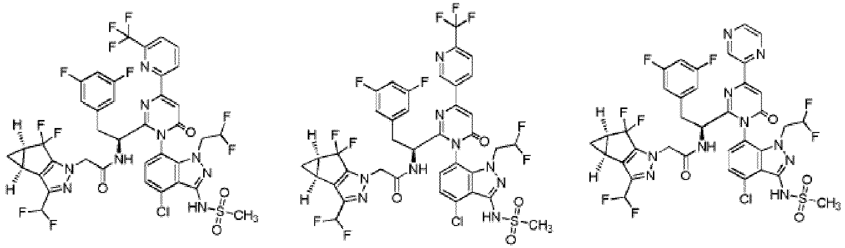 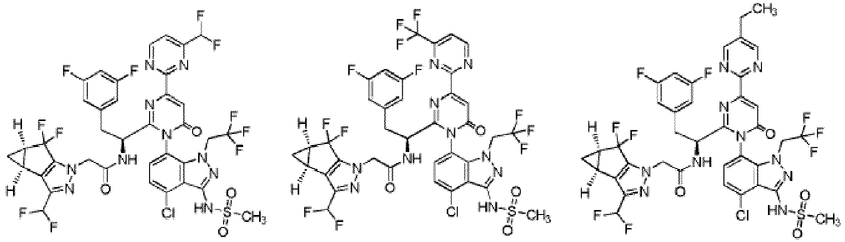 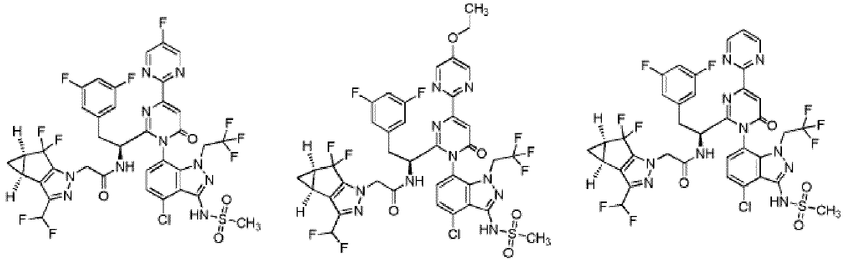 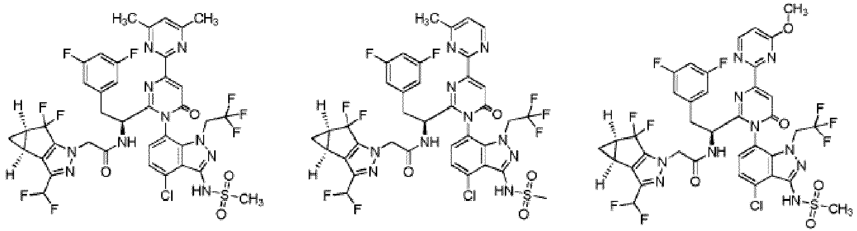 et les sels pharmaceutiquement acceptables de ceux-ci.

9. Composition pharmaceutique comprenant un composé ou un sel selon l'une quelconque des revendications 1 à 8 ; ou
une composition pharmaceutique comprenant un composé ou un sel selon l'une quelconque des revendications 1 à 8 et comprenant en outre un excipient pharmaceutiquement acceptable.

10. Composition pharmaceutique selon la revendication 9 appropriée pour une administration par voie orale, pour une injection intramusculaire, ou pour une injection sous-cutanée.

11. Composé ou sel pharmaceutiquement acceptable de celui-ci selon l'une quelconque des revendications 1 à 8 pour une utilisation en thérapie.

12. Composé ou sel pharmaceutiquement acceptable de celui-ci selon l'une quelconque des revendications 1 à 8 pour une utilisation dans le traitement d'une infection par le VIH chez un humain.

13. Composé ou sel pharmaceutiquement acceptable de celui-ci pour une utilisation selon la revendication 12, dans lequel le composé ou sel pharmaceutiquement acceptable de celui-ci est administré par voie orale ; ou
dans lequel le composé ou sel pharmaceutiquement acceptable de celui-ci est administré par injection intramusculaire.

14. Composé ou sel pharmaceutiquement acceptable de celui-ci pour une utilisation selon la revendication 12, où l'utilisation comprend en outre l'administration d'au moins un autre agent utilisé pour le traitement d'une infection par le VIH chez un humain.

15. Composé ou sel pharmaceutiquement acceptable de celui-ci pour une utilisation selon la revendication 14 dans lequel ledit au moins un autre agent est sélectionné dans le groupe constitué du dolutégravir, du bictégravir, de la lamivudine, du fostemsavir, et du cabotégravir.
